(19) Europäisches Patentamt
European Patent Office
Office européen des brevets

(11) EP 4 748 840 A1

(12) EUROPEAN PATENT APPLICATION
published in accordance with Art. 153(4) EPC

(43) Date of publication:
**27.05.2026 Bulletin 2026/22**

(21) Application number: **24855503.9**

(22) Date of filing: **08.07.2024**

(51) International Patent Classification (IPC):
***C07D 471/04*** *(2006.01)* ***A61K 31/454*** *(2006.01)*
***A61K 31/4188*** *(2006.01)* ***A61P 29/00*** *(2006.01)*
***A61P 35/00*** *(2006.01)* ***A61P 37/02*** *(2006.01)*

(86) International application number:
**PCT/CN2024/104246**

(87) International publication number:
**WO 2025/039774 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.08.2023 CN 202311057840**

(71) Applicant: **TAINUORUI (TIANJIN) BIOTECHNOLOGY CO., LTD**
**Tianjin 301726 (CN)**

(72) Inventors:
- **ZHAO, Tengfei**
  **Tianjin 301726 (CN)**
- **KONG, Kai**
  **Tianjin 301726 (CN)**
- **WANG, Jianli**
  **Tianjin 301726 (CN)**
- **YAO, Xiaoqing**
  **Tianjin 301726 (CN)**

(74) Representative: **Buzzi, Notaro & Antonielli d'Oulx S.p.A.**
**Corso Vittorio Emanuele II, 6**
**10123 Torino (IT)**

(54) **IMIDAZO[1,2-A]PYRIDINE TYPE IRAK DEGRADATION AGENT AND USE THEREOF**

(57) Provided in the present application are an imidazo[1,2-a]pyridine type IRAK4 degradation agent and the use thereof. Said IRAK4 degradation agent has a structure shown as formula I. Said IRAK4 degradation agent has remarkable degradation activity on IRAK4 in cells, and thus can be used for preparing drugs for treating and/or preventing IRAK4-mediated related diseases or disorders.

EP 4 748 840 A1

## Description

### TECHNICAL FIELD

The present application belongs to the field of pharmaceutical synthesis technology and relates to an imidazo [1,2-a]pyridine IRAK4 degrader and use thereof.

### BACKGROUND

Interleukin-1 receptor-associated kinase 4 (IRAK4) is one of the isozymes of the intracellular serine/threonine protein kinase IRAK family, is a key node in downstream signaling pathways of Toll-like receptors (TLRs) and interleukin-1 receptors (IL-1Rs), and plays an important role in an immune system. IRAK4, which is overactivated or abnormally activated due to mutations, participates in the occurrence and development of inflammatory diseases and tumors.

TLRs and IL-1Rs share a conserved TLR/IL-1R (TIR) domain and recruit an adaptor protein MyD88 intracellularly, and MyD88 further recruits IRAK4 to form a Myddosome. In the Myddosome, IRAK4 is activated through trans-autophosphorylation and then activates a series of transcription factors downstream, such as NF-κB, CREB, AP-1, and IRFs, thereby promoting the secretion of pro-inflammatory cytokines and the proliferation and differentiation of immune cells. Therefore, IRAK4 plays an important role in the onset and progression of inflammatory diseases.

The dysregulation of the TLR/IL-1R signaling pathway is closely related to the occurrence and progression of cancer. Mutations of proteins in the TLR/IL-1R pathway may lead to overactive signaling and increased NF-κB activity, promoting cancer.

IRAK4 mediates the downstream signaling pathways of IL-1R/TLRs and participates in immune surveillance through two routes: IRAK4 has kinase activity and can phosphorylate downstream proteins IRF5/7; moreover, IRAK4 acts as a scaffold structure and is responsible for assembling a protein oligomer, a myddosome complex. Many small-molecule IRAK4 kinase inhibitors in the existing art can only inhibit the activity of the IRAK4 kinase and exhibit moderate potency and cannot disrupt the formation of the complex to completely block the generation of inflammatory response signals.

Targeted protein degradation (TPD) is an emerging therapeutic approach and has attracted considerable attention because the TPD can modulate proteins that are difficult to target by traditional small molecules. Proteolysis targeting chimeras (PROTACs) achieve the targeted degradation of proteins of interest (POIs) by using an intracellular natural protein degradation system, ubiquitin proteasome system (UPS). A PROTAC is a heterobifunctional small molecule, one end of which targets a POI and the other end of which recruits an E3 ubiquitin ligase to form a ternary complex. The POI is labeled with ubiquitin and then recognized and degraded by proteasomes.

Compared with traditional small-molecule drugs, PROTAC molecules have the following advantages: (1) the PROTAC molecules have a catalytic degradation function; unlike each small-molecule inhibitor that can act on only one protein molecule, each PROTAC molecule can degrade many protein molecules and thus can achieve good efficacy at a very low dose; (2) the PROTAC molecules exert effects by degrading the POIs and thus can overcome resistance of small-molecule drugs due to increased target protein expression and also block a non-enzymatic function of the target. Therefore, in view of the characteristics of IRAK4 having both the kinase activity and the scaffold function, the PROTAC technology can degrade IRAK4 proteins to simultaneously block the kinase activity and disrupt the scaffold function of the target proteins. Compared with conventional kinase inhibitors, the PROTAC technology can completely inhibit the pathways and produce better anti-inflammatory effects or antitumor activity. Accordingly, the further development of such molecules is of great significance in the art.

### SUMMARY

The present application provides an imidazo[1,2-a]pyridine IRAK4 degrader and use thereof.

In a first aspect, the present application provides an imidazo[1,2-a]pyridine IRAK4 degrader. The imidazo[1,2-a] pyridine IRAK4 degrader has a structure represented by Formula I:

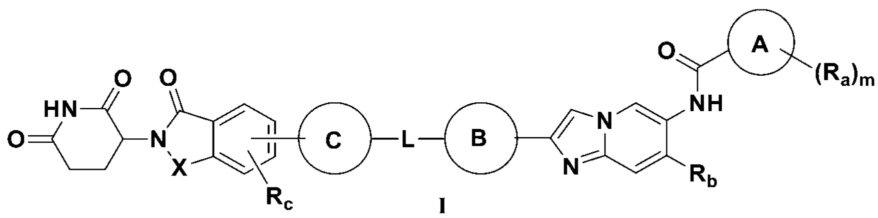

I

;

wherein the ring A is 6- to 10-membered aryl or 5- to 10-membered heteroaryl;
the ring B is substituted or unsubstituted 4- to 10-membered cycloalkyl or substituted or unsubstituted 4- to 10-membered heterocycloalkyl;
the ring C is substituted or unsubstituted 4- to 10-membered cycloalkyl or substituted or unsubstituted 4- to 10-membered heterocycloalkyl;
X is $CH_2$ or C=O;
$R_a$ is each independently selected from halogen, cyano, substituted or unsubstituted C1 to C4 alkyl, substituted or unsubstituted C1 to C4 alkoxy, di(C1 to C4 alkyl)amino, 5- to 10-membered cycloalkyl, or 5- to 10-membered heterocycloalkyl;
m is an integer from 0 to 5;
$R_b$ is selected from C1 to C4 alkoxy, C3 to C6 cycloalkyloxy, or hydroxyldi(C1 to C4 alkyl)methyl;
$R_c$ is hydrogen, C1 to C4 alkyl, C1 to C4 alkoxy, or halogen; and
L is $(CH_2)_n$, $(CH_2)_nCO$, 4- to 8-membered cycloalkyl, or 4- to 8-membered heterocycloalkyl, wherein n is an integer from 0 to 5 (for example, 0, 1, 2, 3, 4, or 5).

In the present application, a substituent in a substituted group is selected from halogen, oxo, cyano, amino, hydroxyl, C1 to C6 alkyl, or -O-(C1 to C6 alkyl).

Preferably, the ring A is

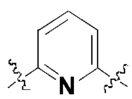, 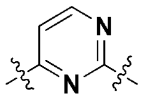, 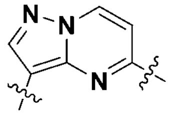, or 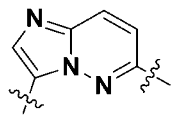,

wherein the squiggle represents a linkage site of the group.

Preferably, the ring B is

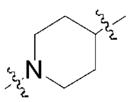, 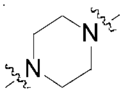, or 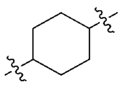,

wherein the squiggle represents a linkage site of the group.

Preferably, the ring C is

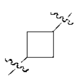, 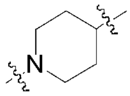, 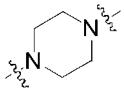, 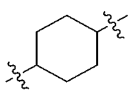, 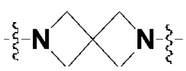,

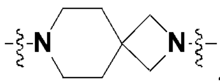, 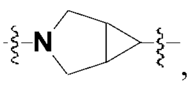, or 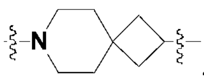,

wherein the squiggle represents a linkage site of the group.

Preferably, $R_a$ is each independently selected from fluorine, chlorine, cyano, trifluoromethyl, trifluoromethoxy,

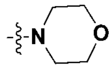, or 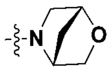,

wherein the squiggle represents a linkage site of the group.

Preferably, $R_b$ is selected from any one of methoxy, ethoxy, isopropoxy, cyclopropyloxy, or

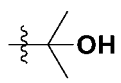,

wherein the squiggle represents a linkage site of the group.

Preferably, $R_c$ is selected from hydrogen, fluorine, chlorine, or bromine.

Preferably, L is $-CH_2-$, $-CH_2CH_2-$, $-CH_2CH_2CH_2-$, $-COCH_2-$, $-COCH_2CH_2-$, $-COCH_2CH_2CH_2-$, or

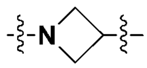.

Preferably, the imidazo[1,2-a]pyridine IRAK4 degrader is selected from any one of the following compounds:

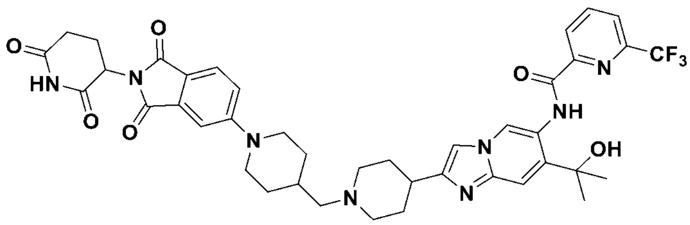,

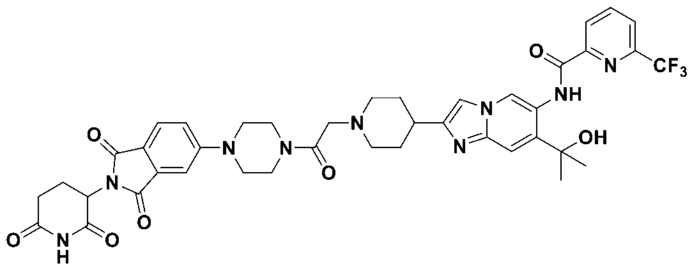,

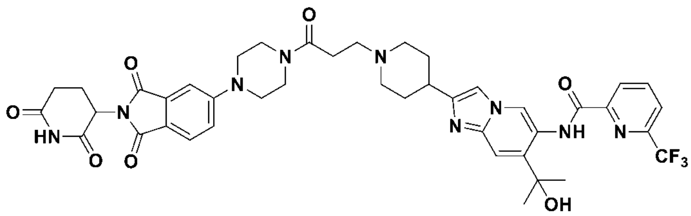,

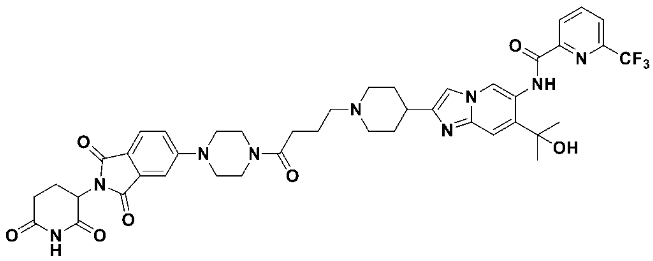,

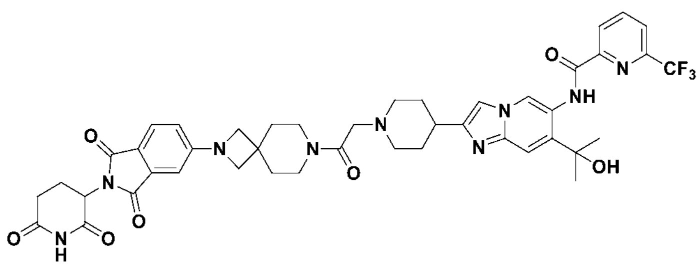,

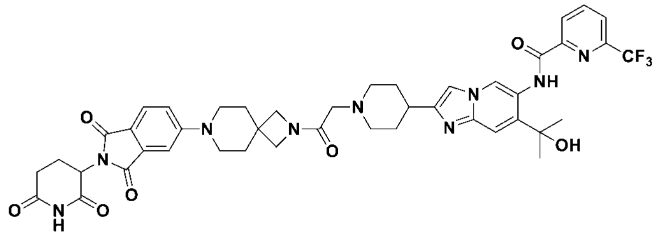

,

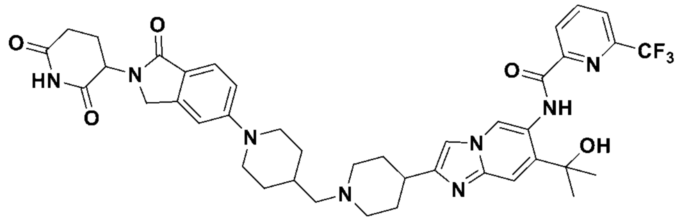

,

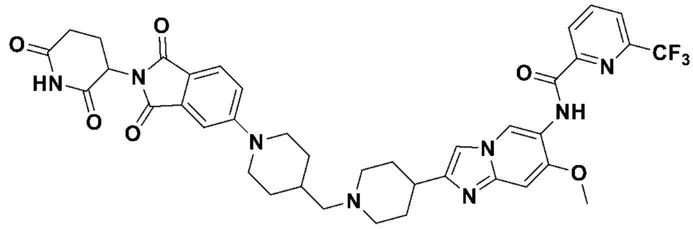

,

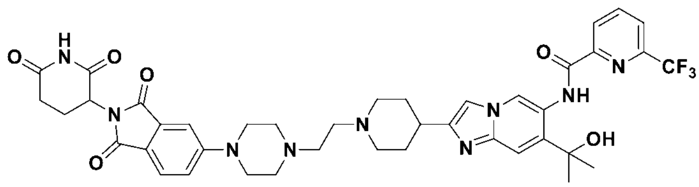

,

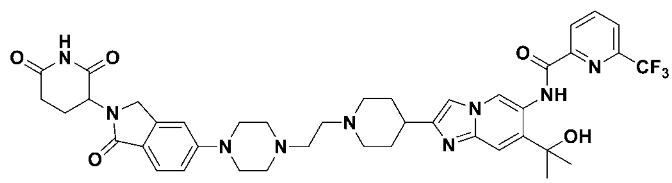

,

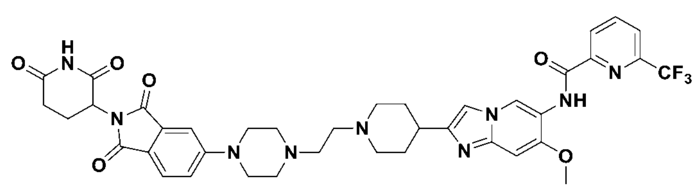

,

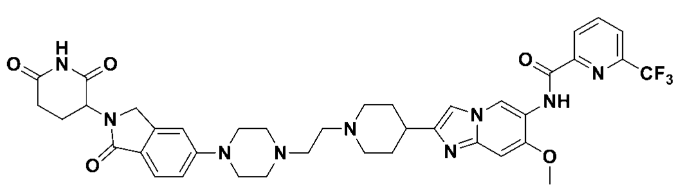

,

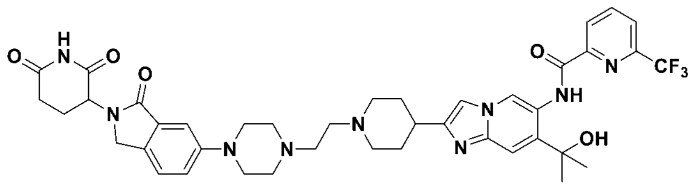

,

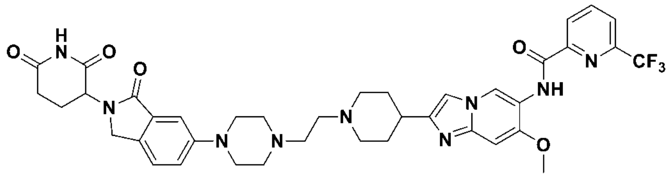

,

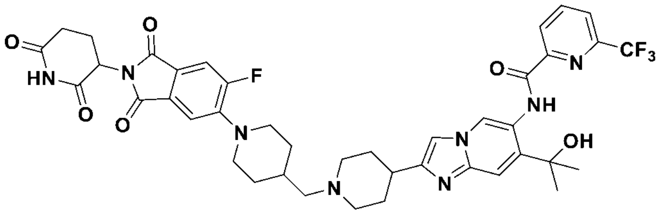

,

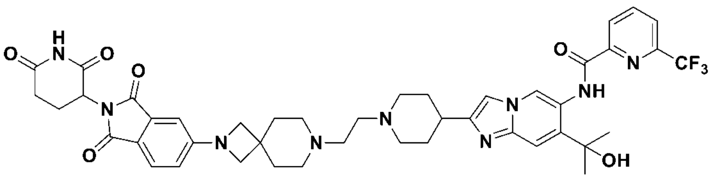

,

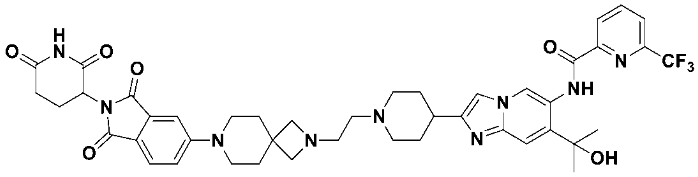

,

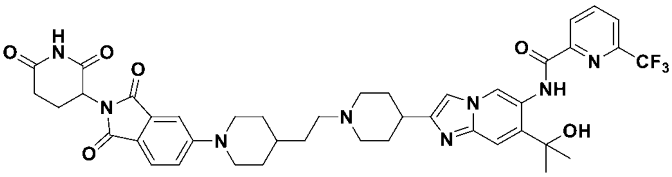

,

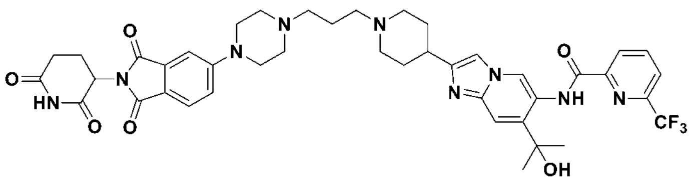

,

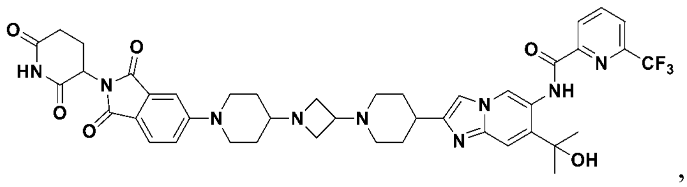
,

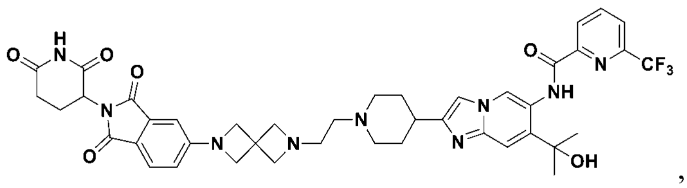
,

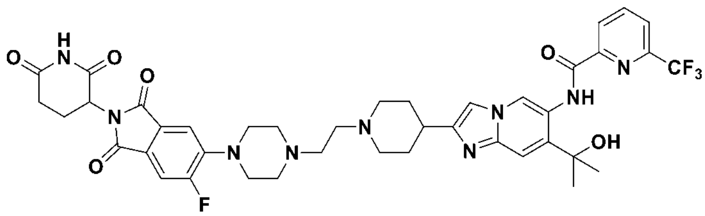
,

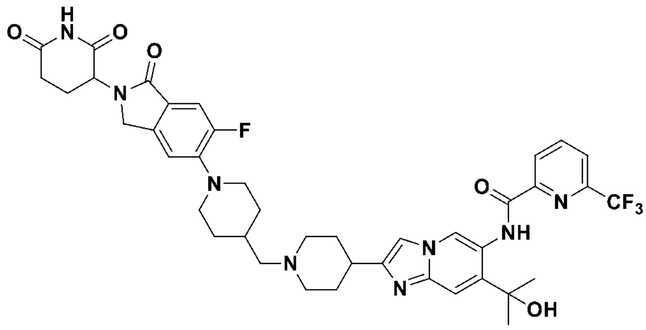
,

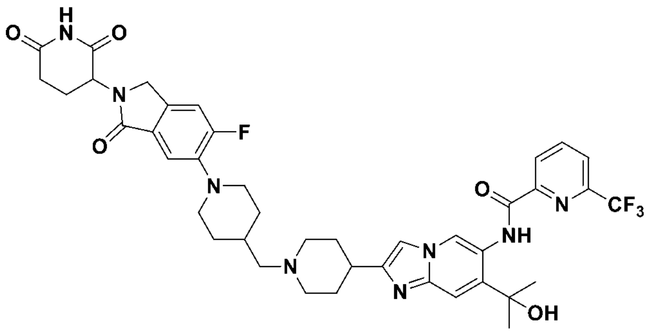
,

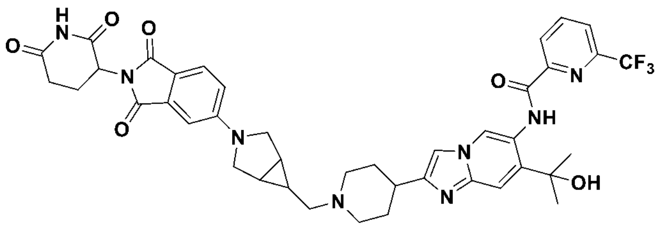
,

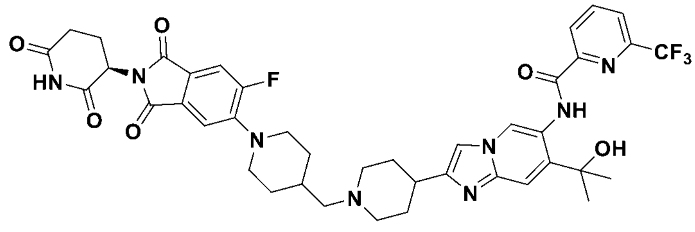

,

, or

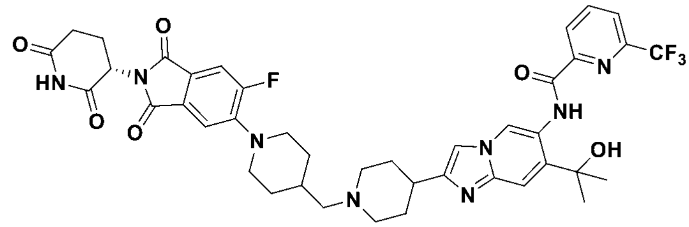

.

In a second aspect, the present application provides a pharmaceutically acceptable salt of the imidazo[1,2-a] pyridine IRAK4 degrader of the first aspect.

Preferably, the pharmaceutically acceptable salt comprises any one of the following salts: sulfate, pyrosulfate, bisulfate, sulfite, bisulfite, phosphate, monohydrogen phosphate, dihydrogen phosphate, metaphosphate, pyrophosphate, hydrochloride, hydrobromide, hydroiodide, acetate, propionate, decanoate, octanoate, acrylate, formate, isobutyrate, hexanoate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, $\gamma$-hydroxybutyrate, glycolate, tartrate, methanesulfonate, propanesulfonate, naphthalene-1-sulfonate, naphthalene-2-sulfonate, or mandelate.

In a third aspect, the present application provides a stereoisomer, geometric isomer, or tautomer of the imidazo [1,2-a]pyridine IRAK4 degrader of the first aspect.

In a fourth aspect, the present application provides a pharmaceutical composition. The pharmaceutical composition comprises the preceding imidazo[1,2-a]pyridine IRAK4 degrader or the stereoisomer, geometric isomer, tautomer, or pharmaceutically acceptable salt thereof.

In a fifth aspect, the present application provides use of the preceding imidazo[1,2-a]pyridine IRAK4 degrader, the stereoisomer, geometric isomer, tautomer, or pharmaceutically acceptable salt thereof, or the pharmaceutical composition for preparing a medicament for treating a disease or disorder mediated by IRAK4.

Preferably, the disease comprises an inflammatory disease, an autoimmune disease, or a tumor.

Preferably, the inflammatory disease is selected from Crohn's disease, ulcerative colitis, asthma, graft-versus-host disease, chronic obstructive pulmonary disease, atopic dermatitis, gout, gouty arthritis, conjunctivitis, hepatitis, chronic pulmonary inflammatory disease, thyroiditis, or interstitial cystitis.

Preferably, the autoimmune disease is selected from Graves' disease, rheumatoid arthritis, systemic lupus erythematosus, lupus nephritis, cutaneous lupus, psoriasis, psoriatic arthritis, cryopyrin-associated periodic syndrome, TNF receptor associated periodic syndrome, multiple sclerosis, allergic rhinitis, scleroderma, dermatomyositis, vasculitis, or nephritis.

Preferably, the tumor is selected from lung cancer, breast cancer, prostate cancer, pancreatic cancer, kidney cancer, liver cancer, gastrointestinal cancer, cervical cancer, endometrial carcinoma, testicular cancer, genitourinary tract cancer, colorectal cancer, laryngeal cancer, skin cancer, bone cancer, head and neck tumor, sarcoma, cerebroma, glioblastoma, melanoma, multiple myeloma, lymphoma, or leukemia.

Compared with the existing art, the present application has the beneficial effects below.

The compounds of the present application have a significant capability to degrade IRAK4 in THP1 cells. The half-maximal degradation concentration ($DC_{50}$) of some compounds can reach a nanomolar level. The results of a Western blotting experiment also show that the compounds of the present application have very strong IRAK4 protein degradation activity in OCI-LY10 cells at concentrations of 100 nM and 1000 nM.

In *in vitro* and *in vivo* metabolic stability studies, the compounds of the present application have very high metabolic stability in liver microsomes of different species. Moreover, in the pharmacokinetic studies in mice, the compounds of the present application have good pharmacokinetic properties such as low clearance, high plasma exposure, and high oral bioavailability.

BRIEF DESCRIPTION OF DRAWINGS

FIGS. 1 to 26 show the Western blotting results of compounds in Examples 1 to 26 on the degradation of IRAK4 proteins in OCI-LY10 cells at concentrations of 100 nM and 1000 nM, respectively, wherein "rabbit anti-IRAK4" indicates detection of IRAK4 proteins with a rabbit anti-IRAK4 antibody as a primary antibody, and "mouse anti-GAPDH" indicates detection of GAPDH with a mouse anti-GAPDH antibody as a primary antibody.

DETAILED DESCRIPTION

Technical solutions of the present application are further described below through examples. It is to be understood by those skilled in the art that the examples described below are intended to facilitate understanding of the present application and are not to be construed as limiting the present application.

In the following examples, a molecule with a single chiral center exists in the form of a racemic mixture unless otherwise noted by a structural formula or a chemical name. A molecule with two or more chiral centers exists in the form of a racemic mixture of diastereomers unless otherwise noted by a structural formula or a chemical name. Single enantiomers/diastereomers can be available by methods known to those skilled in the art.

**Preparation methods**

The compounds of the present application may be synthesized through synthesis solutions herein and/or techniques well-known in the art. For example, the compounds of the present application may be prepared by the general synthesis methods described below.

General Synthesis Method 1

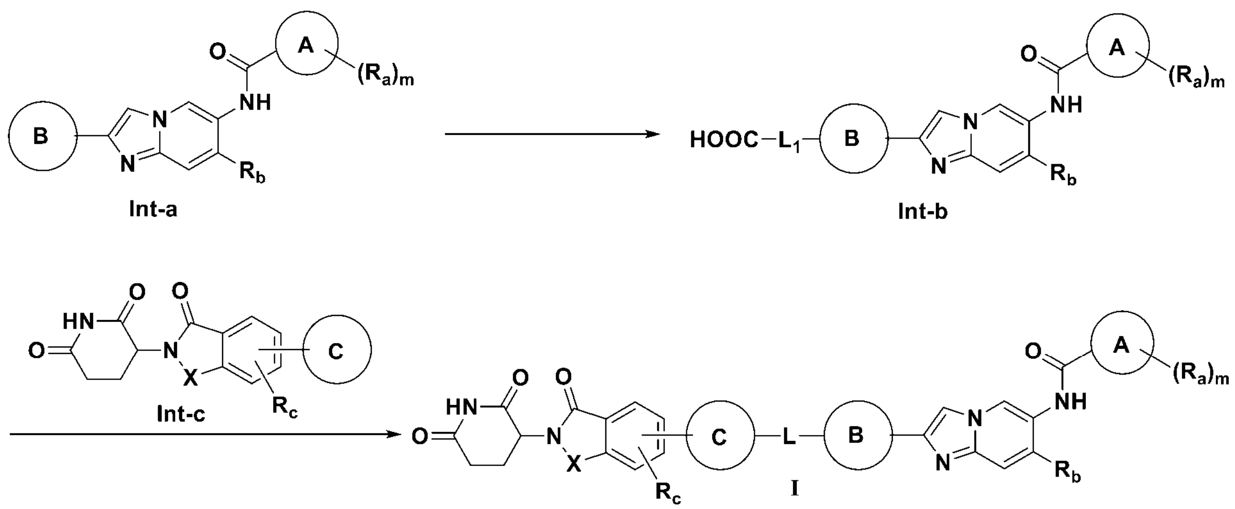
Int-a
Int-b
Int-c
I

Specifically, in general synthesis method 1, Intermediate **Int-a** undergoes a substitution reaction with HOOC-$L_1$-Y under a basic condition to obtain Intermediate **Int-b**. Alternatively, Intermediate **Int-a** undergoes a substitution reaction with t-BuOOC-$L_1$-Y under a basic condition, and the resulting product is then treated with trifluoroacetic acid or hydrochloric acid to remove t-butyl to obtain Intermediate **Int-b.** Intermediate **Int-b** undergoes a condensation reaction with Intermediate **Int-c** in the presence of a condensing agent and a base to obtain the compound of Formula I. The base is an inorganic or organic base, including, but not limited to, triethylamine, *N,N*-diisopropylethylamine, potassium carbonate, sodium carbonate, or sodium bicarbonate. Y is halogen and preferably bromine or iodine. Ra, Rb, Rc, m, the ring A, the ring B, the ring C, L, and X are defined as above.

General Synthesis Method 2

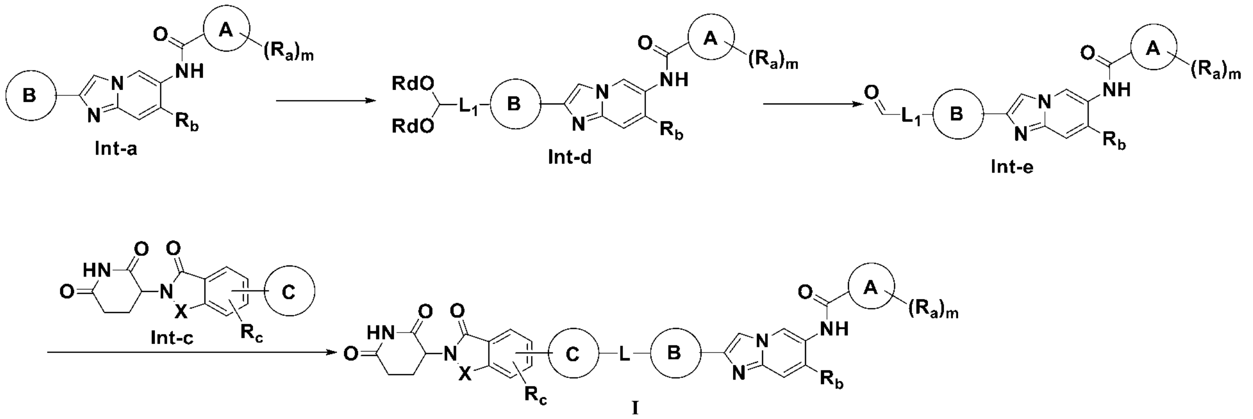

Int-a Int-d Int-e Int-c I

Specifically, in general synthesis method 2, Intermediate **Int-a** undergoes a substitution reaction with $(RdO)_2CH-L_1-Y$ under a basic condition to obtain Intermediate **Int-d;** Intermediate **Int-d** reacts with trifluoroacetic acid or hydrochloric acid to obtain Intermediate **Int-e**; and Intermediate **Int-e** then undergoes a reductive amination reaction with Intermediate **Int-c** to obtain the compound of Formula I. The base is an inorganic or organic base, including, but not limited to, triethylamine, *N*,*N*-diisopropylethylamine, potassium carbonate, sodium carbonate, or sodium bicarbonate. Y is halogen and preferably bromine or iodine. A reducing agent for the reductive amination reaction includes, but is not limited to, Pd/C, sodium borohydride, sodium cyanoborohydride, borane, or sodium triacetoxyborohydride. Rd is methyl or ethyl. Ra, Rb, Rc, m, the ring A, the ring B, the ring C, L, and X are defined as above.

General Synthesis Method 3

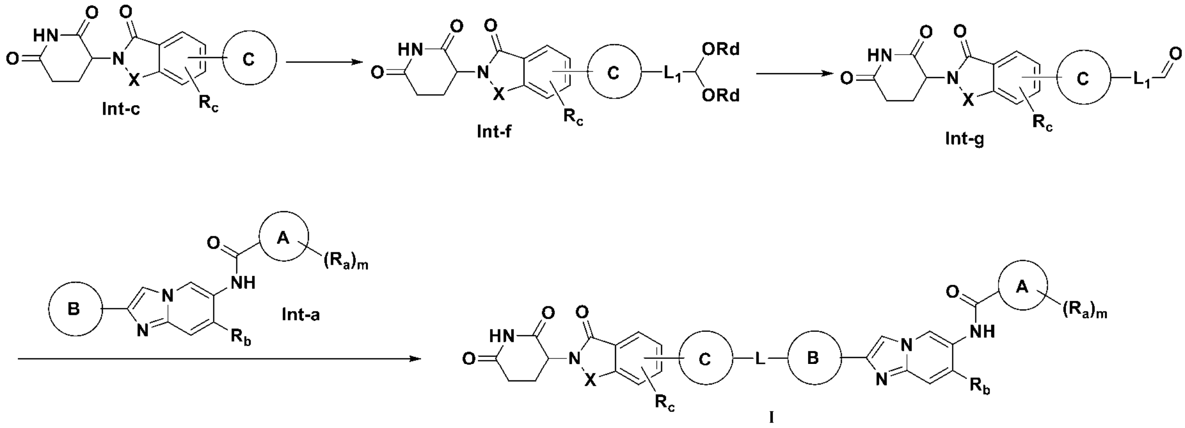

Int-c Int-f Int-g Int-a I

Specifically, in general synthesis method 3, Intermediate **Int-c** undergoes a substitution reaction with $(RdO)_2CH-L_1-Y$ under a basic condition to obtain Intermediate **Int-f;** Intermediate **Int-f** reacts with trifluoroacetic acid or hydrochloric acid to obtain Intermediate **Int-g**; and Intermediate **Int-g** then undergoes a reductive amination reaction with Intermediate **Int-a** to obtain the compound of Formula I. The base is an inorganic or organic base, including, but not limited to, triethylamine, *N*,*N*-diisopropylethylamine, potassium carbonate, sodium carbonate, or sodium bicarbonate. Y is halogen and preferably bromine or iodine. A reducing agent for the reductive amination reaction includes, but is not limited to, Pd/C, sodium borohydride, sodium cyanoborohydride, borane, or sodium triacetoxyborohydride. Rd is methyl or ethyl. Ra, Rb, Rc, m, the ring A, the ring B, the ring C, L, and X are defined as above.

General Synthesis Method 4

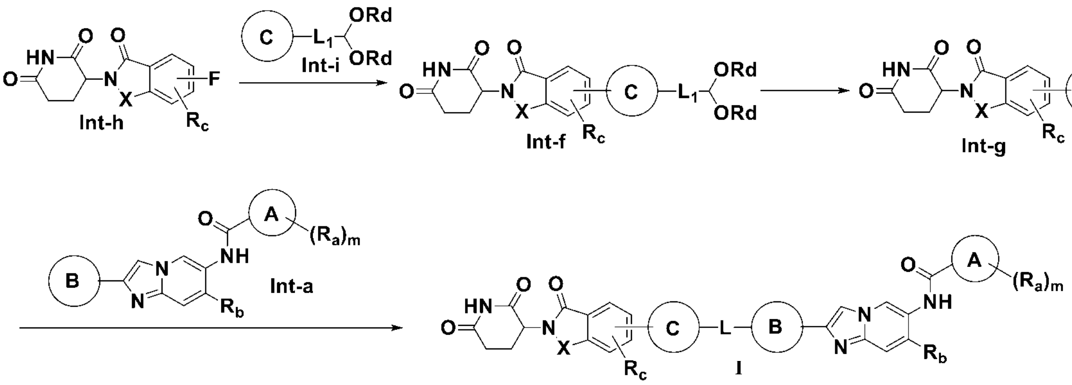


Specifically, in general synthesis method 4, Intermediate **Int-h** undergoes a substitution reaction with Intermediate **Int-i** under a basic condition to obtain Intermediate **Int-f**; Intermediate **Int-f** reacts with trifluoroacetic acid or hydrochloric acid to obtain Intermediate **Int-g;** and Intermediate **Int-g** then undergoes a reductive amination reaction with Intermediate **Int-a** to obtain the compound of Formula I. The base is an inorganic or organic base, including, but not limited to, triethylamine, *N*,*N*-diisopropylethylamine, potassium carbonate, sodium carbonate, or sodium bicarbonate. A reducing agent for the reductive amination reaction includes, but is not limited to, Pd/C, sodium borohydride, sodium cyanoborohydride, borane, or sodium triacetoxyborohydride. Rd is methyl or ethyl. $R_a$, $R_b$, $R_c$, m, the ring A, the ring B, the ring C, L, and X are defined as above.

General Synthesis Method 5

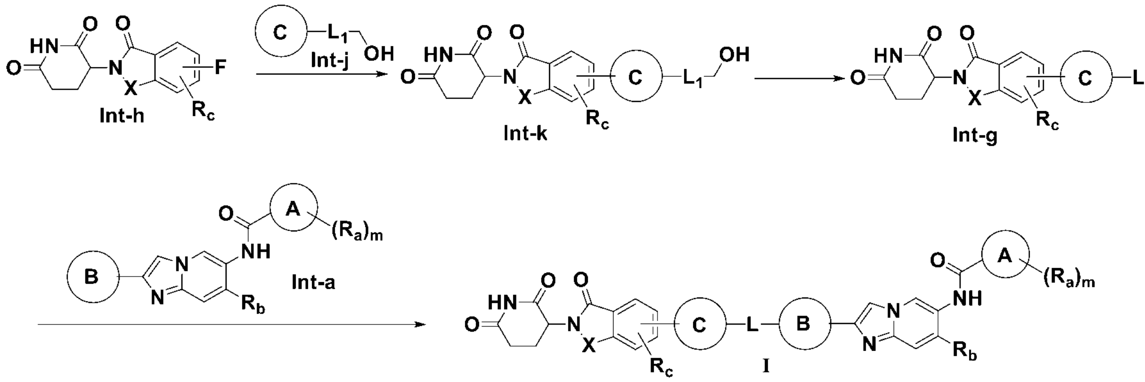


Specifically, in general synthesis method 5, Intermediate **Int-h** undergoes a substitution reaction with Intermediate **Int-j** under a basic condition to obtain Intermediate **Int-k;** Intermediate **Int-k** reacts with an oxidizing agent to obtain Intermediate **Int-g;** and Intermediate **Int-g** then undergoes a reductive amination reaction with Intermediate **Int-a** to obtain the compound of Formula I. The base is an inorganic or organic base, including, but not limited to, triethylamine, *N,N*-diisopropylethylamine, potassium carbonate, sodium carbonate, or sodium bicarbonate. A reducing agent for the reductive amination reaction includes, but is not limited to, Pd/C, sodium borohydride, sodium cyanoborohydride, borane, or sodium triacetoxyborohydride. $R_a$, $R_b$, $R_c$, m, the ring A, the ring B, the ring C, L, and X are defined as above.

General Synthesis Method 6

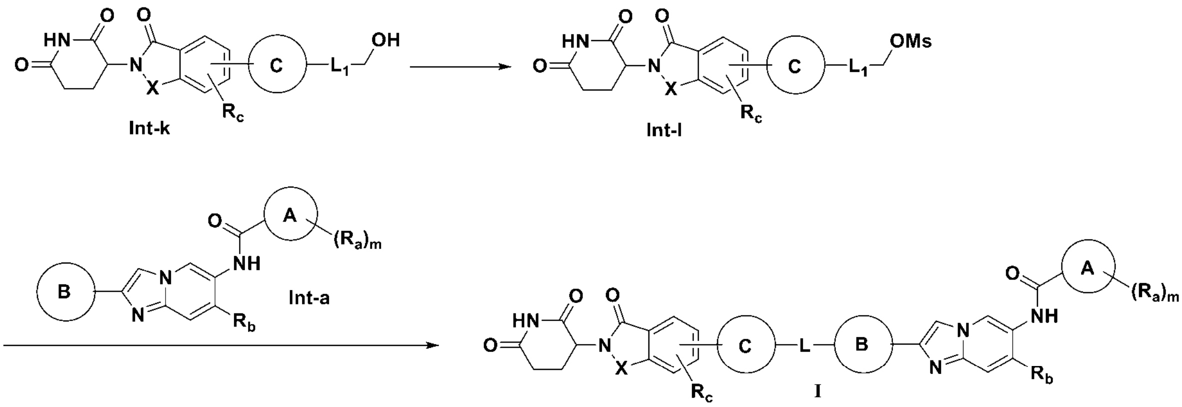

Int-k Int-l I

Specifically, in general synthesis method 6, Intermediate **Int-k** reacts with methanesulfonyl chloride under a basic condition to obtain Intermediate **Int-l**; and Intermediate **Int-l** then undergoes a substitution reaction with Intermediate **Int-a** under a basic condition to obtain the compound of Formula I. The base is an inorganic or organic base, including, but not limited to, triethylamine, *N,N*-diisopropylethylamine, potassium carbonate, sodium carbonate, or sodium bicarbonate. $R_a$, $R_b$, $R_c$, m, the ring A, the ring B, the ring C, L, and X are defined as above.

General Synthesis Method 7

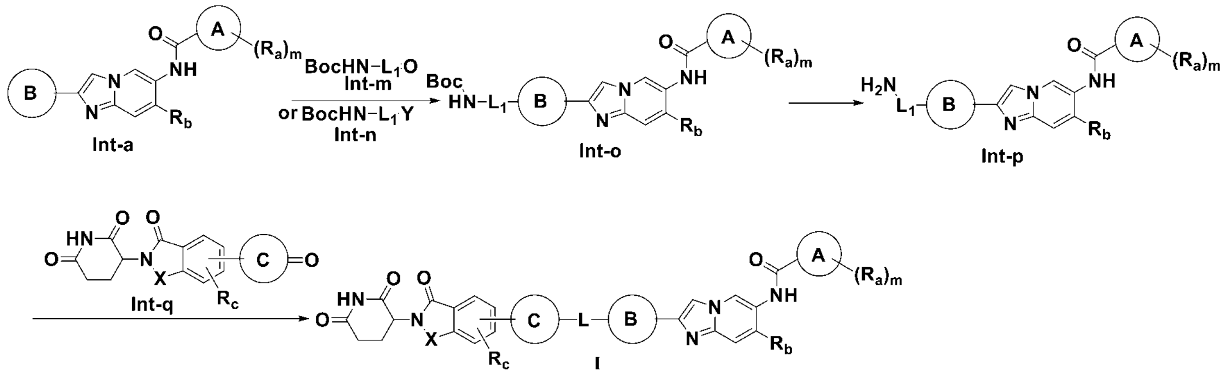

Int-a Int-o Int-p I

Specifically, in general synthesis method 7, Intermediate **Int-a** undergoes a reductive amination reaction with Intermediate **Int-m,** or Intermediate **Int-a** undergoes a substitution reaction with Intermediate **Int-n** under a basic condition to obtain Intermediate **Int-o**; Intermediate **Int-o** reacts with trifluoroacetic acid or hydrochloric acid to obtain Intermediate **Int-p;** and Intermediate **Int-p** then undergoes a reductive amination reaction with Intermediate **Int-q** to obtain the compound of Formula I. The base is an inorganic or organic base, including, but not limited to, triethylamine, *N,N*-diisopropylethylamine, potassium carbonate, sodium carbonate, or sodium bicarbonate. A reducing agent for the reductive amination reaction includes, but is not limited to, Pd/C, sodium borohydride, sodium cyanoborohydride, borane, or sodium triacetoxyborohydride. Ra, Rb, Rc, m, the ring A, the ring B, the ring C, L, and X are defined as above.

## Preparation examples

The compounds of the present application may be synthesized through one or more synthesis solutions herein and/or techniques well-known in the art. It is to be appreciated by those skilled in the art that the synthesis methods of some examples described in detail in the present application can be readily applied to the synthesis of other examples. In some examples, the compounds described herein may be prepared through appropriate combinations of synthesis methods well-known in the art. Many starting materials and other reagents may be commercially available from suppliers such as Alfa Aesar (China) Chemical Co., Ltd. or may be readily prepared by synthesis methods commonly used in the art.

$^1H$ NMR spectra are recorded on an instrument operating at 400 MHz. $^1H$ NMR spectra are obtained in the form of solutions (reported in ppm). When peak multiplicity is reported, the following abbreviations are used: s (singlet), d (doublet), t (triplet), q (quartet), m (multiplet), br (broad), dd (double doublet), and dt (double triplet). Coupling constants are given in hertz (Hz).

Where necessary, (*R*)- and (*S*)-isomers of a non-limiting example compound, if present, may be resolved by methods known to those skilled in the art, for example, diastereomeric salts or complexes are formed and may be separated through, for example, crystallization; diastereomeric derivatives are formed and may be separated through, for

example, crystallization or chromatography; one enantiomer reacts selectively with an enantiomer-specific reagent, and modified and unmodified enantiomers are separated; or separation is performed through chromatography in a chiral environment such as a chiral column. Alternatively, specific enantiomers may be asymmetrically synthesized by using optically active reagents, substrates, catalysts, or solvents or may be prepared through asymmetric transformation of one enantiomer into another.

**Preparation of compounds**

**Intermediate** 1: ***N*-(7-(2-hydroxypropan-2-yl)-2-(piperidin-4-yl)imidazo[1,2-a]pyridin-6-yl)-6-(trifluorometh yl) pyridine-2-carboxamide hydrochloride (IM-1)**

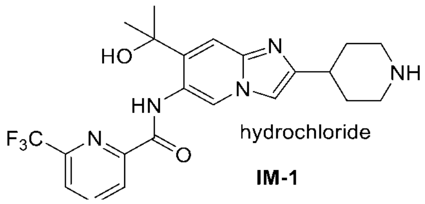

hydrochloride

**IM-1**

**Step 1: Preparation of methyl 6-bromo-2-(1-(*t*-butoxycarbonyl)piperidin-4-yl)imidazo[1,2-a]pyridine-7-carboxylate (IM-1a)**

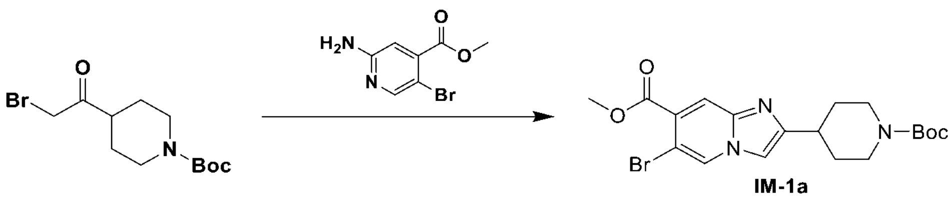

**IM-1a**

T-butyl 4-(2-bromoacetyl)piperidine-1-carboxylate (25.0 g, 81.6 mmol) and methyl 2-amino-5-bromonicotinate (18.8 g, 81.6 mmol) were dissolved in acetonitrile (150 mL) and toluene (100 mL). Sodium bicarbonate (13.6 g, 163 mmol) was added to the mixture at 25°C. The reaction mixture was heated to 90°C and stirred for 12 h at 90°C. Thin-layer chromatography showed that the reaction was completed. The reaction mixture was poured into ice water (500 mL) and stirred for 10 min. An aqueous phase was extracted with ethyl acetate (300 mL × 3). Combined organic phases were washed with brine (500 mL), dried over anhydrous sodium sulfate, filtered, and concentrated in vacuo. The resulting product was purified through column chromatography (silica gel:petroleum ether:ethyl acetate = 100:1-0:1 (v:v:v)) to obtain Compound **IM-1a** as a dark brown solid (20.0 g).

**Step 2: Preparation of methyl 2-(1-(*t*-butoxycarbonyl)piperidin-4-yl)-6-(6-(trifluoromethyl)pyridine-2-carboxamido)imid azo[1,2-a]pyridine-7-carboxylate (IM-1b)**

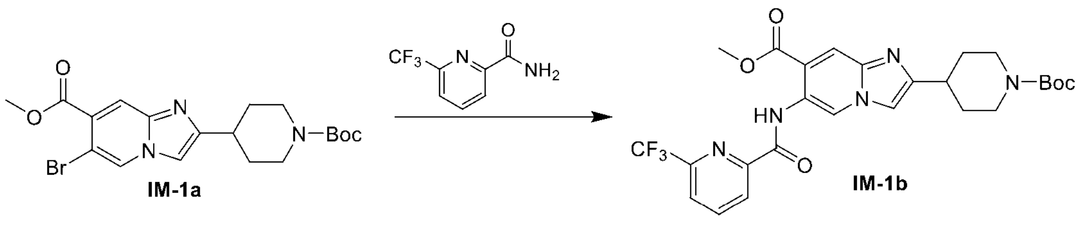

**IM-1a** **IM-1b**

Compound **IM-1a** (20.0 g, 45.6 mmol) and 6-(trifluoromethyl)pyridine-2-carboxamide (26.0 g, 136 mmol) were dissolved in dioxane (200.0 mL). Cesium carbonate (29.7 g, 90.7 mmol), 4,5-bis(diphenylphosphino)-9,9-dimethylxanthene (10.5 g, 18.2 mmol), and palladium acetate (2.04 g, 9.13 mmol) were added to the reaction mixture at 25°C under

nitrogen protection. The reaction mixture was heated to 100°C and stirred for 12 h at 100°C. The reaction mixture was cooled to 25°C and concentrated under reduced pressure. The residue was poured into ice water (300 mL) and stirred for 5 min. An aqueous phase was extracted with ethyl acetate (300 mL × 3). Combined organic phases were washed with brine (500 mL), dried over anhydrous sodium sulfate, filtered, and concentrated in vacuo. The residue was purified through column chromatography (silica gel:petroleum ether:ethyl acetate = 100:1-0:1 (v:v:v)) to obtain Compound **IM-1b** as a yellow solid (10.0 g).

**Step 3: Preparation of t-butyl 4-(7-(2-hydroxypropan-2-yl)-6-(6-(trifluoromethyl)pyridine-2-carboxamido)imidazo[1,2-a] pyridin-2-yl)piperidine-1-carboxylate (IM-1c)**

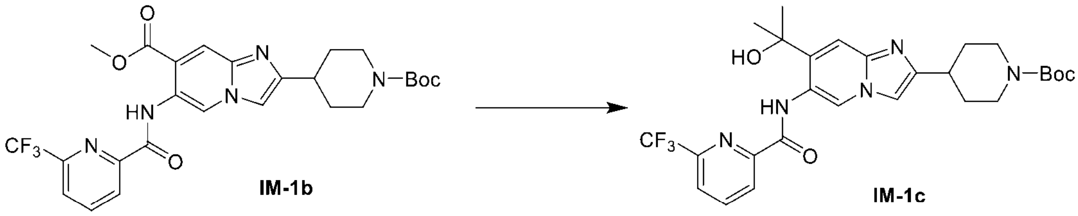

**IM-1b** **IM-1c**

Compound **IM-1b** (10.0 g, 18.1 mmol) was dissolved in tetrahydrofuran (100 mL). The reaction mixture was cooled to 0°C, and methylmagnesium bromide (3 M, 36.5 mL) was slowly added dropwise. After addition, the system was slowly heated to 25°C and stirred for 3 h at 25°C. The reaction was quenched with an aqueous solution of ammonium chloride (200 mL) and extracted with ethyl acetate (3 × 100 mL). Combined organic layers were dried over anhydrous magnesium sulfate, filtered, and concentrated in vacuo. The residue was purified through column chromatography (silica gel:petroleum ether:ethyl acetate = 100:1-0:1 (v:v:v)) to obtain Compound **IM-1c** as a white solid (4.00 g, crude product).

**Step 4: Preparation of *N*-(7-(2-hydroxypropan-2-yl)-2-(piperidin-4-yl)imidazo[1,2-a]pyridin-6-yl)-6-(trifluorometh yl)pyridine-2-carboxamide hydrochloride (IM-1)**

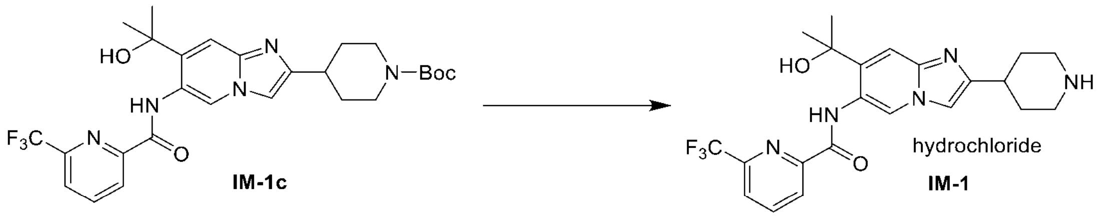

**IM-1c** hydrochloride **IM-1**

Compound **IM-1c** (4.00 g, 7.32 mmol) was dissolved in dioxane (40.0 mL). The system was cooled to 0°C, and hydrochloric acid-dioxane (4.00 M, 2.00 equivalents) was slowly added to the system. After addition, the system was slowly heated to 25°C and stirred for 3 h at 25°C. The reaction mixture was concentrated in vacuo to obtain Compound **IM-1** as a white solid (3.00 g, crude product, hydrochloride).

**Intermediate 2: *N*-(7-methoxy-2-(piperidin-4-yl)imidazo[1,2-a]pyridin-6-yl)-6-(trifluoromethyl)pyridine-2-c arboxamide**

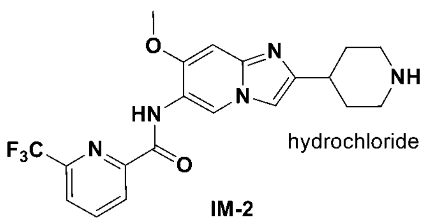

hydrochloride

**IM-2**

**Step 1: Preparation of *t*-butyl 4-(6-bromo-7-methoxyimidazo[1,2-a]pyridin-2-yl)piperidine-1-carboxylate (IM-2a)**

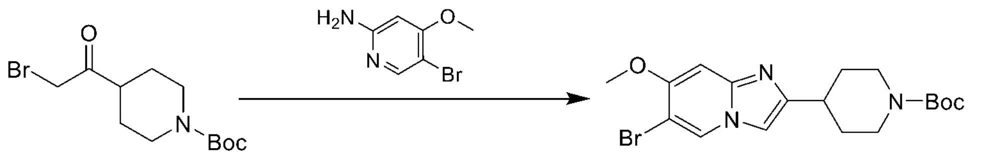

**IM-2a**

*T*-butyl 4-(2-bromoacetyl)piperidine-1-carboxylate (6.00 g, 19.6 mmol) was dissolved in toluene (13.9 mL) and acetonitrile (27.9 mL). Sodium bicarbonate (3.29 g, 39.2 mmol) and 5-bromo-4-methoxy-2-aminopyridine (3.98 g, 19.6 mmol) were added at room temperature. Then, the reaction was heated to 90°C and stirred for 12 h. The reaction mixture was added with water (50 mL) and extracted with ethyl acetate (50 mL × 3). Organic phases were combined, washed with saturated brine, dried over anhydrous sodium sulfate, filtered, and concentrated under reduced pressure. The crude product was purified through column chromatography (silica gel:petroleum ether:ethyl acetate = 100:1-10:1 (v:v:v)) to obtain Intermediate **IM-2a** as a yellow solid (4.00 g).

**Step 2: Preparation of t-butyl 4-(7-methoxy-6-(6-(trifluoromethyl)pyridine-2-carboxamido)imidazo[1,2-a]pyridin-2-yl)pip eridine-1-carboxylate (IM-2b)**

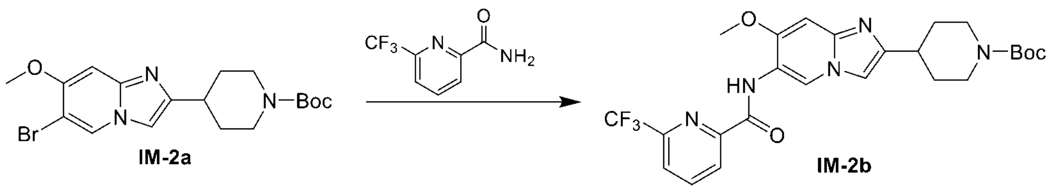

**IM-2a** **IM-2b**

Compound **IM-2a** (4.40 g, 10.7 mmol) and 6-(trifluoromethyl)pyridine-2-carboxamide (4.08 g, 21.4 mmol) were dissolved in 1,4-dioxane (40.0 mL). Under nitrogen protection at room temperature, 4,5-bis(diphenylphosphino)-9,9-dimethylxanthene (2.48 g, 4.29 mmol), cesium carbonate (6.99 g, 21.4 mmol), and palladium acetate (481 mg, 2.14 mmol) were added in sequence. The reaction mixture was heated to 100°C and stirred for 12 h. The reaction mixture was cooled to room temperature, diluted with water (40.0 mL), and extracted with ethyl acetate (50.0 mL × 3). Organic phases were combined, washed with saturated brine, dried over anhydrous sodium sulfate, filtered, and concentrated under reduced pressure. The residue was purified through column chromatography (silica gel:petroleum ether:ethyl acetate = 100:1-10:1 (v:v:v)) to obtain **IM-2b** as a yellow solid (3.00 g).

$^1$H NMR (400 MHz, DMSO-$d_6$) δ 10.19 (s, 1H), 9.34 (s, 1H), 8.36-8.48 (m, 2H), 8.12-8.27 (m, 1H), 7.66 (s, 1H), 7.08 (s, 1H), 3.98 (s, 5H), 2.71-2.97 (m, 3H), 1.90-2.01 (m, 2H), 1.56-1.46 (m, 2H), 1.41 (s, 9H).

**Step** 3: **Preparation of *N*-(7-methoxy-2-(piperidin-4-yl)imidazo[1,2-a]pyridin-6-yl)-6-(trifluoromethyl)pyridine-2-c arboxamide (IM-2)**

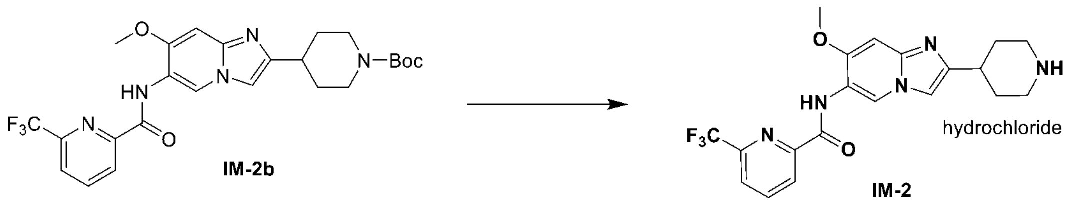

**IM-2b**

hydrochloride

**IM-2**

Compound **IM-2b** (500 mg, 1.06 mmol) was dissolved in 1,4-dioxane (5.00 mL). Hydrochloric acid/1,4-dioxane (4 M, 12.0 mL) was added. The reaction mixture was stirred for 3 h at 25°C. The reaction mixture was concentrated to dryness under reduced pressure to obtain Intermediate **IM-2** as a yellow solid (400 mg, crude product).

**Intermediate 3: 2-(4-(7-(2-hydroxypropan-2-yl)-6-(6-(trifluoromethyl)pyridine-2-carboxamido)imidazo[1,2-a]pyridin-2-yl)piperidin-1-yl)acetic acid (IM-3)**

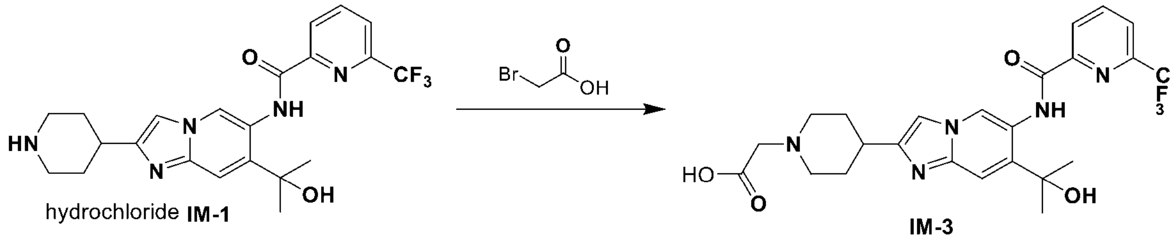

hydrochloride **IM-1** **IM-3**

Compound **IM-1** (500 mg, 1.03 mmol) and 2-bromoacetic acid (215 mg, 1.55 mmol) were dissolved in *N,N*-dimethylformamide (5.00 mL). Triethylamine (313 mg, 3.10 mmol) was added to the reaction mixture at 25°C. The reaction mixture was stirred for 2 h at 25°C. The reaction mixture was concentrated under reduced pressure to obtain Compound **IM-3** as a yellow solid (550 mg, crude product), which was used directly without further purification.

**Intermediate 4: 3-(4-(7-(2-hydroxypropan-2-yl)-6-(6-(trifluoromethyl)pyridine-2-carboxamido)imidazo[1,2-a]pyridin-2-yl)piperidin-1-yl)propionic acid (IM-4)**

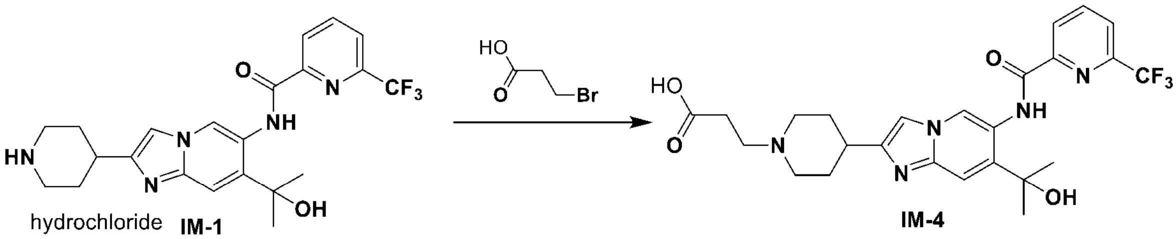

hydrochloride **IM-1** **IM-4**

Intermediate **IM-1** (150 mg, 309 μmol) and 3-bromopropionic acid (71.1 mg, 464 μmol) were dissolved in dichloromethane (2.00 mL). Triethylamine (94.1 mg, 929 μmol) was added to the reaction mixture at 25°C. The reaction was stirred for 12 h at 25°C. The reaction mixture was concentrated under reduced pressure to obtain Compound **IM-4** as a yellow solid (210 mg, crude product), which was used directly without further purification.

**Intermediate 5: 4-(4-(7-(2-hydroxypropan-2-yl)-6-(6-(trifluoromethyl)pyridine-2-carboxamido)imidazo[1,2-a]pyridin-2-yl)piperidin-1-yl)butanoic acid (IM-5)**

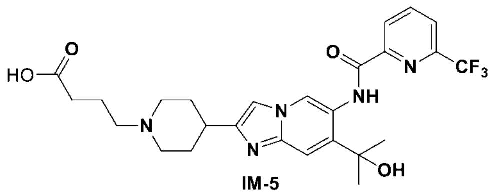

**IM-5**

**Step 1: Preparation of t-butyl 4-(4-(7-(2-hydroxypropan-2-yl)-6-(6-(trifluoromethyl)pyridine-2-carboxamido)imidazo[1,2-a]pyridin-2-yl)piperidin-1-yl)butanoate (IM-5a)**

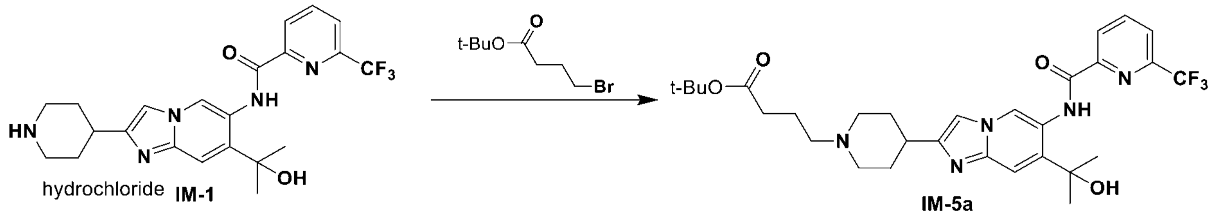


Compound 1 (300 mg, 670 μmol) was dissolved in acetone (3.00 mL), added with t-butyl 4-bromobutanoate (299 mg, 1.34 mmol) and potassium carbonate (277 mg, 2.01 mmol) at 25°C, and heated to 60°C and stirred for 12 h. The reaction mixture was concentrated to dryness under reduced pressure. The residue was purified through preparative HPLC (column: Waters Xbridge BEH C18 100×30 mm × 10 μm; mobile phase: [$H_2O$ ($NH_4HCO_3$)-ACN]; B%: 35%-65%, 8 min) to obtain Intermediate **IM-5a** as an off-white solid (150 mg, crude product).

**Step 2: Preparation of 4-(4-(7-(2-hydroxypropan-2-yl)-6-(6-(trifluoromethyl)pyridine-2-carboxamido)imidazo [1,2-a]pyridin-2-yl)piperidin-1-yl)butanoic acid (IM-5)**

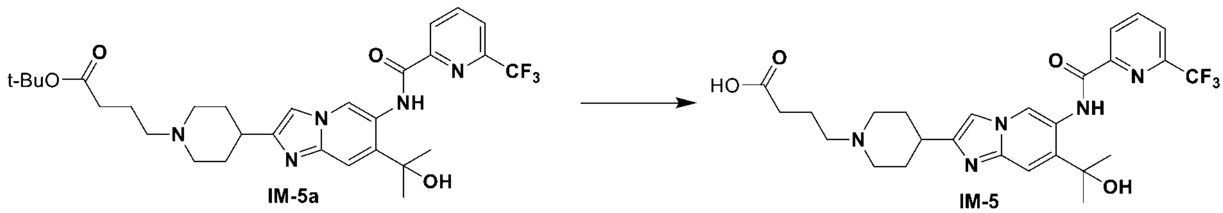


Intermediate **IM-5a** (120 mg, 203 μmol) was dissolved in dichloromethane (2.00 mL), added with trifluoroacetic acid (1.30 g, 11.4 mmol), and stirred for 12 h at 25°C. The reaction mixture was concentrated to dryness under reduced pressure to obtain Intermediate **IM-5** as a yellow oily compound (100 mg, crude product), which was used directly without further purification.

**Intermediate 6: *N*-(7-(2-hydroxypropan-2-yl)-2-(1-(2-oxoethyl)piperidin-4-yl)imidazo[1,2-a]pyridin-6-yl)-6-(trifluoromethyl)pyridine-2-carboxamide (IM-6)**

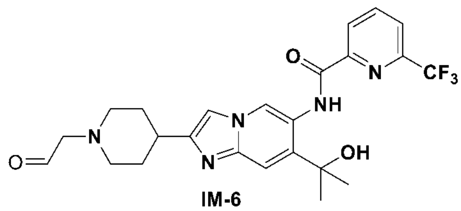

IM-6

**Step 1: Preparation of *N*-(2-(1-(2,2-diethoxyethyl)piperidin-4-yl)-7-(2-hydroxypropan-2-yl)imidazo[1,2-a]pyridin-6-yl)-6-(trifluoromethyl)pyridine-2-carboxamide (IM-6a)**

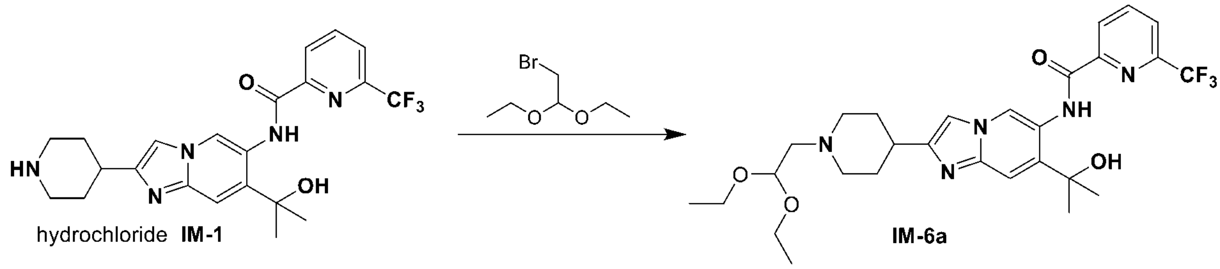

hydrochloride **IM-1** **IM-6a**

Compound **IM-1** (1.00 g, 2.23 mmol) and 2-bromo-1,1-diethoxyethane (880 mg, 4.47 mmol) were dissolved in acetone (2.00 mL). Potassium carbonate (926 mg, 6.70 mmol) and potassium iodide (185 mg, 1.12 mmol) were added in sequence. The obtained mixture was heated to 80°C and stirred for 12 h. The mixture was concentrated to dryness under reduced pressure to obtain Intermediate **IM-6a** as a yellow solid (300 mg).

**Step 2: Preparation of *N*-(7-(2-hydroxypropan-2-yl)-2-(1-(2-oxoethyl)piperidin-4-yl)imidazo[1,2-a]pyridin-6-yl)-6-(trifluoromethyl)pyridine-2-carboxamide (IM-6)**

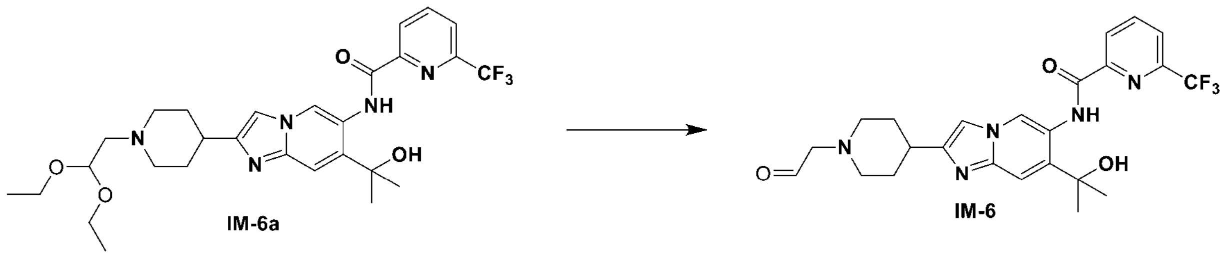

**IM-6a** **IM-6**

Compound **IM-6a** (300 mg, 532 μmol) was dissolved in water (3.00 mL). Hydrochloric acid/1,4-dioxane (4 M, 3.00 mL) was slowly added dropwise to the system at 25°C. The mixture was stirred for 12 h at 60°C and concentrated to dryness in vacuo to obtain Intermediate **IM-6** as a white solid (430 mg).

**Intermediate 7: *N*-(7-methoxy-2-(1-(2-oxoethyl)piperidin-4-yl)imidazo[1,2-a]pyridin-6-yl)-6-(trifluoromethy l) pyridine-2-carboxamide (IM-7)**

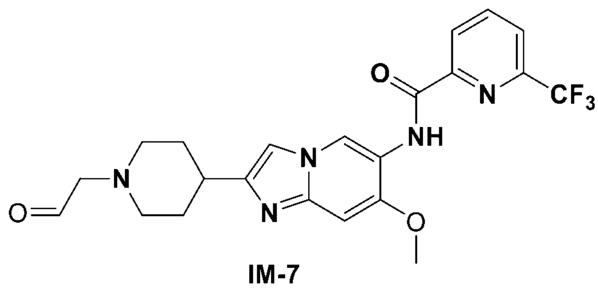

**IM-7**

**Step 1: Preparation of *N*-(2-(1-(2,2-diethoxyethyl)piperidin-4-yl)-7-methoxyimidazo[1,2-a]pyridin-6-yl)-6-(trifluor omethyl)pyridine-2-carboxamide (IM-7a)**

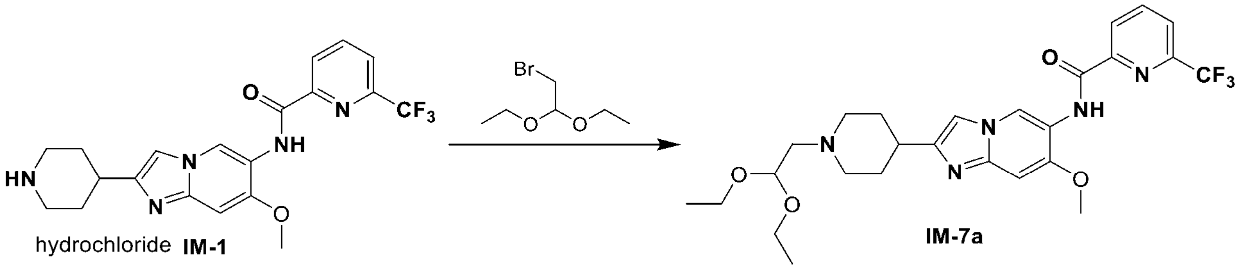

hydrochloride **IM-1**

**IM-7a**

At room temperature, Intermediate **IM-1** (500 mg, 1.19 mmol) was dissolved in *N,N*-dimethylformamide (5.00 mL) and added with 2-bromo-1,1-diethoxyethane (469 mg, 2.38 mmol) and *N,N*-diisopropylethylamine (462 mg, 3.58 mmol). The mixture was subjected to a microwave reaction for 2 h at 100°C. The reaction mixture was concentrated to dryness under reduced pressure and purified through preparative HPLC (column: Phenomenex C18 75×30 mm × 3 μm; mobile phase: [$H_2O$ ($NH_4HCO_3$)-ACN]; B%: 35%-65%, 8 min) to obtain Intermediate **IM-7a** as a white solid (150 mg).

**Step 2: Preparation of *N*-(7-methoxy-2-(1-(2-oxoethyl)piperidin-4-yl)imidazo[1,2-a]pyridin-6-yl)-6-(trifluoromethy l)pyridine-2-carboxamide (IM-7)**

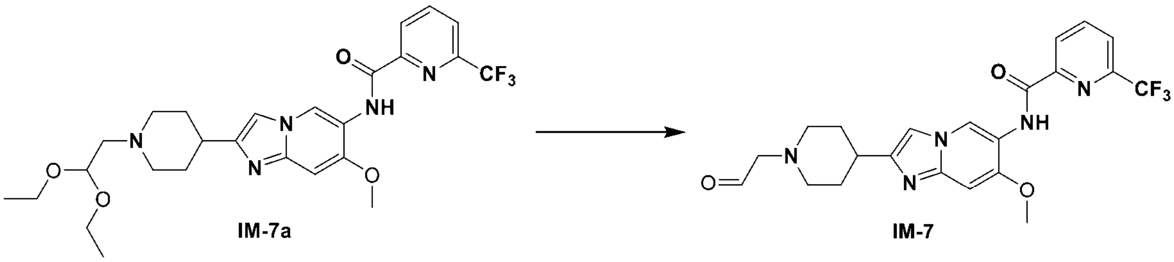

**IM-7a**

**IM-7**

Compound **2** (150 mg, 280 μmol) was added to water (2.00 mL), and hydrochloric acid/dioxane (4.00 M, 2.00 mL) was slowly added at room temperature. The reaction mixture was heated to 50°C and stirred for 12 h. The reaction mixture was concentrated to dryness to obtain Intermediate **IM-7** (140 mg, crude product), which was used directly in the next step without further purification.

**Intermediate 8: *N*-(2-(1-(azetidin-3-yl)piperidin-4-yl)-7-(2-hydroxypropan-2-yl)imidazo[1,2-a]pyridin-6-yl)-6-(trifluoromethyl)pyridine-2-carboxamide (IM-8)**

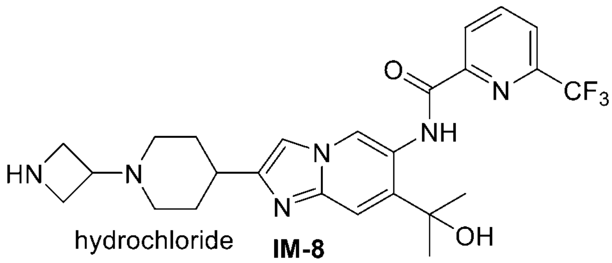

hydrochloride **IM-8**

**Step 1: Preparation of t-butyl 3-(4-(7-(2-hydroxypropan-2-yl)-6-(6-(trifluoromethyl)pyridine-2-carboxamido)imidazo[1,2-a]pyridin-2-yl)piperidin-1-yl)azetidine-1-carboxylate (IM-8a)**

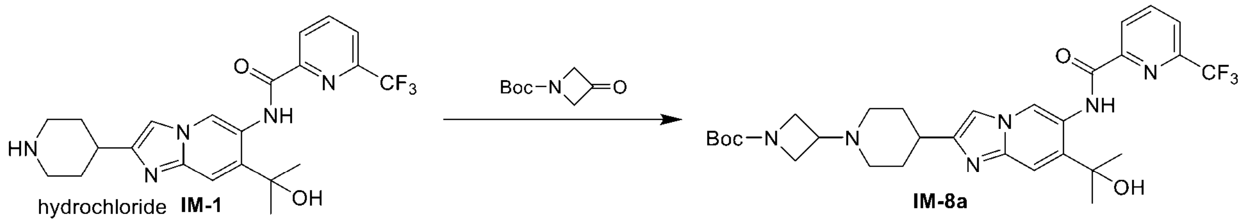

hydrochloride **IM-1** **IM-8a**

Intermediate **IM-1** (300 mg, 670 μmol) and t-butyl 3-oxoazetidine-1-carboxylate (114 mg, 670 μmol) were dissolved in 1,2-dichloroethane (3.00 mL) and *N,N*-dimethylformamide (3.00 mL). Triethylamine (203 mg, 2.01 mmol) was added to the reaction mixture at 25°C under nitrogen protection. The reaction mixture was stirred for 30 min at 25°C. Sodium triacetoxyborohydride (284 mg, 1.34 mmol) and acetic acid (40.2 mg, 670 μmol) were added to the reaction mixture. The reaction mixture was stirred for 11.5 h at 25°C. The reaction was quenched with water (5.00 mL) and extracted with ethyl acetate (10.0 mL × 3). Combined organic layers were dried over anhydrous sodium sulfate, filtered, and concentrated under reduced pressure. The residue was purified through high-performance liquid chromatography (HPLC) (in a formic acid system) to obtain Intermediate **IM-8a** as a yellow solid (100 mg).

**Step 2: Preparation of *N*-(2-(1-(azetidin-3-yl)piperidin-4-yl)-7-(2-hydroxypropan-2-yl)imidazo[1,2-a]pyridin-6-yl)-6-(trifluoromethyl)pyridine-2-carboxamide (IM-8)**

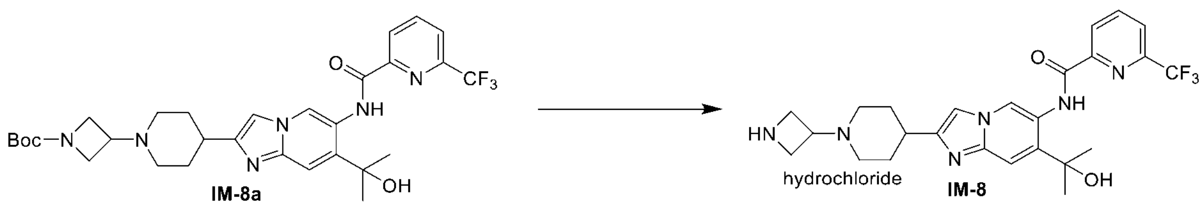

**IM-8a** hydrochloride **IM-8**

Compound **IM-8a** (100 mg, 165 μmol) was dissolved in dioxane (1.00 mL). Hydrochloric acid/dioxane (4.00 M, 166 μL) was added to the reaction mixture at 25°C. The reaction mixture was stirred for 12 h at 25°C. The reaction mixture was concentrated to dryness under reduced pressure to obtain Intermediate **IM-8** as a white solid (100.0 mg, hydrochloride).

**Intermediate 9: 1-(2-(2,6-dioxopiperidin-3-yl)-1,3-dioxoisoindolin-5-yl)piperidine-4-carbaldehyde (IM-9)**

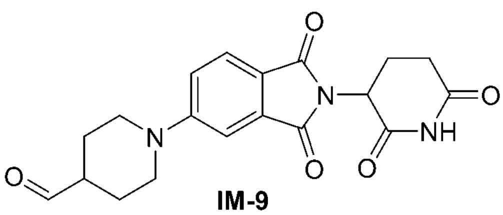

**IM-9**

**Step 1: Preparation of 5-(4-(dimethoxymethyl)piperidin-1-yl)-2-(2,6-dioxopiperidin-3-yl)isoindoline-1,3-dione (IM-9a)**

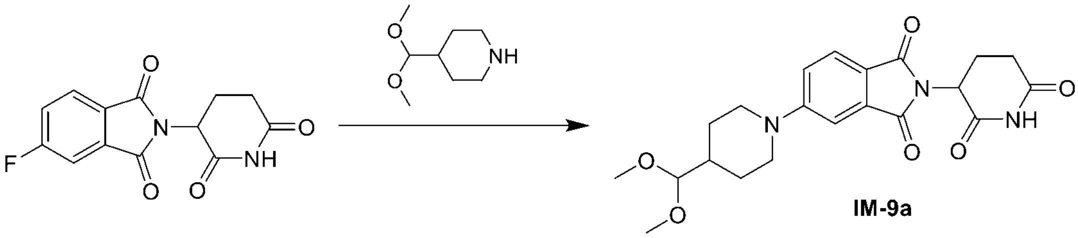

IM-9a

At 25°C, 2-(2,6-dioxopiperidin-3-yl)-5-fluoroisoindoline-1,3-dione (4.00 g, 14.4 mmol) was dissolved in dimethylsulfoxide (40.0 mL). N,N-diisopropylethylamine (3.74 g, 28.9 mmol) and 4-(dimethoxymethyl)piperidine (2.53 g, 15.9 mmol) were added to the reaction mixture. The reaction mixture was heated to 90°C and stirred for 15 h at 90°C. The reaction mixture was quenched with water (50.0 mL) at 25°C. The resulting mixture was extracted with ethyl acetate (50.0 mL × 3). Combined organic phases were washed with brine (50.0 mL × 2), dried over anhydrous sodium sulfate, filtered, and concentrated in vacuo. The residue was purified through column chromatography (silica gel:petroleum ether:ethyl acetate = 100:1-10:1 (v:v:v)) to obtain Intermediate **IM-9a** as a yellow solid (3.10 g, crude product).

**Step 2: Preparation of 1-(2-(2,6-dioxopiperidin-3-yl)-1,3-dioxoisoindolin-5-yl)piperidine-4-carbaldehyde (IM-9)**

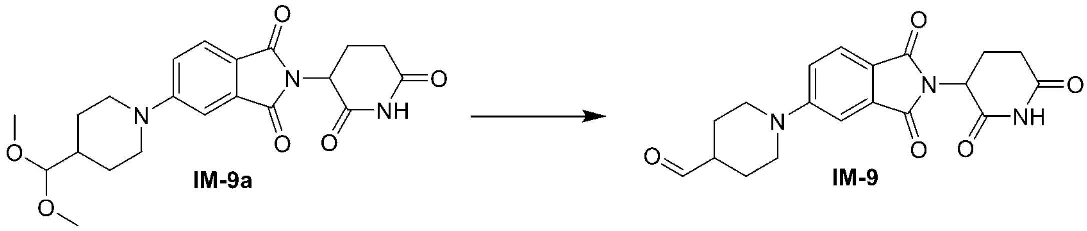

IM-9a IM-9

Intermediate **IM-9a** (500 mg, 1.20 mmol) was added to formic acid (5.00 mL). The reaction mixture was stirred for 12 h at 25°C. The reaction mixture was concentrated to obtain Intermediate **IM-9** as a yellow solid (400 mg, crude product), which was used directly without further purification.

**Intermediate 10: 2-(2,6-dioxopiperidin-3-yl)-5-(piperazin-1-yl)isoindoline-1,3-dione (IM-10)**

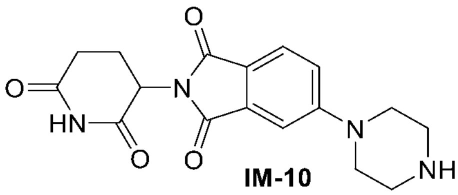

IM-10

**Step 1: Preparation of *t*-butyl 4-(2-(2,6-dioxopiperidin-3-yl)-1,3-dioxoisoindolin-5-yl)piperazine-1-carboxylate (IM-10a)**

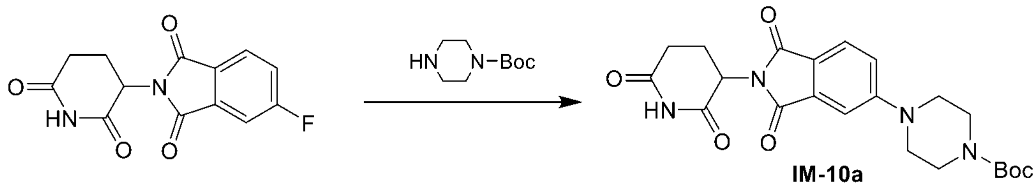

IM-10a

At 25°C, 2-(2,6-dioxopiperidin-3-yl)-5-fluoroisoindoline-1,3-dione (7.00 g, 25.3 mmol) was dissolved in dimethylsulfoxide (60.0 mL). T-butyl piperazine-1-carboxylate (5.62 g, 30.1 mmol) and *N,N*-diisopropylethylamine (6.55 g, 50.6 mmol) were added to the reaction mixture. The obtained reaction mixture was heated to 95°C and stirred for 12 h. Water (60.0 mL) was added to the reaction mixture. The resulting mixture was extracted with ethyl acetate (20.0 mL × 3). Combined organic phases were washed with brine, dried over anhydrous sodium sulfate, filtered, and concentrated in vacuo. The residue was purified through column chromatography (silica gel:petroleum ether:ethyl acetate = 100:1-10:1 (v:v:v)) to obtain Intermediate **IM-10a** as a yellow solid (8.00 g).

**Step 2: Preparation of 2-(2,6-dioxopiperidin-3-yl)-5-(piperazin-1-yl)isoindoline-1,3-dione (IM-10)**

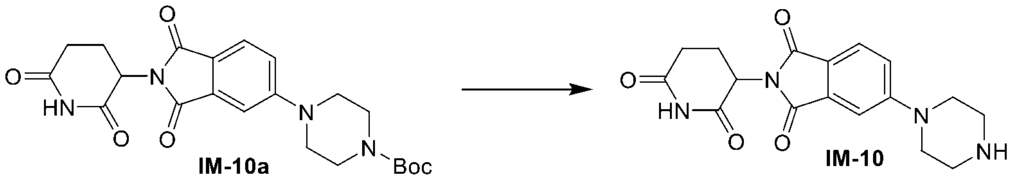

IM-10a IM-10

Intermediate **IM-10a** (2.00 g, 4.52 mmol) was dissolved in dichloromethane (28.0 mL) and added with trifluoroacetic acid (21.5 g, 189 mmol). The reaction mixture was stirred for 12 h at 25°C and concentrated to dryness in vacuo to obtain Intermediate **IM-10** as a yellow solid (2.00 g, trifluoroacetate, crude product), which was used directly without further purification.

$^1$H NMR (400 MHz, DMSO-$d_6$) $\delta$ 11.10 (s, 1H), 7.76 (d, $J$ = 8.5 Hz, 1H), 7.46 (d, $J$ = 2.1 Hz, 1H), 7.31-7.35 (m, 1H), 5.03-5.16 (m, 1H), 3.65 (br s, 1H), 2.89 (s, 8H), 2.72-2.73 (m, 1H), 2.52-2.64 (m, 2H), 1.99-2.06 (m, 1H).

**Intermediate 11: 2-(2,6-dioxopiperidin-3-yl)-5-(2,7-diazaspiro[3.5]nonan-2-yl)isoindoline-1,3-dione**

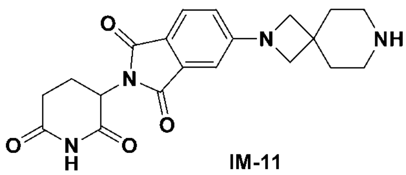

IM-11

**Step 1: Preparation of *t*-butyl 2-(2-(2,6-dioxopiperidin-3-yl)-1,3-dioxoisoindolin-5-yl)-2,7-diazaspiro[3.5]nonane-7-carbox ylate (IM-11a)**

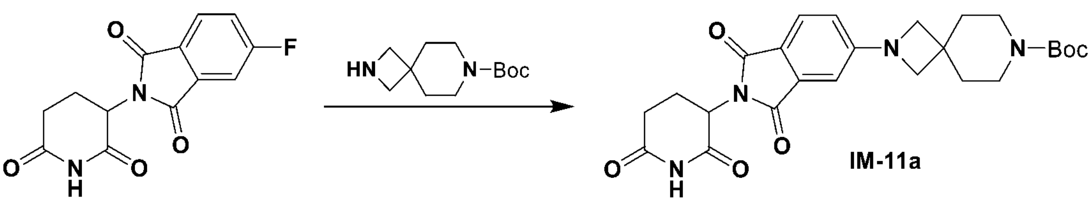

IM-11a

At 25°C, 2-(2,6-dioxopiperidin-3-yl)-5-fluoroisoindoline-1,3-dione (200 mg, 0.724 mmol) was dissolved in dimethylsulfoxide (2.00 mL) and added with *t*-butyl 2,7-diazaspiro[3.5]nonane-7-carboxylate (195 mg, 0.861 mmol) and *N,N*-diisopropylethylamine (187 mg, 1.45 mmol). The reaction mixture was heated to 95°C and stirred for 12 h. The reaction mixture was diluted with water (10 mL) and extracted with ethyl acetate (10 mL × 3). Combined organic phases were washed with saturated brine, dried over anhydrous sodium sulfate, filtered, and concentrated under reduced pressure. The residue was purified through column chromatography (silica gel:petroleum ether:ethyl acetate = 100:1-10:1 (v:v:v)) to obtain Intermediate **IM-11a** as a yellow solid (300 mg, crude product).

**Step 2: Preparation of 2-(2,6-dioxopiperidin-3-yl)-5-(2,7-diazaspiro[3.5]nonan-2-yl)isoindoline-1,3-dione (IM-11)**

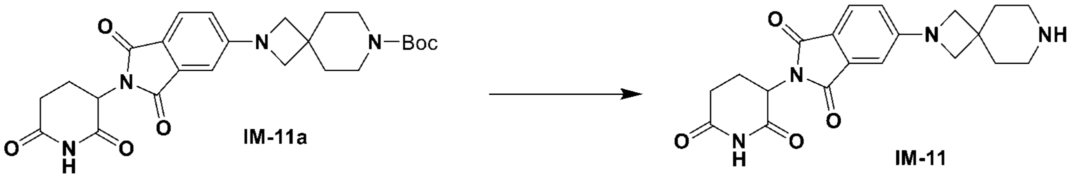

IM-11a IM-11

Compound **IM-11a** (300 mg, 0.621 mmol) was dissolved in dichloromethane (5.00 mL) and added with trifluoroacetic acid (2.91 g, 25.4 mmol). The reaction mixture was stirred for 12 h at 25°C and concentrated under reduced pressure to obtain Intermediate **IM-11** as a yellow solid (300 mg, crude product), which was used directly without further purification.

**Intermediate 12: 2-(2,6-dioxopiperidin-3-yl)-5-(2,7-diazaspiro[3.5]nonan-7-yl)isoindoline-1,3-dione (IM-12, hydrochloride)**

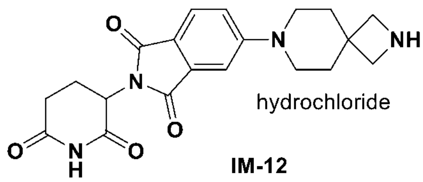


IM-12

**Step 1: Preparation of *t*-butyl 7-(2-(2,6-dioxopiperidin-3-yl)-1,3-dioxoisoindolin-5-yl)-2,7-diazaspiro[3.5]nonane-2-carbox ylate (IM-12a)**

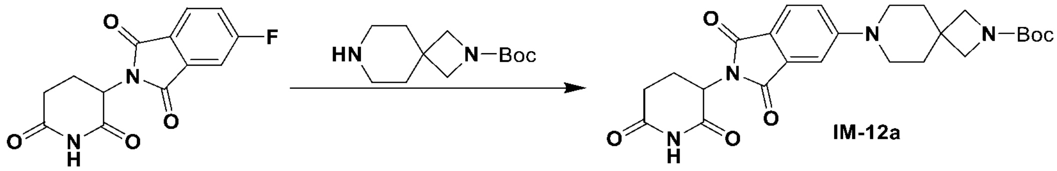

IM-12a

2-(2,6-Dioxopiperidin-3-yl)-5-fluoroisoindoline-1,3-dione (500 mg, 1.81 mmol) was dissolved in dimethylsulfoxide (5.00 mL) and added with t-butyl 2,7-diazaspiro[3.5]nonane-2-carboxylate (450 mg, 1.99 mmol) and N,N-diisopropylethylamine (467 mg, 3.62 mmol) in sequence. The obtained mixture was stirred for 12 h at 95°C. The reaction mixture was concentrated under reduced pressure to obtain Intermediate **IM-12a** as a yellow solid (800 mg), which was used directly without further purification.

**Step 2: Preparation of 2-(2,6-dioxopiperidin-3-yl)-5-(2,7-diazaspiro[3.5]nonan-7-yl)isoindoline-1,3-dione (IM-12)**

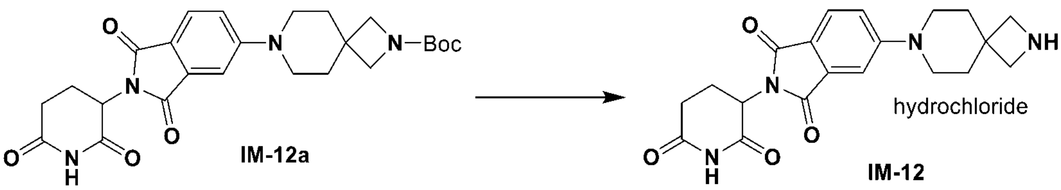


IM-12a IM-12

Compound **2** (400 mg, 0.828 mmol) was dissolved in a 1,4-dioxane solution (5.00 mL) and added with hydrochloric acid/1,4-dioxane (5.00 g, 4.00 M). The reaction mixture was stirred for 2 h at 25°C and concentrated to dryness under reduced pressure to obtain Intermediate **IM-12** as a white solid (250 mg, hydrochloride).

$^1$H NMR (400 MHz, DMSO-$d_6$) $\delta$ 11.07 (s, 1H), 9.28 (br s, 2H), 7.66 (d, $J$ = 8.6 Hz, 1H), 7.18-7.43 (m, 2H), 5.06 (dd, $J$ = 5.4, 12.9 Hz, 1H), 3.73 (br t, $J$ = 6.1 Hz, 4H), 3.41-3.52 (m, 4H), 2.80-2.94 (m, 1H), 2.58 (br d, $J$ = 16.1 Hz, 1H), 2.96-1.06 (m, 1H), 1.79-1.89 (m, 4H).

**Intermediate 13: 1-(2-(2,6-dioxopiperidin-3-yl)-1-oxoisoindolin-5-yl)piperidine-4-carbaldehyde (IM-13)**

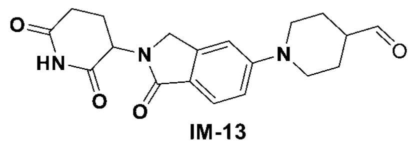

IM-13

**Step 1: Preparation of methyl 2-cyano-4-(4-(hydroxymethyl)piperidin-1-yl)benzoate (IM-13a)**

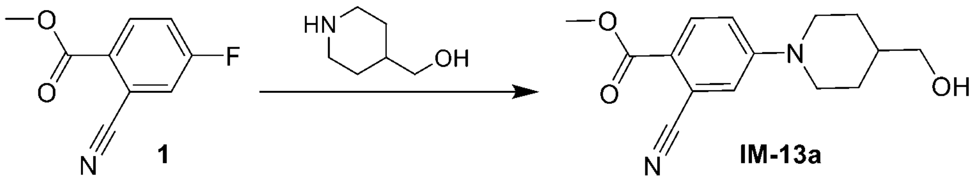

1 IM-13a

Methyl 2-cyano-4-fluorobenzoate (10.0 g, 55.8 mmol) and 4-piperidinemethanol (9.64 g, 83.7 mmol) were dissolved in dimethylsulfoxide (100 mL), and N,N-diisopropylethylamine (20.4 g, 158 mmol) was added at 25°C in a nitrogen atmosphere. The reaction was heated to 110°C and stirred for 16 h at 110°C. The residue was poured into ice water (200 mL) and stirred for 10 min. An aqueous phase was extracted with ethyl acetate (200 mL × 3). Combined organic phases were washed with brine (500 mL), dried over anhydrous sodium sulfate, filtered, and concentrated in vacuo. The residue was purified through column chromatography (silica gel:petroleum ether:ethyl acetate = 100:1-0:1 (v:v:v)) to obtain Intermediate **IM-13a** as a yellow oil (18.0 g, crude product).

**Step 2: Preparation of methyl 2-formyl-4-(4-(hydroxymethyl)piperidin-1-yl)benzoate (IM-13b)**

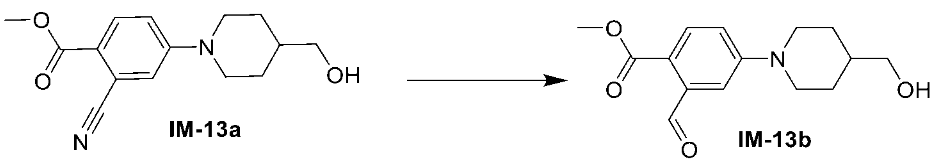

IM-13a IM-13b

Intermediate **IM-13a** (10.0 g, 36.4 mmol) was dissolved in a mixed solvent of glacial acetic acid (10.0 mL), pyridine (20.0 mL), and water (10.0 mL), and sodium hypophosphite monohydrate (50.3 g, 364 mmol) and Raney nickel (4.37 g, 51.0 mmol) were added at 25°C in a nitrogen atmosphere. The reaction system was purged with hydrogen three times and heated to 65°C. The reaction mixture was stirred for 16 h at 65°C in a hydrogen atmosphere. The mixture was filtered in vacuo and concentrated to obtain Intermediate **IM-13b** as a yellow oil (4.50 g, crude product).

**Step 3: Preparation of 3-(5-(4-(hydroxymethyl)piperidin-1-yl)-1-oxoisoindolin-2-yl)piperidine-2,6-dione (IM-13c)**

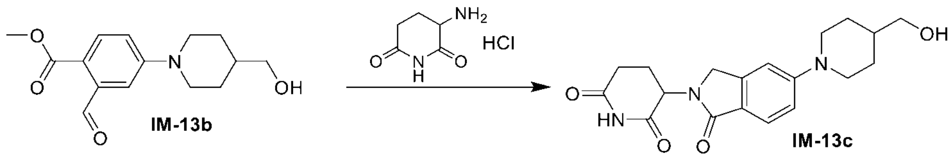


IM-13b IM-13c

Compound **IM-13b** (4.20 g, 15.1 mmol) and 3-aminopiperidine-2,6-dione (2.99 g, 18.1 mmol) were dissolved in dichloromethane (30.0 mL), and *N,N*-diisopropylethylamine (5.87 g, 45.4 mmol), glacial acetic acid (8.82 g, 146 mmol), and sodium cyanoborohydride (2.86 g, 45.4 mmol) were added at 25°C in a nitrogen atmosphere. The reaction mixture was stirred for 12 h at 25°C. The reaction was quenched with water (100 mL) and adjusted to a pH of 8 with a saturated aqueous solution of sodium bicarbonate. The resulting mixture was extracted with dichloromethane/methanol (10/1, 100 mL × 6). Combined organic phases were dried over anhydrous sodium sulfate and concentrated in vacuo. The residue was purified through column chromatography (silica gel:petroleum ether:ethyl acetate = 100:1-0:1 (v:v:v)) to obtain Compound **IM-13c** as a white solid (2.00 g).

**Step 4: Preparation of 1-(2-(2,6-dioxopiperidin-3-yl)-1-oxoisoindolin-5-yl)piperidine-4-carbaldehyde (IM-13)**

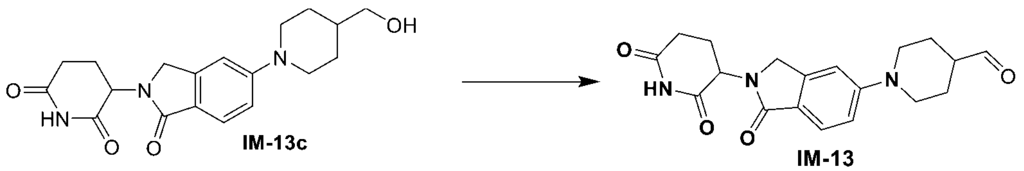

IM-13c IM-13

Compound **IM-13c** (0.50 g, 1.40 mmol) was dissolved in dichloromethane (20.0 mL). A Dess-Martin oxidizing agent (593 mg, 1.40 mmol) was added at 25°C in a nitrogen atmosphere. The reaction mixture was stirred for 1 h at 35°C. The reaction mixture was filtered, and the filtrate was concentrated in vacuo. The residue was purified through high-performance liquid chromatography (under a formic acid condition) to obtain Compound **IM-13** as a yellow solid (1.00 g, crude product).

**Intermediate 14: 2-(4-(2-(2,6-dioxopiperidin-3-yl)-1,3-dioxoisoindolin-5-yl)piperazin-1-yl)acetaldehyde (IM-14)**

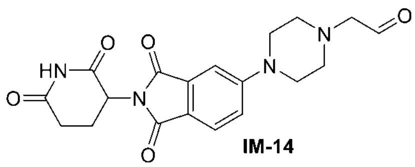

IM-14

**Step 1: Preparation of 5-(4-(2,2-diethoxyethyl)piperazin-1-yl)-2-(2,6-dioxopiperidin-3-yl)isoindoline-1,3-dione (IM-14a)**

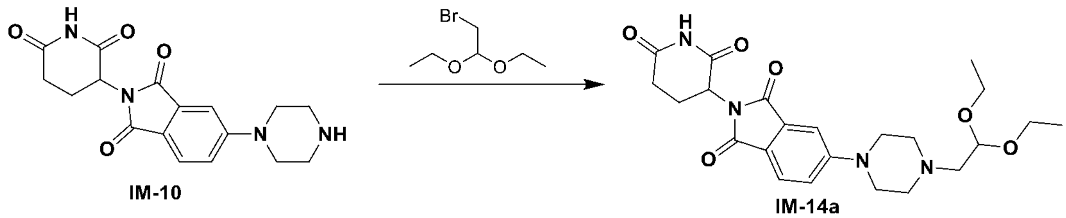

IM-10 IM-14a

At room temperature, Intermediate **IM-10** (1.00 g, 2.92 mmol) was dissolved in N,N-dimethylformamide (8.00 mL) and added with 2-bromo-1,1-diethoxyethane (1.15 g, 5.84 mmol) and N,N-diisopropylethylamine (1.13 g, 8.76 mmol). The reaction mixture was heated to 100°C and stirred for 12 h. The reaction mixture was diluted with water (30.0 mL) and

extracted with ethyl acetate (30.0 mL × 3). Combined organic phases were washed with saturated brine, dried over anhydrous sodium sulfate, filtered, and concentrated under reduced pressure. The residue was subjected to preparative HPLC (column: Phenomenex Luna C18 80×40 mm × 3 μm; mobile phase: [$H_2O$ (FA)-ACN]; B%: 1%-35%, 8 min) to obtain Intermediate **IM-14a** as a yellow solid (200 mg).

**Step 2: Preparation of 2-(4-(2-(2,6-dioxopiperidin-3-yl)-1,3-dioxoisoindolin-5-yl)piperazin-1-yl)acetaldehyde (IM-14)**

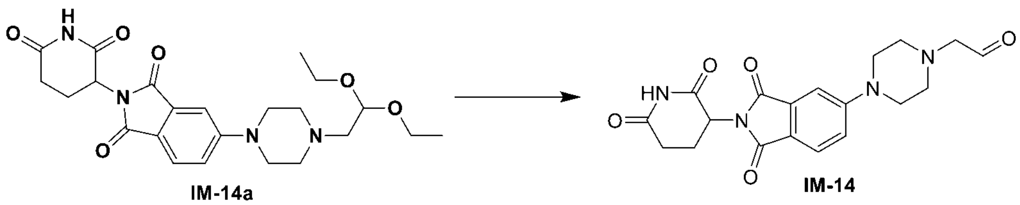

IM-14a IM-14

Intermediate **IM-14a** (100 mg, 218.10 μmol) was dissolved in formic acid (2.00 mL). The reaction mixture was stirred for 12 h at 65°C. The reaction mixture was concentrated to dryness to obtain Intermediate **IM-14** as a yellow solid (40.0 mg, crude product).

**Intermediate 15: 1-(2-(2,6-dioxopiperidin-3-yl)-6-fluoro-1,3-dioxoisoindolin-5-yl)piperidine-4-carbaldehyde (IM-15)**

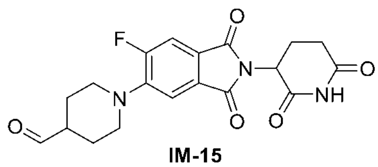

IM-15

**Step 1: Preparation of 2-(2,6-dioxopiperidin-3-yl)-5,6-difluoroisoindoline-1,3-dione (IM-15a)**

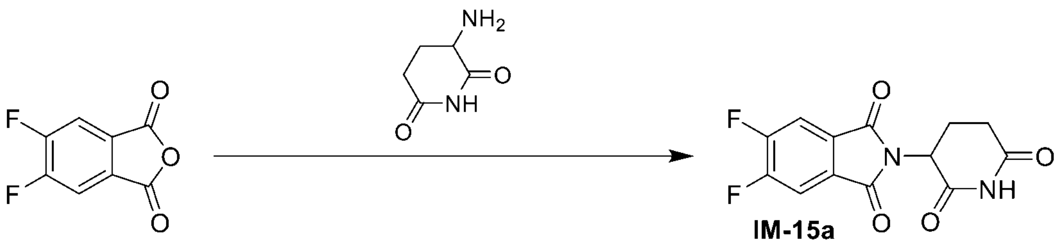

IM-15a

3-Aminopiperidine-2,6-dione hydrochloride (9.83 g, 59.7 mmol) was dissolved in toluene (100 mL). Triethylamine (16.4 g, 162 mmol) and 5,6-difluoroisobenzofuran-1,3-dione (10.0 g, 54.3 mmol) were added at room temperature. The reaction mixture was stirred for 12 h at 60°C. The reaction mixture was diluted with water (100 mL) and extracted with ethyl acetate (100 mL × 3). Combined organic phases were washed with saturated brine, dried over anhydrous sodium sulfate, filtered, and concentrated under reduced pressure. The residue was purified through column chromatography (silica gel:petroleum ether:ethyl acetate = 100:1-10:1 (v:v:v)) to obtain Intermediate **IM-15a** as a yellow solid (4.50 g, crude product).

**Step 2: Preparation of 5-(4-(dimethoxymethyl)piperidin-1-yl)-2-(2,6-oxopiperidin-3-yl)-6-fluoroisoindoline-1,3-dio ne (IM-15b)**

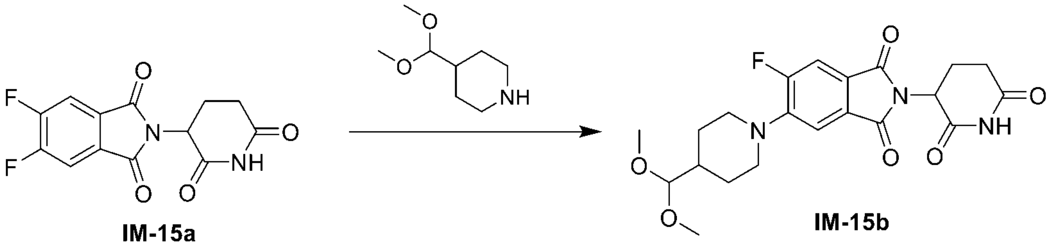

IM-15a IM-15b

At room temperature, **IM-15a** (4.50 g, 13.6 mmol) was dissolved in dimethylsulfoxide (40.0 mL) and added with *N,N*-diisopropylethylamine (3.51 g, 27.1 mmol) and 4-(dimethoxymethyl)piperidine (2.16 g, 13.6 mmol). The reaction mixture was stirred for 12 h at 90°C. The reaction mixture was diluted with water (40.0 mL) and extracted with ethyl acetate (40.0 mL × 3). Combined organic phases were washed with saturated brine, dried over anhydrous sodium sulfate, filtered, and concentrated under reduced pressure. The crude product was purified through column chromatography (silica gel:petroleum ether:ethyl acetate = 100:1-10:1 (v:v:v)) to obtain Intermediate **IM-15b** as a yellow solid (2.50 g, crude product).

**Step 3: Preparation of 1-(2-(2,6-dioxopiperidin-3-yl)-6-fluoro-1,3-dioxoisoindolin-5-yl)piperidine-4-carbaldehyde (IM-15)**

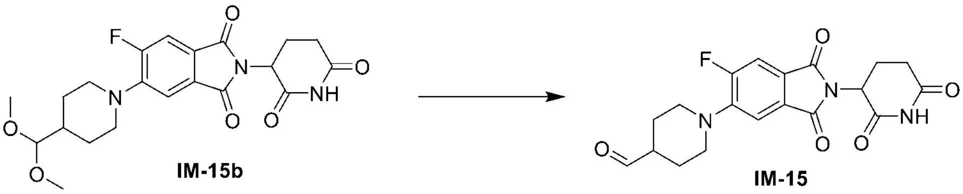

IM-15b IM-15

At room temperature, Intermediate **IM-15b** (1.00 g, 2.31 mmol) was dissolved in tetrahydrofuran (10.0 mL) and water (2.00 mL) and added with pyridinium *p*-toluenesulfonate (1.16 g, 4.61 mmol). The reaction mixture was stirred for 12 h at 70°C and concentrated to obtain Intermediate **IM-15** as a yellow solid (500 mg), which was used directly without further purification.

**Intermediate 16: 2-(1-(2-(2,6-dioxopiperidin-3-yl)-1,3-dioxoisoindolin-5-yl)piperidin-4-yl)acetaldehyde (IM-16)**

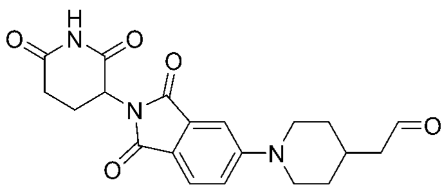

**IM-16**

**Step 1: Preparation of 2-(2,6-dioxopiperidin-3-yl)-5-(4-(2-hydroxyethyl)piperidin-1-yl)isoindoline-1,3-dione (IM-16a)**

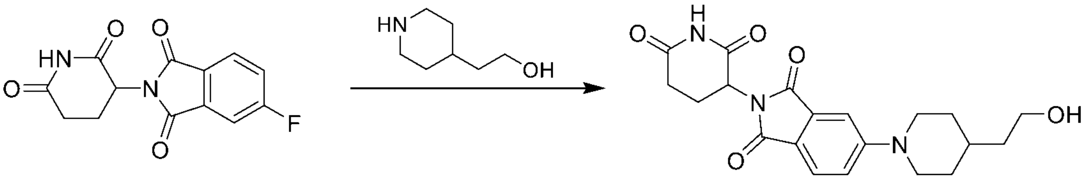

1

IM-16a

2-(2,6-Dioxopiperidin-3-yl)-5-fluoroisoindoline-1,3-dione (2.00 g, 7.24 mmol) was dissolved in dimethylsulfoxide (10.0 mL) and added with *N,N*-diisopropylethylamine (1.87 g, 14.4 mmol) and 2-(piperidin-4-yl)ethan-1-ol (1.03 g, 7.96 mmol). The reaction mixture was stirred for 12 h at 90°C. The reaction was quenched by slowly adding water (10.0 mL) and extracted with ethyl acetate (10.0 mL × 3). Combined organic phases were washed with saturated brine, dried over anhydrous sodium sulfate, filtered, and concentrated under reduced pressure. The residue was purified through column chromatography to obtain Intermediate **IM-16a** as a yellow solid (1.10 g).

**Step 2: 2-(1-(2-(2,6-dioxopiperidin-3-yl)-1,3-dioxoisoindolin-5-yl)piperidin-4-yl)acetaldehyde**

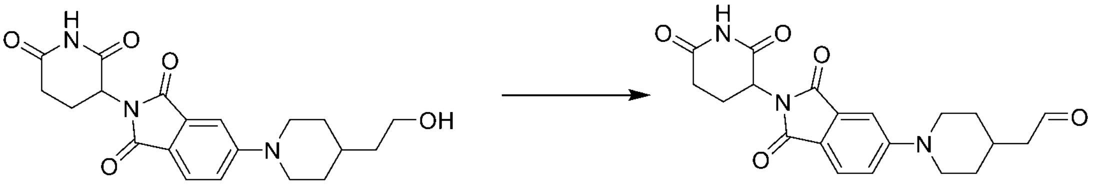

IM-16a

IM-16

At 25°C, Intermediate **IM-16a** (1.10 g, 2.85 mmol) was dissolved in dichloromethane (10.0 mL) and added with Dess-Martin periodinane (1.21 g, 2.85 mmol). The reaction mixture was stirred for 2 h at 25°C. The reaction was quenched by slowly adding water (10.0 mL) and extracted with ethyl acetate (30 mL × 3). Combined organic phases were washed with saturated brine, dried over anhydrous sodium sulfate, filtered, and concentrated under reduced pressure to obtain Intermediate **IM-16** as a yellow solid (600 mg), which was used directly without further purification.

**Intermediate 17: 3-(4-(2-(2,6-dioxopiperidin-3-yl)-1,3-dioxoisoindolin-5-yl)piperazin-1-yl)propyl methanesulfonate (IM-17)**

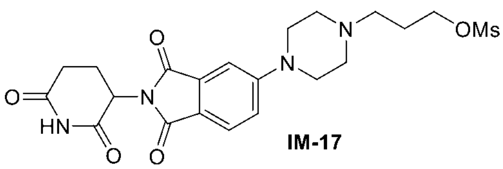

IM-17

**Step 1: Preparation of 2-(2,6-dioxopiperidin-3-yl)-5-(4-(3-hydroxypropyl)piperazin-1-yl)isoindoline-1,3-dione (IM-17a)**

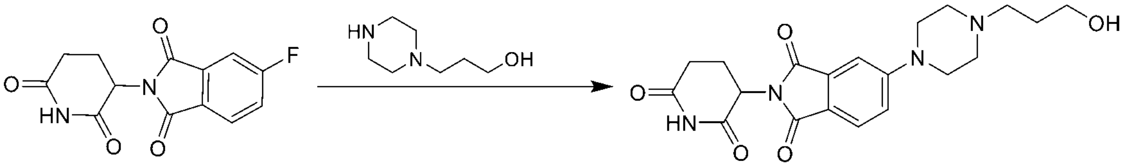

1

IM-17a

2-(2,6-Dioxopiperidin-3-yl)-5-fluoroisoindoline-1,3-dione (1.72 g, 6.24 mmol) was dissolved in dimethylsulfoxide (10.0 mL) and added with *N,N*-diisopropylethylamine (1.61 g, 12.4 mmol) and 3-(piperazin-1-yl)propan-1-ol (900 mg, 6.24 mmol). The reaction mixture was stirred for 12 h at 90°C. The reaction was diluted with water (10.0 mL). An aqueous layer was extracted with ethyl acetate (10 mL × 3). Combined organic phases were washed with saturated brine, dried over anhydrous sodium sulfate, filtered, and concentrated under reduced pressure. The residue was purified through column chromatography (in a formic acid system) to obtain Intermediate **IM-17a** as a yellow solid (1.50 g).

**Step 2: Preparation of 3-(4-(2-(2,6-dioxopiperidin-3-yl)-1,3-dioxoisoindolin-5-yl)piperazin-1-yl)propyl methanesulfonate (IM-17)**

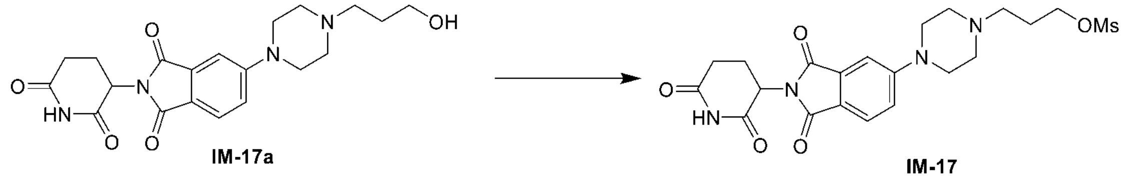

**IM-17a** **IM-17**

Compound **IM-17a** (1.00 g, 2.50 mmol) was dissolved in tetrahydrofuran (10.0 mL) and added with triethylamine (631 mg, 6.24 mmol) and methanesulfonic anhydride (870 mg, 4.99 mmol). The reaction mixture was stirred for 12 h at 25°C. The reaction mixture was diluted with water (10 mL) and extracted with ethyl acetate (10 mL × 3). Combined organic phases were washed with saturated brine, dried over anhydrous sodium sulfate, filtered, and concentrated under reduced pressure to obtain Intermediate **IM-17** as a yellow solid (600 mg, crude product).

**Intermediate** 18: **2-(2,6-dioxopiperidin-3-yl)-5-(4-oxopiperidin-1-yl)isoindoline-1,3-dione (IM-18)**

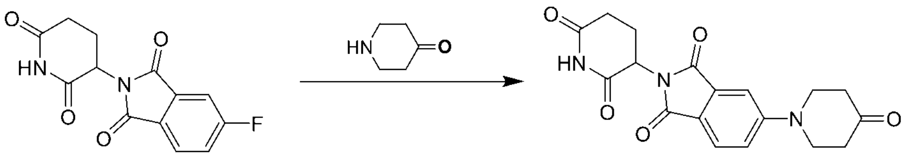

**IM-18**

At room temperature, piperidin-4-one (490 mg, 3.62 mmol) was dissolved in dimethylsulfoxide (10.0 mL) and added with N,N-diisopropylethylamine (935 mg, 7.24 mmol) and 2-(2,6-dioxopiperidin-3-yl)-5-fluoroisoindoline-1,3-dione (1.00 g, 3.62 mmol). The reaction mixture was heated to 90°C and stirred for 12 h. The reaction mixture was cooled to 25°C, added with water (10.0 mL), and extracted with ethyl acetate (10.0 mL × 3). Combined organic layers were dried over anhydrous sodium sulfate, filtered, and concentrated under reduced pressure. The residue was purified through column chromatography to obtain Intermediate **IM-18** as a yellow oil (310 mg).

**Intermediate 19: 2-(2,6-dioxopiperidin-3-yl)-5-(2,6-diazaspiro[3.3]heptan-2-yl)isoindoline-1,3-dione trifluoroacetate (IM-19)**

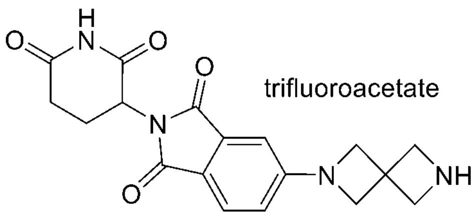


**IM-19**

**Step 1: Preparation of *t*-butyl 6-(2-(2,6-dioxopiperidin-3-yl)-1,3-dioxoisoindolin-5-yl)-2,6-diazaspiro[3.3]heptane-2-carbox ylate (IM-19a)**

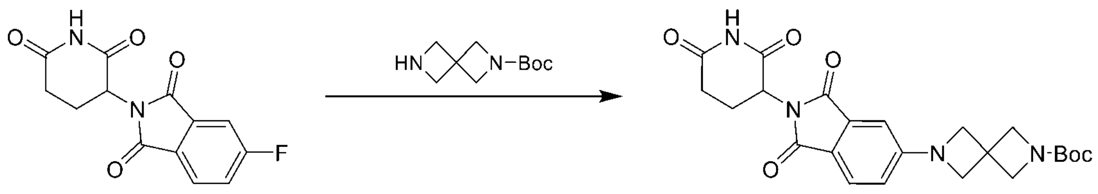

**1** **IM-19a**

2-(2,6-Dioxopiperidin-3-yl)-5-fluoroisoindoline-1,3-dione (3.00 g, 10.8 mmol) and t-butyl 2,6-diazaspiro[3.3] heptane-2-carboxylate (2.15 g, 10.8 mmol) were dissolved in dimethylsulfoxide (30.0 mL) and added with N,N-diisopropylethylamine (4.21 g, 32.5 mmol). The reaction mixture was heated to 95°C and stirred for 12 h. The reaction was quenched with 20.0 mL of water at 5°C. The resulting mixture was extracted with 45.0 mL (15.0 mL × 3) of dichloromethane. An organic layer was washed with an aqueous solution of sodium chloride (5.00 mL × 3), dried over anhydrous sodium sulfate, filtered, and concentrated under reduced pressure. The residue was purified through column chromatography (silica gel:petroleum ether:ethyl acetate = 100:1-10:1 (v:v:v)) to obtain Intermediate **IM-19a** as a yellow oil (1.20 g).

**Step 2: Preparation of 2-(2,6-dioxopiperidin-3-yl)-5-(2,6-diazaspiro[3.3]heptan-2-yl)isoindoline-1,3-dione trifluoroacetate (IM-19)**

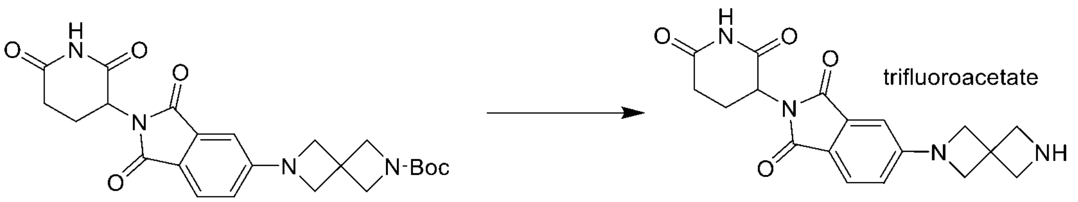


**IM-19a** **IM-19**

Intermediate **IM-19a** (200 mg, 440 μmol) was dissolved in dichloromethane (2.00 mL) and added with trifluoroacetic acid (3.07 g, 26.9 mmol). The reaction mixture was stirred for 12 h at 25°C and concentrated under reduced pressure to obtain Intermediate **IM-19a** as a white solid (150 mg).

**Intermediate 20: 2-(4-(2-(2,6-dioxopiperidin-3-yl)-6-fluoro-1,3-dioxoisoindolin-5-yl)piperazin-1-yl)ethyl methanesulfonate (IM-20)**

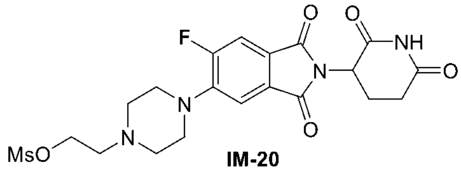

**IM-20**

**Step 1: Preparation of** 2-(2,6-dioxopiperidin-3-yl)-5-fluoro-6-(4-(2-hydroxyethyl)piperazin-1-yl)isoindoline-1,3-dio ne (IM-20a)

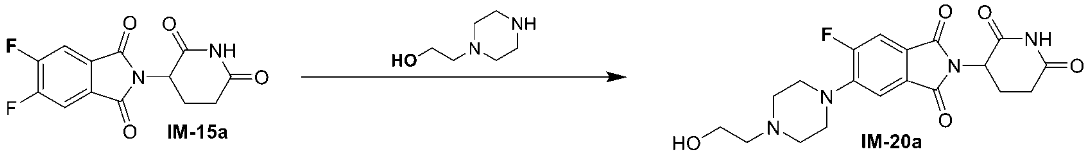

IM-15a IM-20a

At 25°C, Intermediate **IM-15a** (1.00 g, 3.40 mmol) and 2-(piperazin-1-yl)ethan-1-ol (486 mg, 3.74 mmol) were dissolved in dimethylsulfoxide (10.0 mL). N,N-diisopropylethylamine (439 mg, 3.40 mmol) was added to the reaction mixture. The reaction mixture was heated to 90°C and stirred for 12 h. The reaction was quenched with 20.0 mL of water at 25°C and extracted with ethyl acetate (30.0 mL × 3). Combined organic layers were dried over anhydrous sodium sulfate, filtered, and concentrated under reduced pressure. The residue was purified through column chromatography (silica gel:petroleum ether:ethyl acetate = 100:1-0:1 (v:v:v)) to obtain Intermediate **IM-20a** as a yellow solid (1.00 g, 2.97 mmol).

**Step 2: Preparation of 2-(4-(2-(2,6-dioxopiperidin-3-yl)-6-fluoro-1,3-dioxoisoindolin-5-yl)piperazin-1-yl)ethyl methanesulfonate (IM-20)**

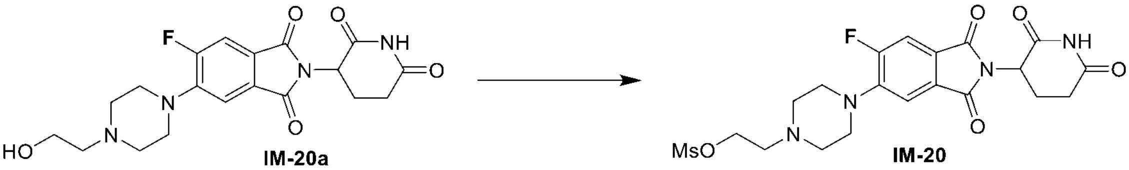

IM-20a IM-20

Intermediate **IM-20a** (200 mg, 494 μmol) was dissolved in dichloromethane (2.00 mL). Triethylamine (125 mg, 1.24 mmol) was added to the reaction mixture at 25°C. The reaction mixture was stirred for 30 min at 25°C. Methanesulfonic anhydride (129 mg, 741 μmol) was added to the reaction mixture. The reaction mixture was stirred for 11.5 h at 25°C. The reaction mixture was slowly poured into water (10 mL) and extracted with dichloromethane (5 mL × 3). Combined organic phases were washed with brine (5.00 mL × 2), dried over anhydrous sodium sulfate, filtered, and concentrated in vacuo to obtain Intermediate **IM-20** as a yellow solid (200 mg, crude product), which was used directly without further purification.

**Intermediate 21: 3-(1-oxo-5-(piperazin-1-yl)isoindolin-2-yl)piperidine-2,6-dione hydrochloride (IM-21)**

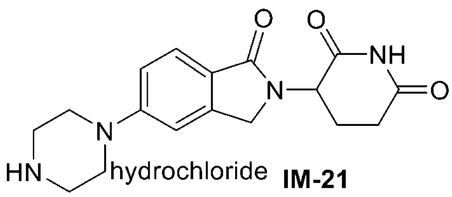

hydrochloride **IM-21**

**Step 1: Preparation of methyl 4-fluoro-2-formylbenzoate (IM-21a)**

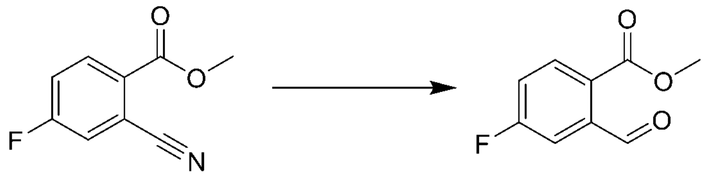

**IM-21a**

Pyridine (15.0 g, 190 mmol) was added to water (50.0 mL) and acetic acid (50 mL). Raney nickel (7.72 g, 90.1 mmol) was added in a nitrogen atmosphere, and a solution of methyl 2-cyano-4-fluorobenzoate (5.00 g, 27.9 mmol) in

tetrahydrofuran (50.0 mL) was added. The reaction mixture was heated to 65°C and stirred for 16 h. The reaction was quenched by slowly adding water (50.0 mL). An aqueous layer was extracted with ethyl acetate (50 mL × 3). Combined organic layers were washed with saturated brine, dried over anhydrous sodium sulfate, filtered, and concentrated under reduced pressure. The residue was purified through column chromatography (silica gel:petroleum ether:ethyl acetate = 100:1-10:1 (v:v:v)) to obtain Intermediate **IM-21a** as a white solid (3.00 g, crude product).

**Step 2: Preparation of t-butyl 4-(3-formyl-4-(methoxycarbonyl)phenyl)piperazine-1-carboxylate (IM-21b)**

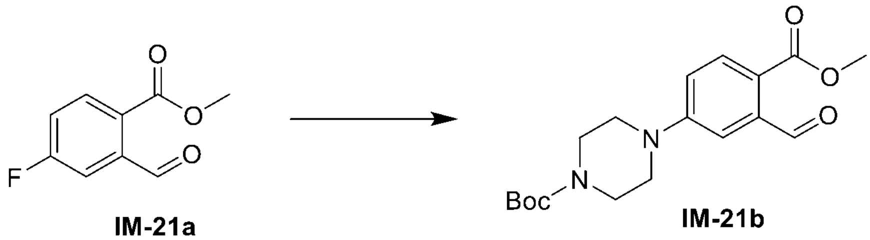

**IM-21a** **IM-21b**

At room temperature, Boc-piperazine (2.61 g, 14.0 mmol) was dissolved in dimethylsulfoxide (30.0 mL) and added with *N,N*-diisopropylethylamine (6.39 g, 49.4 mmol) and Intermediate **IM-21a** (3.00 g, 16.4 mmol). The reaction mixture was heated to 100°C and stirred for 16 h. The reaction was quenched by slowly adding water (30.0 mL). An aqueous layer was extracted with ethyl acetate (30 mL × 3). Combined organic phases were washed with saturated brine, dried over anhydrous sodium sulfate, filtered, and concentrated under reduced pressure. The residue was purified through column chromatography (silica gel:petroleum ether:ethyl acetate = 100:1-10:1 (v:v:v)) to obtain Intermediate **IM-21b** as a yellow solid (1.40 g).

$^1$H NMR (400 MHz, DMSO-$d_6$) δ 10.49 (s, 1H), 7.84 (d, *J* = 8.6 Hz, 1H), 7.12-7.26 (m, 2H), 3.84 (s, 2H), 3.64-3.70 (m, 1H), 3.42-3.50 (m, 4H), 3.34-3.42 (m, 4H), 1.44-1.41 (m, 9H).

**Step 3: Preparation of t-butyl 4-(2-(2,6-dioxopiperidin-3-yl)-1-oxoisoindolin-5-yl)piperazine-1-carboxylate (IM-21c)**

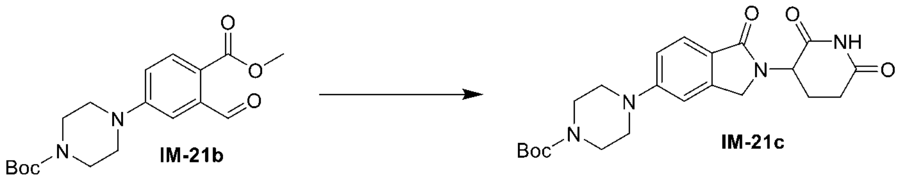

**IM-21b** **IM-21c**

3-Aminopiperidine-2,6-dione hydrochloride (992 mg, 6.03 mmol) was dissolved in dimethylsulfoxide (10.0 mL), added with N,N-diisopropylethylamine (1.56 g, 12.0 mmol), and added with acetic acid (1.54 g, 25.6 mmol), sodium triacetoxyborohydride (1.70 g, 8.04 mmol), and Intermediate **IM-21b** (1.40 g, 4.02 mmol). The reaction mixture was stirred for 12 h at 25°C. The reaction was quenched by slowly adding water (30 mL). An aqueous layer was extracted with ethyl acetate (20 mL × 3). Combined organic phases were washed with saturated brine, dried over anhydrous sodium sulfate, filtered, and concentrated under reduced pressure. The residue was purified through column chromatography (silica gel:petroleum ether:ethyl acetate = 100:1-10:1 (v:v:v)) to obtain Intermediate **IM-21c** as a yellow solid (900 mg).

**Step 4: Preparation of 3-(1-oxo-5-(piperazin-1-yl)isoindolin-2-yl)piperidine-2,6-dione hydrochloride (IM-21)**

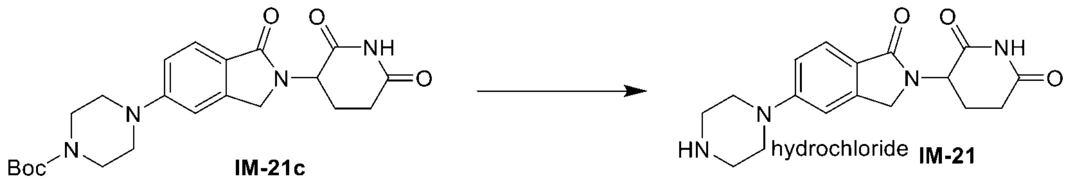


IM-21c IM-21

Intermediate **IM-21c** (182 mg, 424 μmol) was dissolved in dioxane (2.00 mL) and added with hydrochloric acid/dioxane (4 M, 1.82 mL). The reaction mixture was stirred for 12 h at 25°C. The reaction mixture was concentrated to obtain Intermediate IM-21 as an off-white solid (160 mg).

**Intermediate 22: 3-(1-oxo-6-(piperazin-1-yl)isoindolin-2-yl)piperidine-2,6-dione hydrochloride (IM-22)**

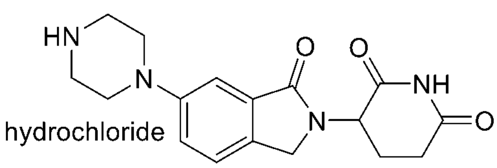


**IM-22**

**Step 1: Preparation of t-butyl 4-(4-formyl-3-(methoxycarbonyl)phenyl)piperazine-1-carboxylate (IM-22a)**

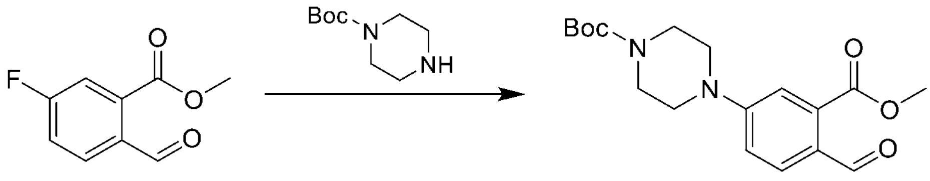

**IM-22a**

At room temperature, Boc-piperazine (869 mg, 4.67 mmol) was dissolved in dimethylsulfoxide (8.00 mL) and added with N,N-diisopropylethylamine (2.13 g, 16.4 mmol) and methyl 5-fluoro-2-formylbenzoate (1.00 g, 5.49 mmol). The reaction mixture was heated to 80°C and stirred for 16 h. The reaction was quenched by slowly adding water (30 mL). An aqueous layer was extracted with ethyl acetate (10 mL × 3). Organic phases were combined, washed with saturated brine, dried over anhydrous sodium sulfate, filtered, and concentrated under reduced pressure to obtain Intermediate **IM-22a** as a yellow oil (1.80 g, crude product).

**Step 2: Preparation of t-butyl 4-(2-(2,6-dioxopiperidin-3-yl)-3-oxoisoindolin-5-yl)piperazine-1-carboxylate (IM-22b)**

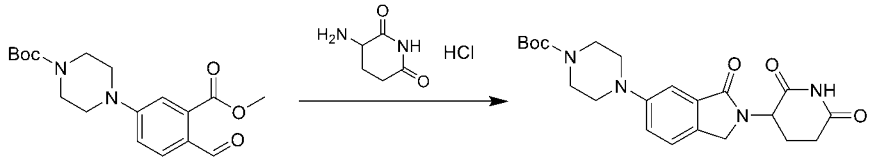


IM-22a IM-22b

3-Aminopiperidine-2,6-dione hydrochloride (935 mg, 5.68 mmol) was dissolved in N,N-dimethylformamide (5 mL), added with N,N-diisopropylethylamine (1.34 g, 10.3 mmol) at room temperature, and added with acetic acid (3.10 g, 51.6 mmol), sodium triacetoxyborohydride (3.29 g, 15.5 mmol), and Intermediate **IM-22a** (1.80 g, 5.17 mmol). The reaction mixture was stirred for 12 h at 25°C. The reaction was quenched by slowly adding water (20 mL). An aqueous layer was

extracted with ethyl acetate (10 mL × 3). Organic phases were combined, washed with saturated brine, dried over anhydrous sodium sulfate, filtered, and concentrated under reduced pressure to obtain Intermediate **IM-22b** as a yellow oil (650 mg, crude product).

**Step 3: Preparation of 3-(1-oxo-6-(piperazin-1-yl)isoindolin-2-yl)piperidine-2,6-dione hydrochloride (IM-22)**

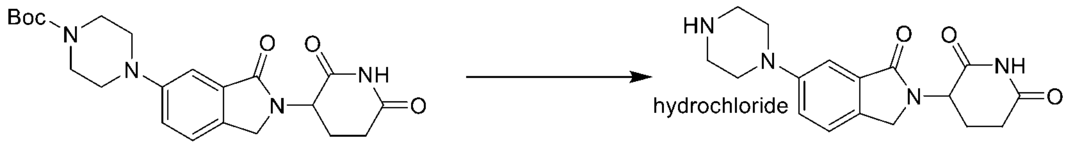


IM-22b IM-22

Intermediate **IM-22b** (650 mg, 1.52 mmol) was dissolved in ethyl acetate (2.00 mL). Hydrochloric acid/ethyl acetate (4 M, 2.00 mL) was added and stirred for 2 h at room temperature. The reaction mixture was concentrated to obtain Intermediate **IM-22** as a yellow solid (220 mg).

**Intermediate 23: 1-(2-(2,6-dioxopiperidin-3-yl)-6-fluoro-1-oxoisoindolin-5-yl)piperidine-4-carbaldehyde (IM-23)**

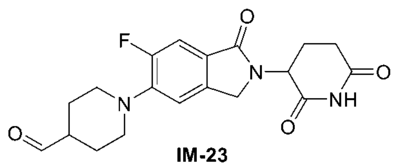

IM-23

**Step 1: Preparation of methyl 4-bromo-2-(bromomethyl)-5-fluorobenzoate (IM-23a)**

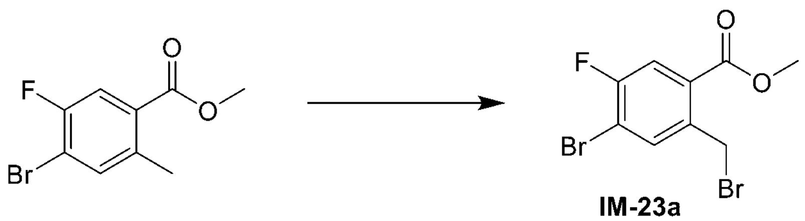

IM-23a

Methyl 4-bromo-5-fluoro-2-methylbenzoate (4.50 g, 18.2 mmol) was dissolved in dichloroethane (25.0 mL). N-bromosuccinimide (3.53 g, 19.8 mmol) and azobisisobutyronitrile (149 mg, 910 μmol) were added in sequence at room temperature. The reaction mixture was stirred for 12 h at 80°C. The reaction mixture was diluted with water (30.0 mL) and extracted with ethyl acetate (30.0 mL × 3). Combined organic phases were washed with saturated brine, dried over anhydrous sodium sulfate, filtered, and concentrated under reduced pressure. The residue was purified through column chromatography (silica gel) to obtain Intermediate **IM-23a** as a yellow solid (2.50 g, crude product).

**Step 2: Preparation of 3-(5-bromo-6-fluoro-1-oxoisoindolin-2-yl)piperidine-2,6-dione (IM-23b)**

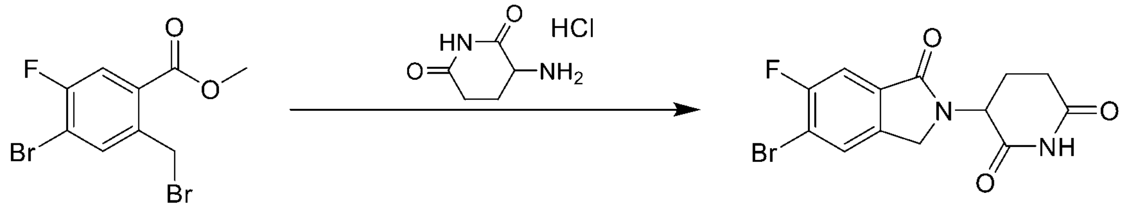


IM-23a IM-23b

Compound **IM-23a** (2.30 g, 7.06 mmol) was dissolved in acetonitrile (15.0 mL). 3-Aminopiperidine-2,6-dione hydrochloride (1.39 g, 8.47 mmol) and *N,N*-diisopropylethylamine (2.74 g, 21.1 mmol) were added in sequence at 25°C. The reaction mixture was stirred for 12 h at 80°C. The reaction mixture was diluted with water (30.0 mL) and extracted with ethyl acetate (30.0 mL × 3). Combined organic phases were washed with saturated brine, dried over anhydrous sodium sulfate, filtered, and concentrated under reduced pressure. The residue was purified through column chromatography (silica gel) to obtain Intermediate **IM-23b** as a brownish-black solid (1.00 g).

MS (ESI+) m/z 342.0 $[M+H]^+$.

**Step 3: Preparation of 3-(5-(4-(dimethoxymethyl)piperidin-1-yl)-6-fluoro-1-oxoisoindolin-2-yl)piperidine-2,6-dion e (IM-23c)**

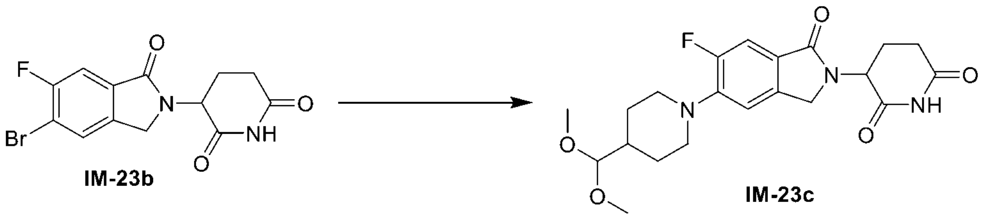

IM-23b IM-23c

Compound **IM-23b** (1.00 g, 2.93 mmol) was dissolved in dimethylsulfoxide (10.0 mL). 4-(Dimethoxymethyl) piperidine, cesium carbonate (2.87 g, 8.79 mmol), and Pd-PEPPSI-IPentCl (285 mg, 293 μmol) were added in sequence at room temperature. The reaction mixture was stirred for 12 h at 80°C. The reaction mixture was diluted with water (20.0 mL) and extracted with ethyl acetate (20.0 mL × 3). Combined organic phases were washed with saturated brine, dried over anhydrous sodium sulfate, filtered, and concentrated under reduced pressure. The residue was purified through column chromatography (silica gel) to obtain Intermediate **IM-23c** as a yellow solid (200 mg, crude product).

MS (ESI+) m/z 420.1 $[M+H]^+$.

**Step 4: Preparation of 1-(2-(2,6-dioxopiperidin-3-yl)-6-fluoro-1-oxoisoindolin-5-yl)piperidine-4-carbaldehyde (IM-23)**

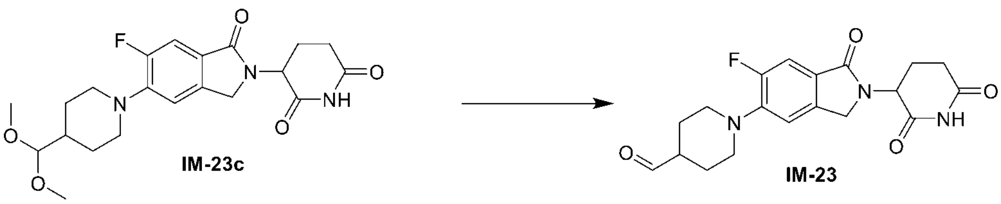

IM-23c IM-23

Intermediate **23c** (200 mg, 47.6 μmol) was dissolved in formic acid (2.00 mL) at room temperature. The reaction mixture was stirred for 12 h at 55°C and concentrated under reduced pressure to obtain Intermediate **IM-23** as a yellow oil (150 mg), which was used directly in the next step without purification.

MS (ESI+) m/z 374.1 $[M+H]^+$.

**Intermediate** 24: **3-(2-(2,6-dioxopiperidin-3-yl)-1,3-dioxoisoindolin-5-yl)-3-azabicyclo[3.1.0]hexane-6-carbaldehyde (IM-24)**

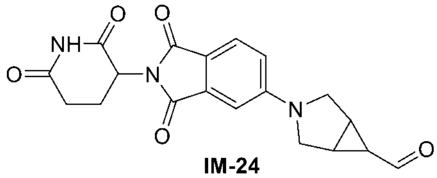

**IM-24**

**Step 1: Preparation of 3-*t*-butyl 6-ethyl 3-azabicyclo[3.1.0]hexane-3,6-dicarboxylate (IM-24a)**

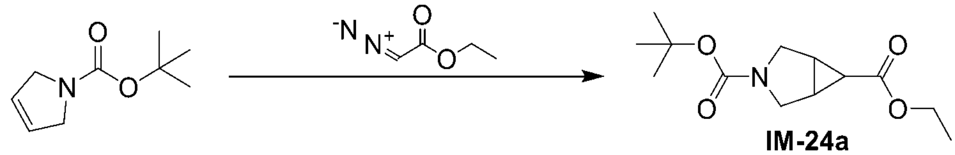

**IM-24a**

Ethyl diazoacetate (10.0 g, 59.0 mmol) and rhodium(II) acetate dimer (261 mg, 590 μmol) were dissolved in dichloromethane (100 mL), and t-butyl 2,5-dihydro-1H-pyrrole-1-carboxylate (10.1 g, 88.6 mmol) was slowly added dropwise. After addition, the obtained reaction mixture was stirred for 12 h at 25°C and concentrated under reduced pressure. The residue was purified through column chromatography (silica gel:petroleum ether:ethyl acetate = 100:1-10:1 (v:v:v)) and subjected to preparative HPLC (column: Welch Xtimate C18 250×70 mm × 10 μm; mobile phase: [$H_2O$ (10 mM $NH_4HCO_3$)-ACN]; gradient: 40%-70% B, 18.0 min) to obtain Intermediate **IM-24a** as a yellow oily compound (500 mg).

**Step 2: Preparation of *t*-butyl 6-(hydroxymethyl)-3-azabicyclo[3.1.0]hexane-3-carboxylate (IM-24b)**

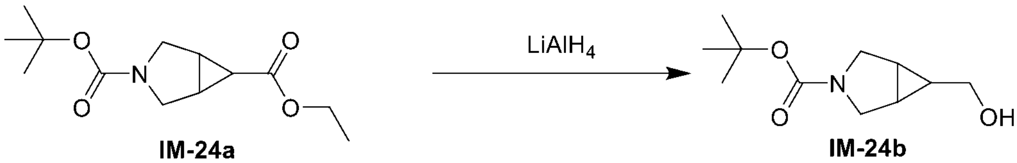


**IM-24a** **IM-24b**

At 0°C, Intermediate **IM-24a** (500 mg, 1.96 mmol) was dissolved in tetrahydrofuran (10.0 mL), and lithium aluminum hydride (2.50 M, 1.96 mL) was slowly added dropwise. The reaction mixture was stirred for 2 h at 25°C. The reaction was quenched with water (20 mL) at 0°C and extracted with dichloromethane (90 mL). An organic layer was washed with an aqueous solution of sodium chloride (30 mL), dried over sodium sulfate, and filtered and concentrated under reduced pressure. The residue was purified through column chromatography (silica gel:petroleum ether:ethyl acetate = 100:1-10:1 (v:v:v)) to obtain Intermediate **IM-24b** as a yellow solid (250 mg).

**Step 3: Preparation of (3-azabicyclo[3.1.0]hexan-6-yl)methanol (IM-24c)**

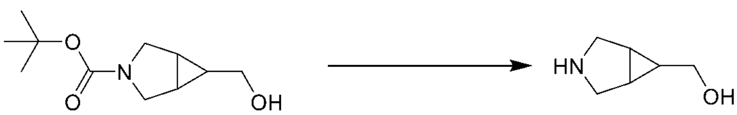

**IM-24b** **IM-24c**

At 25°C, Intermediate **IM-24b** (240 mg, 1.13 mmol) was dissolved in 1,4-dioxane (10.0 mL) and added with hydrochloric acid/dioxane (4 M, 8.44 mL). The reaction mixture was stirred for 2 h and concentrated under reduced

pressure to obtain Intermediate **IM-24c** as a white solid (200 mg), which was used directly in the next step without further purification.

$^1$H NMR: (400 MHz, $CDCl_3$) $\delta$ 3.49 (brd, $J$ = 6.9 Hz, 2H), 2.99 (brd, $J$ = 11.3 Hz, 2H), 2.87 (brd, $J$ = 11.0 Hz, 2H), 2.33 (brs, 2H), 1.34 (brs, 2H), 0.80-1.00(m, 1H).

**Step 4: Preparation of 2-(2,6-dioxopiperidin-3-yl)-5-(6-(hydroxymethyl)-3-azabicyclo[3.1.0]hexan-3-yl)isoindoline-1,3-dione (IM-24d)**

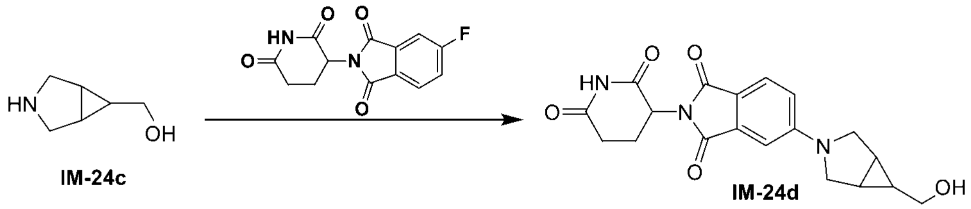

IM-24c IM-24d

At room temperature, Intermediate **IM-24c** (200 mg, 883 μmol) and 2-(2,6-dioxopiperidin-3-yl)-5-fluoroisoindoline-1,3-dione (244.10 mg, 883 μmol) were dissolved in *N*-methylpyrrolidone (5.00 mL) and added with diisopropylethylamine (342 mg, 2.65 mmol). The obtained reaction mixture was heated to 80°C and stirred for 12 h. The reaction mixture was concentrated under reduced pressure. The obtained residue was subjected to preparative HPLC (column: Waters Xbridge Prep OBD C18 150×40 mm × 10 μm; mobile phase: [$H_2O$ (10 mM $NH_4HCO_3$)-ACN]; gradient: 2%-40% B, 8.0 min) to obtain Intermediate **IM-24d** as a yellow solid (150 mg).

**Step 5: Preparation of 3-(2-(2,6-dioxopiperidin-3-yl)-1,3-dioxoisoindolin-5-yl)-3-azabicyclo[3.1.0]hexane-6-carbald ehyde (IM-24)**

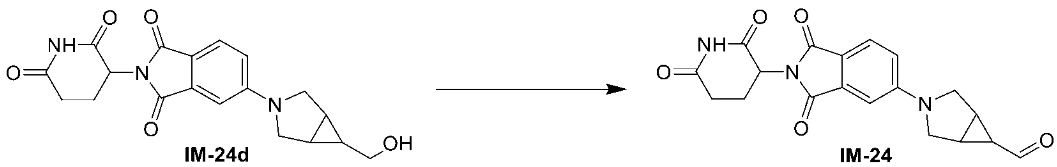

IM-24d IM-24

At room temperature, Intermediate **IM-24d** (150 mg, 391 μmol) was dissolved in dichloromethane (5.00 mL) and added with a Dess-Martin oxidizing agent (342 mg, 810 μmol). The reaction mixture was stirred for 12 h at 25°C. The reaction was quenched with water (20 mL) at 0°C and extracted with dichloromethane (90.0 mL). An organic layer was washed with an aqueous solution of sodium chloride (30 mL), dried over sodium sulfate, filtered, and concentrated under reduced pressure to obtain Intermediate **IM-24** as a yellow solid (100 mg).

**Example 1:**

***N*-2-(1-((1-(2-(2,6-dioxopiperidin-3-yl)-1,3-dioxoisoindolin-5-yl)piperidin-4-yl)met hyl)piperidin-4-yl)-7-(2-hydroxypropan-2-yl)imidazo[1,2-a]pyridin-6-yl)-6-(trifluoromethyl )pyridine-2-carboxamide**

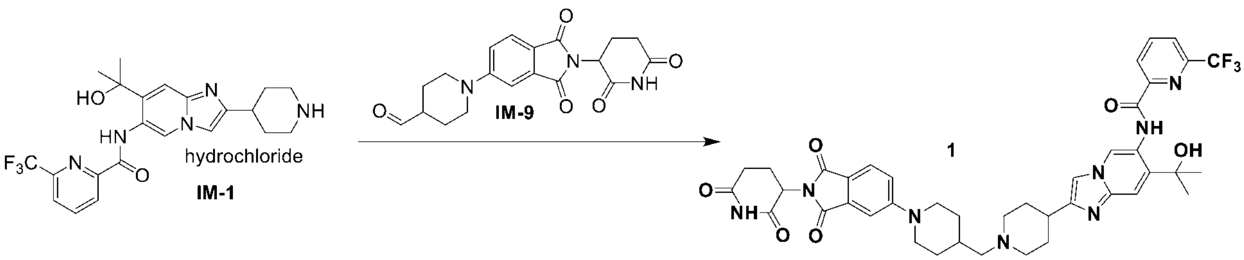


IM-1 IM-9 1

At 25°C, **IM-1** (100 mg, 206 μmol) and Compound **IM-9** (76.3 mg, 206 μmol) were dissolved in *N,N*-dimethylformamide (1.00 mL) and tetrahydrofuran (1.00 mL) and added with sodium acetate (25.4 mg, 309 μmol). The reaction mixture was stirred for 30 min and then added with sodium triacetoxyborohydride (131 mg, 619 μmol) and glacial acetic acid (37.2 mg, 619 μmol). The reaction mixture was stirred for 2 h at 25°C. The reaction mixture was filtered and concentrated. The obtained residue was purified through preparative HPLC (column: Waters Xbridge BEH C18 100×30 mm × 10 μm; mobile phase: [$H_2O$ ($NH_4HCO_3$)-ACN]; B%: 30%-60%, 8 min) to obtain Compound **1** as a yellow solid (30.0 mg).

$^1H$ NMR (400 MHz, DMSO-*d*$_6$) δ 12.22 (s, 1H), 11.08 (s, 1H), 9.54 (s, 1H), 8.34-8.50 (m, 2H), 8.19 (d, *J* = 7.6 Hz, 1H), 7.79 (s, 1H), 7.65 (d, *J* = 8.6 Hz, 1H), 7.41 (s, 1H), 7.31 (s, 1H), 7.23 (br d, *J* = 7.7 Hz, 1H), 6.16 (s, 1H), 5.06 (dd, *J* = 5.4, 12.8 Hz, 1H), 4.05 (br d, *J* = 12.0 Hz, 2H), 2.78-3.08 (m, 5H), 2.51-2.73 (m, 4H), 2.20 (br s, 1H), 1.92-2.02 (m, 4H), 1.81 (br d, *J* = 13.6 Hz, 2H), 1.69 (br d, *J* = 10.5 Hz, 2H), 1.60 (s, 6H), 1.41-1.55 (m, 1H), 1.06-1.28 (m, 3H).

MS (ESI+) m/z 801.3 $[M+H]^+$.

**Example 2:**

*N*-(2-(1-(2-(4-(2-(2,6-dioxopiperidin-3-yl)-1,3-dioxoisoindolin-5-yl)piperazin-1-yl)-2-o xoethyl)piperidin-4-yl)-7-(2-hydroxypropan-2-yl)imidazo[1,2-a]pyridin-6-yl)-6-(trifluoromethyl) pyridine-2-carboxamide

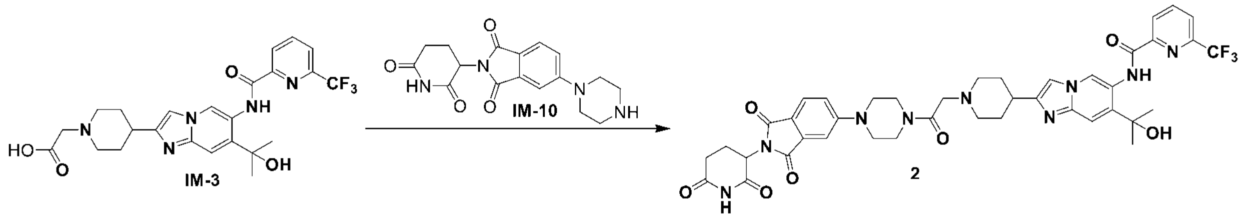

At 25°C, Intermediate **IM-3** (100 mg, 197 μmol) and Intermediate **IM-10** (54.1 mg, 158 μmol) were dissolved in *N,N*-dimethylformamide (2.00 mL) and added with *N,N*-diisopropylethylamine (102 mg, 791 μmol). The reaction mixture was stirred for 30 min at 25°C. O-(7-azabenzotriazol-1-yl)-*N,N,N,N*-tetramethyluronium hexafluorophosphate (112 mg, 296 μmol) was added to the reaction mixture, and the reaction mixture was stirred for 2.5 h at 25°C. The reaction mixture was concentrated under reduced pressure. The obtained residue was purified through HPLC (column: Waters Xbridge BEH C18 100×30 mm × 10 μm; mobile phase: [$H_2O$ ($NH_4HCO_3$)-ACN]; B%: 40%-60%, 8 min) to obtain Compound 2 as a yellow solid (28.0 mg).

$^1H$ NMR (400 MHz, DMSO-*d*$_6$) δ 12.01-12.40 (m, 1H), 9.54 (s, 1H), 8.36-8.47 (m, 2H), 8.19 (d, *J* = 7.6 Hz, 1H), 7.79 (s, 1H), 7.71 (d, *J* = 8.5 Hz, 1H), 7.40 (br d, *J* = 9.9 Hz, 2H), 7.29 (br d, *J* = 8.7 Hz, 1H), 5.08 (br dd, *J* = 5.2, 12.9 Hz, 1H), 3.78 (br s, 2H), 3.47-3.65 (m, 6H), 3.23 (br s, 4H), 2.84-2.96 (m, 3H), 2.58-2.70 (m, 2H), 2.12-2.21 (m, 2H), 1.93-2.05 (m, 3H), 1.65-1.74 (m, 2H), 1.60 (s, 6H), 1.22 (br d, *J* = 7.2 Hz, 1H).

MS (ESI+) m/z 830.3 $[M+H]^+$.

**Example 3:**

*N*-(2-(1-(3-(4-(2-(2,6-dioxopiperidin-3-yl)-1,3-dioxoisoindolin-5-yl)piperazin-1-yl)-3-o xopropyl)piperidin-4-yl)-7-(2-hydroxypropan-2-yl)imidazo[1,2-a]pyridin-6-yl)-6-(trifluoromethy l)pyridine-2-carboxamide

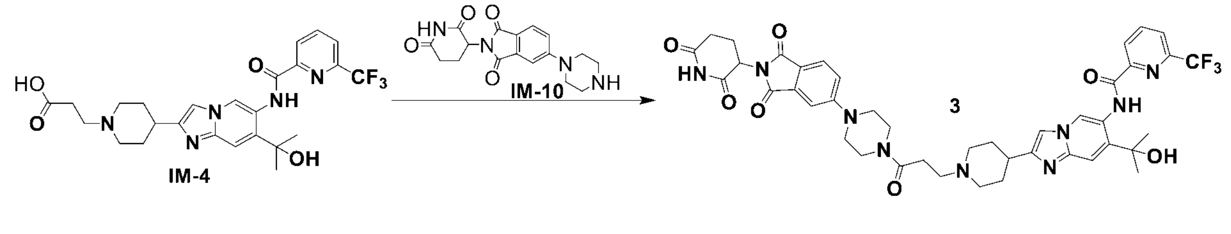

At 25°C, Compound **IM-4** (210 mg, 577 μmol) and Compound **IM-10** (158 mg, 461 μmol) were dissolved in *N,N*-dimethylformamide (4.00 mL) and added with *N,N*-diisopropylethylamine (298 mg, 2.31 mmol). The reaction mixture was

stirred for 30 min at 25°C. O-(7-azabenzotriazol-1-yl)-*N*,*N*,*N*,*N*-tetramethyluronium hexafluorophosphate (329 mg, 866 μmol) was added. The reaction mixture was stirred for 2.5 h at 25°C and concentrated under reduced pressure. The obtained residue was purified through HPLC (in a formic acid system) to obtain Compound 3 as a yellow solid (30.0 mg).

$^1$H NMR (400 MHz, DMSO-$d_6$) δ 12.22 (s, 1H), 11.08 (s, 1H), 9.54 (s, 1H), 8.34-8.50 (m, 2H), 8.14-8.22 (m, 1H), 7.64-7.87 (m, 2H), 7.33-7.45 (m, 2H), 7.26 (dd, *J* = 2.0, 8.4 Hz, 1H), 6.16 (s, 1H), 5.08 (dd, *J* = 5.3, 12.8 Hz, 1H), 3.64 (br d, *J* = 4.4 Hz, 5H), 3.04-3.13 (m, 3H), 2.81-2.94 (m, 2H), 2.54-2.80 (m, 8H), 2.34 (br d, *J* = 9.4 Hz, 2H), 1.96-2.07 (m, 3H), 1.68-1.79 (m, 2H), 1.60 (s, 6H).

MS (ESI+) m/z 844.4 $[M+H]^+$.

## Example 4:

*N*-(2-(1-(4-(4-(2-(2,6-dioxopiperidin-3-yl)-1,3-dioxoisoindolin-5-yl)piperazin-1-yl)-4-o xobutyl)piperidin-4-yl)-7-(2-hydroxypropan-2-yl)imidazo[1,2-a]pyridin-6-yl)-6-(trifluoromethyl )pyridine-2-carboxamide

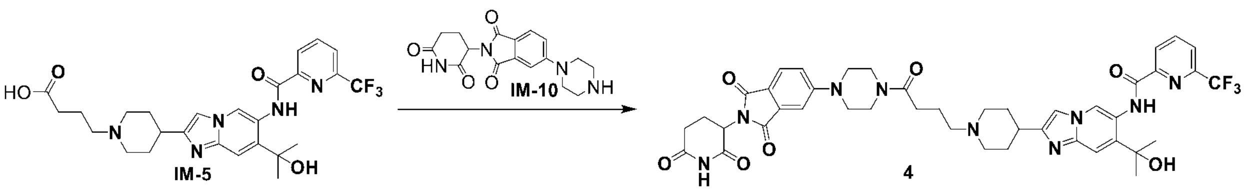


At 25°C, Intermediate **IM-5** (100 mg, 187 μmol) was dissolved in dimethylformamide (2.00 mL) and added with O-(7-azabenzotriazol-1-yl)-*N*,*N*,*N*,*N*-tetramethyluronium hexafluorophosphate (142 mg, 374 μmol) and N,N-diisopropylethylamine (121 mg, 937 μmol). The reaction mixture was stirred for 30 min, added with Intermediate **IM-10** (77.00 mg, 224 μmol), and stirred for 11.5 h at 25°C. The reaction mixture was concentrated under reduced pressure. The obtained residue was purified through preparative HPLC (column: Waters Xbridge BEH C18 100×30 mm × 10 μm; mobile phase: [$H_2O$ ($NH_4HCO_3$)-ACN]; B%: 25%-55%, 8 min) to obtain Compound **4** as a yellow solid (8.00 mg).

$^1$H NMR (400 MHz, DMSO-$d_6$) δ 12.23 (s, 1H), 11.09 (s, 1H), 9.56 (s, 1H), 8.33-8.50 (m, 2H), 8.14-8.21 (m, 1H), 7.85 (s, 1H), 7.71 (d, *J* = 8.4 Hz, 1H), 7.24-7.42 (m, 2H), 6.19 (s, 1H), 4.94-5.19 (m, 1H), 3.58-3.68 (m, 8H), 2.70-2.97 (m, 8H), 2.54-2.64 (m, 2H), 2.29-2.46 (m, 2H), 2.08-2.16 (m, 2H), 1.98-2.05 (m, 1H), 1.85 (br s, 5H), 1.60 (s, 6H).

MS (ESI+) m/z 858.3 $[M+H]^+$.

## Example 5:

*N*-(2-(1-(2-(2-(2-(2,6-dioxopiperidin-3-yl)-1,3-dioxoisoindolin-5-yl)-2,7-diazaspiro[3.5 ]nonan-7-yl)-2-oxoethyl)piperidin-4-yl)-7-(2-hydroxypropan-2-yl)imidazo[1,2-a]pyridin-6-yl)-6-(trifluoromethyl)pyridine-2-carboxamide

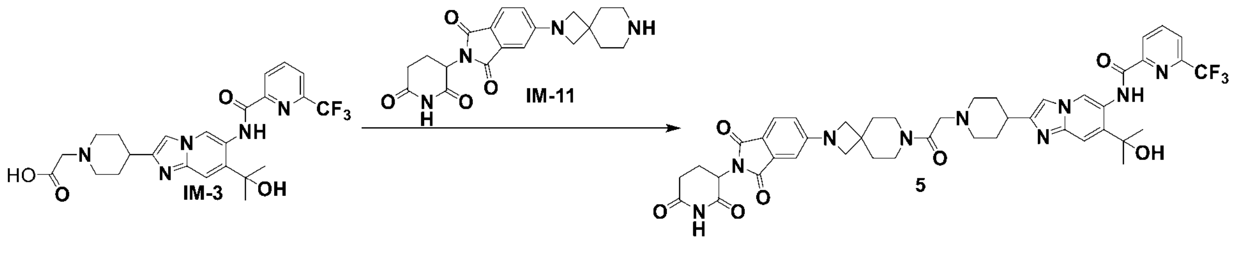


At room temperature, Intermediate **IM-3** (100 mg, 197 μmol) was dissolved in dimethylformamide (5.00 mL) and added with O-(7-azabenzotriazol-1-yl)-*N*,*N*,*N*,*N*-tetramethyluronium hexafluorophosphate (107 mg, 282 μmol) and N,N-diisopropylethylamine (109 mg, 847 μmol). The reaction mixture was stirred for 15 min and then added with Intermediate **IM-11** (54.0 mg, 141 μmol). The reaction mixture was stirred for 12 h at room temperature and concentrated under reduced pressure. The obtained residue was purified through preparative HPLC (column: Waters Xbridge BEH C18 250×50 mm × 10 μm; mobile phase: [$H_2O$ ($NH_4HCO_3$)-ACN]; B%: 25%-55%, 10 min) to obtain Compound **5** as a yellow solid (14.7 mg).

$^1$H NMR (400 MHz, DMSO-$d_6$) δ 12.22 (s, 1H), 11.06 (s, 1H), 9.54 (s, 1H), 8.50-8.33 (m, 2H), 8.19 (d, *J* = 7.8 Hz, 1H), 7.79 (s, 1H), 7.65 (d, *J* = 8.3 Hz, 1H), 7.41 (s, 1H), 6.77 (s, 1H), 6.65 (br d, *J* = 8.4 Hz, 1H), 6.14 (s, 1H), 5.05 (dd, *J* = 5.3,

12.9 Hz, 1H), 3.82 (s, 4H), 3.42-3.59 (m, 2H), 2.81-2.96 (m, 4H), 2.52-2.71 (m, 6H), 2.09-2.19 (m, 2H), 1.97 (br d, *J* = 10.8 Hz, 3H), 1.81 (br s, 2H), 1.65-1.75 (m, 4H), 1.60 (s, 6H).

MS (ESI+) m/z 870.3 $[M+H]^+$.

**Example 6:**

*N*-(2-(1-(2-(7-(2-(2,6-dioxopiperidin-3-yl)-1,3-dioxoisoindolin-5-yl)-2,7-diazaspiro[3.5 ]nonan-2-yl)-2-oxoethyl)piperidin-4-yl)-7-(2-hydroxypropan-2-yl)imidazo[1,2-a]pyridin-6-yl)-6-(trifluoromethyl)pyridine-2-carboxamide

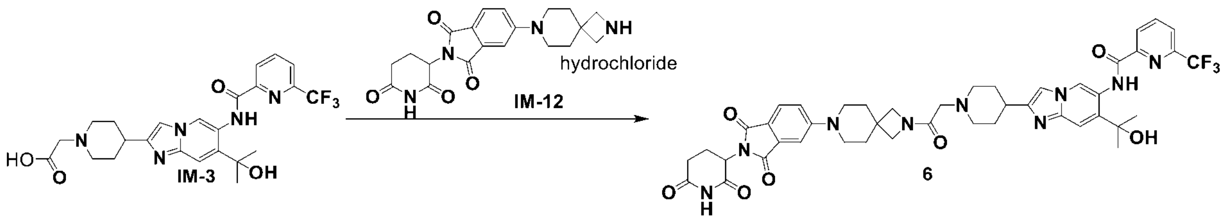


Intermediate **IM-3** (200 mg, 395 μmol) and O-(7-azabenzotriazol-1-yl)-*N*,*N*,*N*,*N*-tetramethyluronium hexafluorophosphate (90.7 mg, 238 μmol) were dissolved in dichloromethane (3.00 mL). The reaction mixture was stirred for 1 h at 25°C. Intermediate **IM-12** (100 mg, 238 μmol) and *N,N*-diisopropylethylamine (30.8 mg, 238 μmol) were added in sequence. The obtained reaction mixture was stirred for 2 h at 25°C. The reaction mixture was concentrated under reduced pressure. The resulting residue was purified through preparative HPLC (column: Waters Xbridge BEH C18 250×50 mm × 10 μm; mobile phase: [$H_2O$ ($NH_4HCO_3$)-ACN]; B%: 25%-55%, 10 min) to obtain Compound **6** as a yellow solid (17.8 mg).

$^1H$ NMR (400 MHz, DMSO-$d_6$) $\delta$ 12.22 (s, 1H), 11.08 (br s, 1H), 9.53 (s, 1H), 8.31-8.51 (m, 2H), 8.18 (d, *J* = 7.7 Hz, 1H), 7.79 (s, 1H), 7.65(d, *J* = 8.4 Hz, 1H), 7.21-7.46 (m, 3H), 6.16 (s, 1H), 5.05 (s, 1H), 3.98 (s, 2H), 3.63 (s, 2H), 3.47 (br s, 4H), 2.94-3.07 (m, 2H), 2.79-2.93 (m, 3H), 2.60 (br d, *J* = 2.7 Hz, 3H), 2.16 (br s, 2H), 1.96 (br d, *J* = 13.7 Hz, 3H), 1.53-1.82 (m, 12H).

MS (ESI+) m/z 870.3 $[M+H]^+$.

**Example 7:**

*N*-(2-(1-((1-(2-(2,6-dioxopiperidin-3-yl)-1-oxoisoindolin-5-yl)piperidin-4-yl)methyl)pi peridin-4-yl)-7-(2-hydroxypropan-2-yl)imidazo[1,2-a]pyridin-6-yl)-6-(trifluoromethyl)pyridine-2-carboxamide

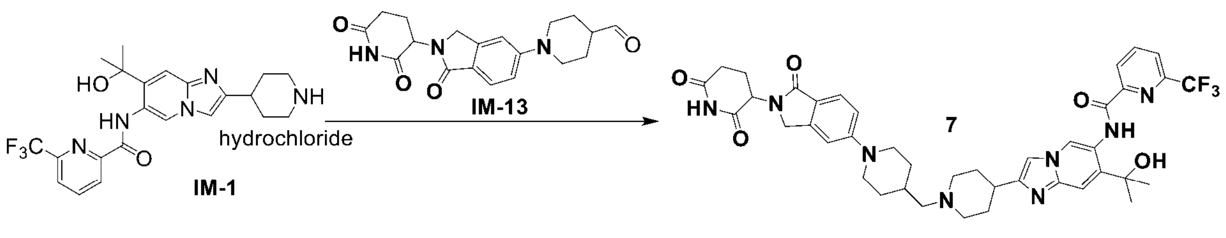


At 25°C, Intermediate **IM-1** (100 mg, 223 μmol) and Intermediate **IM-13** (79.4 mg, 223 μmol) were dissolved in N,N-dimethylformamide (2.00 mL) and tetrahydrofuran (2.00 mL) and added with sodium acetate (27.5 mg, 335 μmol). The reaction mixture was stirred for 30 min at 25°C. Sodium triacetoxyborohydride (27.5 mg, 335 μmol) and glacial acetic acid (40.2 mg, 670 μmol) were added. The reaction mixture was stirred for 11.5 h at 25°C. The reaction mixture was concentrated under reduced pressure. The obtained residue was purified through preparative HPLC (column: Waters Xbridge BEH C18 100×30 mm × 10 μm; mobile phase: [$H_2O$ ($NH_4HCO_3$)-ACN]; B%: 35%-65%, 8 min) to obtain Compound 7 as a white solid (42.0 mg).

$^1H$ NMR (400 MHz, DMSO-$d_6$) $\delta$ 12.22 (s, 1H), 10.93 (br s, 1H), 9.54 (s, 1H), 8.48-8.35 (m, 2H), 8.19 (br d, *J* = 7.8 Hz, 1H), 7.78 (s, 1H), 7.50 (br d, *J* = 8.6 Hz, 1H), 7.41 (s, 1H), 7.10-6.98 (m, 2H), 6.15 (s, 1H), 5.04 (br dd, *J* = 4.9, 13.1 Hz, 1H), 4.32 (br d, *J* = 17.0 Hz, 1H), 4.19 (br d, *J* = 16.6 Hz, 1H), 3.87 (br d, *J* = 11.9 Hz, 2H), 2.97-2.77 (m, 5H), 2.70-2.54 (m, 2H), 2.42-2.31 (m, 1H), 2.22-2.14 (m, 2H), 2.06-1.91 (m, 5H), 1.80 (br d, *J* = 10.9 Hz, 3H), 1.72-1.65 (m, 2H), 1.63-1.54 (m,

6H), 1.23 (br s, 1H), 1.19-1.10 (m, 1H).

MS (ESI+) m/z 787.4 $[M+H]^+$.

**Example 8:**

*N*-(2-(1-((1-(2-(2,6-dioxopiperidin-3-yl)-1,3-dioxoisoindolin-5-yl)piperidin-4-yl)methy l)piperidin-4-yl)-7-methoxyimidazo [1,2-a]pyridin-6-yl)-6-(trifluoromethyl)pyridine-2-carboxami de

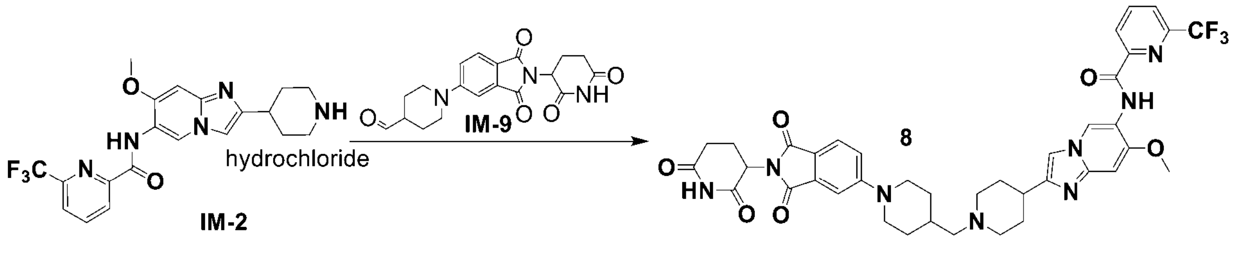


Intermediate **IM-2** (400 mg, 953 μmol) and Intermediate **IM-9** (422 mg, 1.14 mmol) were dissolved in *N,N*-dimethylformamide (4.00 mL) and added with sodium acetate (117 mg, 1.43 mmol). The reaction mixture was stirred for 30 min and then added with sodium triacetoxyborohydride (606 mg, 2.86 mmol) and acetic acid (171 mg, 2.86 mmol). The reaction mixture was stirred for 12 h at 25°C and diluted with water (10.0 mL). The resulting mixture was extracted with ethyl acetate (10 mL × 3). Combined organic phases were washed with saturated brine, dried over anhydrous sodium sulfate, filtered, and concentrated under reduced pressure. The obtained residue was purified through preparative HPLC (column: Phenomenex Luna C18 80×40 mm × 3 μm; mobile phase: [$H_2O$ (FA)-ACN]; B%: 1%-35%, 8 min) to obtain Compound **8** as a yellow solid (12.0 mg).

$^1H$ NMR (400 MHz, DMSO-$d_6$) δ 11.07 (s, 1H), 10.19 (s, 1H), 9.35 (s, 1H), 8.49-8.37 (m, 2H), 8.21-8.26 (m, 1H), 7.65 (t, *J* = 4.2 Hz, 2H), 7.31 (d, *J* = 1.6 Hz, 1H), 7.21-7.27 (m, 1H), 7.07 (s, 1H), 5.02-5.11 (m, 1H), 3.97-4.07 (m, 5H), 2.89-3.03 (m, 5H), 2.54-2.66 (m, 3H), 2.19 (br d, *J* = 6.6 Hz, 2H), 1.93-2.07 (m, 5H), 1.77-1.86 (m, 3H), 1.61-1.72 (m, 2H), 1.12-1.26 (m, 2H).

MS (ESI+) m/z 773.2 $[M+H]^+$.

**Example 9:**

*N*-(2-(1-(2-(4-(2-(2,6-dioxopiperidin-3-yl)-1,3-dioxoisoindolin-5-yl)piperazin-1-yl)ethy l)piperidin-4-yl)-7-(2-hydroxypropan-2-yl)imidazo[1,2-a]pyridin-6-yl)-6-(trifluoromethyl)pyridi ne-2-carboxamide

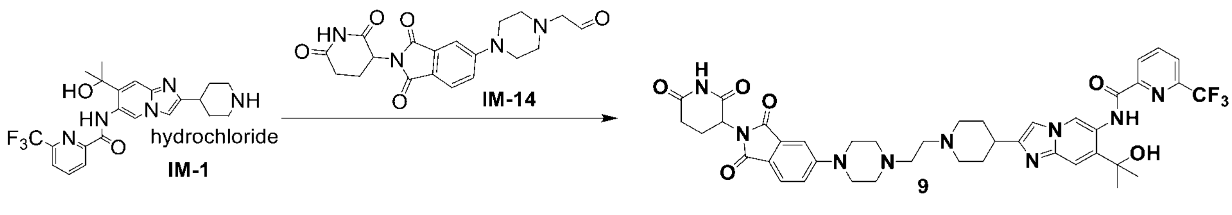


At 25°C, Intermediate **IM-1** (23.2 mg, 52.0 μmol) and Intermediate **IM-14** (20.0 mg, 52.0 μmol) were dissolved in *N,N*-dimethylformamide (1.00 mL) and 1,2-dichloroethane (1.00 mL) and added with *N,N*-diisopropylethylamine (20.1 mg, 156 μmol). The reaction mixture was stirred for 30 min at 25°C. Glacial acetic acid (9.37 mg, 156 μmol) and sodium triacetoxyborohydride (16.5 mg, 78.0 μmol) were added. The reaction mixture was stirred for 11.5 h at 25°C. Water (3.00 mL) was added, and the resulting mixture was extracted with ethyl acetate (5.00 mL × 3). Combined organic layers were dried over anhydrous sodium sulfate, filtered, and concentrated under reduced pressure. The obtained residue was purified through preparative HPLC (in a formic acid system) to obtain Compound **9** as a yellow solid (2.00 mg).

$^1H$ NMR (400 MHz, DMSO-$d_6$) δ 12.23 (s, 1H), 11.08 (br s, 1H), 9.54 (s, 1H), 8.32-8.52 (m, 2H), 8.10-8.29 (m, 2H), 7.79 (s, 1H), 7.68 (br d, *J* = 8.4 Hz, 1H), 7.20-7.47 (m, 3H), 6.15 (br s, 1H), 5.08 (br dd, *J* = 5.0, 12.6 Hz, 1H), 2.75-3.07 (m, 5H), 2.67-2.67 (m, 1H), 2.53-2.69 (m, 10H), 1.86-2.24 (m, 6H), 1.65-1.76 (m, 2H), 1.61 (s, 6H).

MS (ESI+) m/z 816.3 $[M+H]^+$.

**Example 10:**

*N*-(2-(1-(2-(4-(2-(2,6-dioxopiperidin-3-yl)-1-oxoisoindolin-5-yl)piperazin-1-yl)ethyl)pi peridin-4-yl)-7-(2-hydroxypropan-2-yl)imidazo[1,2-a]pyridin-6-yl)-6-(trifluoromethyl)pyridine-2-carboxamide

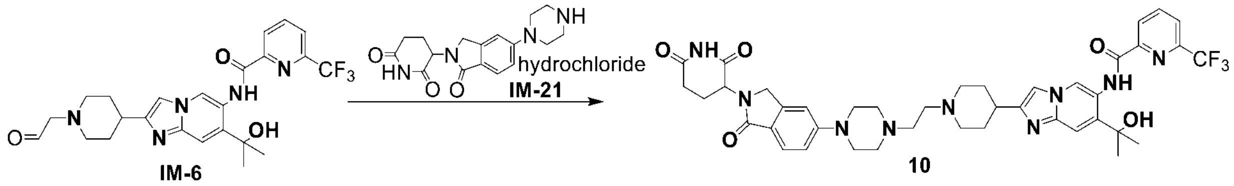


At 25°C, Intermediate **IM-6** (40.2 mg, 122 μmol) was dissolved in 1,2-dichloroethane (1.00 mL) and *N,N*-dimethylformamide (1.00 mL) and added with triethylamine (31.0 mg, 306 μmol). The reaction mixture was stirred for 0.5 h at 25°C. Acetic acid (6.13 mg, 102 μmol), sodium triacetoxyborohydride (32.4 mg, 153 μmol), and Intermediate **IM-21** (50.0 mg, 102 μmol) were added. The reaction mixture was stirred for 11.5 h at 25°C and concentrated under reduced pressure. The obtained residue was purified through preparative HPLC (column: Phenomenex Luna C18 75×30 mm × 3 μm; mobile phase: [$H_2O$ (FA)-ACN]; B%: 5%-35%, 8 min) to obtain Compound **10** as a white solid (12.0 mg).

$^1$H NMR (400 MHz, DMSO-*d*$_6$) δ 12.22 (s, 1H), 10.49-11.50 (m, 1H), 9.54 (s, 1H), 8.36-8.47 (m, 2H), 8.19 (d, *J* = 8.1 Hz, 1H), 7.78 (s, 1H), 7.44-7.58 (m, 1H), 7.41 (s, 1H), 7.01-7.12 (m, 2H), 6.15 (s, 1H), 5.05 (dd, *J* = 5.0, 13.3 Hz, 1H), 4.33 (d, *J* = 17.0 Hz, 1H), 4.20 (d, *J* = 16.8 Hz, 1H), 3.22-3.32 (m, 8H), 2.84-3.02 (m, 3H), 2.53-2.68 (m, 6H), 2.28-2.46 (m, 2H), 2.04-2.13 (m, 2H), 1.93-1.98 (m, 2H), 1.63-1.72 (m, 2H), 1.60 (s, 6H).

MS (ESI+) m/z 802.3 [M+H]$^+$.

**Example 11:**

*N*-(2-(1-(2-(4-(2-(2,6-dioxopiperidin-3-yl)-1,3-dioxoisoindolin-5-yl)piperazin-1-yl)ethy l)piperidin-4-yl)-7-methoxyimidazo[1,2-a]pyridin-6-yl)-6-(trifluoromethyl)pyridine-2-carboxami de

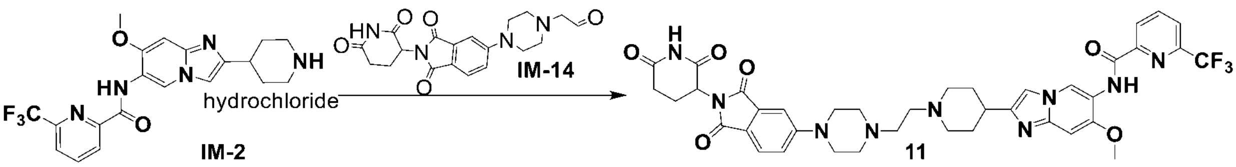


At room temperature, Intermediate **IM-2** (50.0 mg, 119 μmol) was dissolved in *N,N*-dimethylformamide (1.00 mL) and 1,2-dichloroethane (1.00 mL) and added with *N,N*-diisopropylethylamine (61.0 mg, 476 μmol). The reaction mixture was stirred for 15 min. Sodium triacetoxyborohydride (75.8 mg, 357 μmol), glacial acetic acid (7.16 mg, 119 μmol), and Intermediate **IM-14** (45.8 mg, 119 μmol) were added. The reaction mixture was stirred for 12 h at room temperature and concentrated under reduced pressure. The obtained residue was purified through preparative HPLC (column: Phenomenex Luna C18 80×40 mm × 3 μm; mobile phase: [$H_2O$ (FA)-ACN]; B%: 1%-35%, 8 min) to obtain Compound **11** as a yellow solid (7.50 mg).

$^1$H NMR (400 MHz, DMSO-*d*$_6$) δ 11.08 (br s, 1H), 10.19 (s, 1H), 9.35 (s, 1H), 8.37-8.49 (m, 2H), 8.23 (br d, *J* = 7.5 Hz, 1H), 7.57-7.75 (m, 2H), 7.19-7.43 (m, 2H), 7.07 (s, 1H), 5.01-5.15 (m, 1H), 3.98 (s, 3H), 3.46-3.49 (m, 4H), 2.82-3.03 (m, 4H), 2.52-2.71 (m, 9H), 1.89-2.32 (m, 6H), 1.59-1.73 (m, 2H).

MS (ESI+) m/z 788.3 [M+H]$^+$.

**Example 12:**

*N*-(2-(1-(2-(4-(2-(2,6-dioxopiperidin-3-yl)-1-oxoisoindolin-5-yl)piperazin-1-yl)ethyl)pi peridin-4-yl)-7-methoxyimidazo [1,2-a]pyridin-6-yl)-6-(trifluoromethyl)pyridine-2-carboxamide

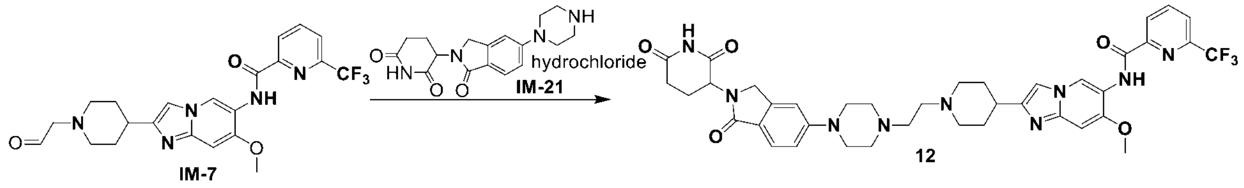


Intermediate **IM-21** (39.1 mg, 119 μmol) was dissolved in N,N-dimethylformamide (1.00 mL) and dichloroethane (1.00 mL) and added with N,N-diisopropylethylamine (70.0 mg, 541 μmol). The reaction mixture was stirred for 30 min at 25°C. Sodium triacetoxyborohydride (68.9 mg, 325 μmol), acetic acid (6.51 mg, 108 μmol), and Intermediate **IM-7** (50.0 mg, 108 μmol) were added. The reaction mixture was stirred for 11.5 h at 25°C and concentrated under reduced pressure. The obtained residue was purified through preparative HPLC (column: Waters Xbridge BEH C18 100×30 mm × 10 μm; mobile phase: [$H_2O$ (10 mM $NH_4HCO_3$)-ACN]; gradient: 20%-50% B, 8.0 min) to obtain Compound **12** as a white solid (8.00 mg).

$^1H$ NMR (400 MHz, DMSO-$d_6$) δ 10.93 (br s, 1H), 10.19 (s, 1H), 9.35 (s, 1H), 8.37-8.49 (m, 2H), 8.23 (br d, *J* = 7.8 Hz, 1H), 7.64 (s, 1H), 7.52 (d, *J* = 8.6 Hz, 1H), 7.07 (s, 2H), 4.98-5.11 (m, 1H), 4.17-4.36 (m, 2H), 3.98 (s, 3H), 3.29 (br d, *J* = 3.6 Hz, 6H), 2.84-3.02 (m, 4H), 2.56-2.68 (m, 8H), 2.33-2.43 (m, 1H), 2.08-2.20 (m, 2H), 1.89-2.02 (m, 3H), 1.58-1.74 (m, 2H).

MS (ESI+) m/z 774.3 $[M+H]^+$.

**Example 13:**

*N*-(2-(1-(2-(4-(2-(2,6-dioxopiperidin-3-yl)-3-oxoisoindolin-5-yl)piperazin-1-yl)ethyl)pi peridin-4-yl)-7-(2-hydroxypropan-2-yl)imidazo[1,2-a]pyridin-6-yl)-6-(trifluoromethyl)pyridine-2-carboxamide

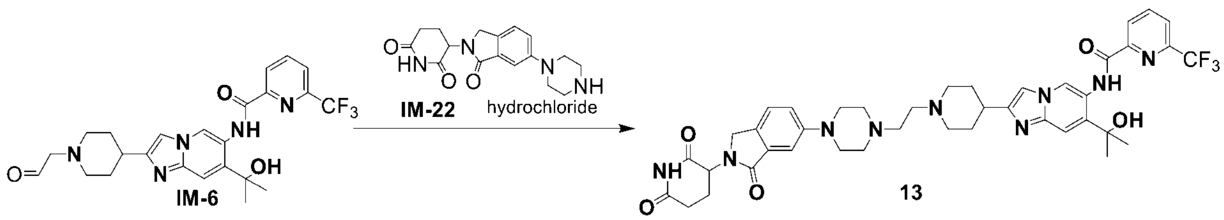


At 25°C, Intermediate **IM-6** (161 mg, 328 μmol) was dissolved in 1,2-dichloroethane (5.00 mL) and *N,N*-dimethylformamide (5.00 mL) and added with triethylamine (106 mg, 822 μmol). The reaction mixture was stirred for 0.5 h. Acetic acid (16.4 mg, 274 μmol), sodium triacetoxyborohydride (87.0 mg, 410 μmol), and Intermediate **IM-22** (100 mg, 274 μmol) were added. The reaction mixture was stirred for 12 h at 25°C and concentrated under reduced pressure. The obtained residue was purified through preparative HPLC (column: Phenomenex Gemini NX-C18 75×30 mm × 3 μm; mobile phase: [$H_2O$ (0.05% $NH_3H_2O$ + 10 mM $NH_4HCO_3$)-ACN]; gradient: 20%-50% B, 8.0 min) to obtain Compound 13 as an off-white solid (10.2 mg).

$^1H$ NMR (400 MHz, DMSO-$d_6$) δ 12.22 (s, 1H), 10.97 (br s, 1H), 9.54 (s, 1H), 8.36-8.48 (m, 2H), 8.19 (d, *J* = 7.7 Hz, 1H), 7.78 (s, 1H), 7.38-7.46 (m, 2H), 7.21-7.31 (m, 1H), 7.11-7.20 (m, 1H), 6.15 (s, 1H), 5.10 (dd, *J* = 5.1, 13.3 Hz, 1H), 4.33 (d, *J* = 16.8 Hz, 1H), 4.20 (d, *J* = 16.8 Hz, 1H), 3.11-3.26 (m, 4H), 2.84-3.04 (m, 3H), 2.67 (br d, *J* = 1.5 Hz, 1H), 2.58 (br s, 4H), 2.50 (br s, 4H), 2.29-2.46 (m, 2H), 2.07-2.20 (m, 2H), 1.88-2.02 (m, 3H), 1.63-1.77 (m, 2H), 1.60 (s, 6H).

MS (ESI+) m/z 802.3 $[M+H]^+$.

**Example 14:**

*N*-(2-(1-(2-(4-(2-(2,6-dioxopiperidin-3-yl)-3-oxoisoindolin-5-yl)piperazin-1-yl)ethyl)pi peridin-4-yl)-7-methoxyimidazo [1,2-a]pyridin-6-yl)-6-(trifluoromethyl)pyridine-2-carboxamide

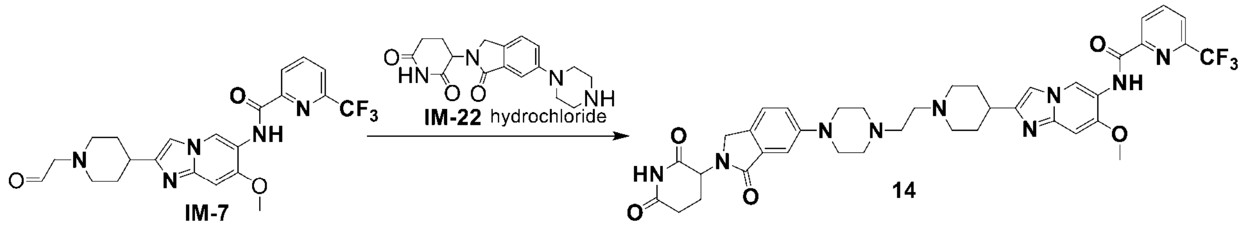


Intermediate **IM-22** (39.1 mg, 119 μmol) was dissolved in N,N-dimethylformamide (0.50 mL) and 1,2-dichloroethane (0.5 mL) and added with N,Ndiisopropylethylamine (56.0 mg, 433 μmol), Intermediate **IM-7** (50.0 mg, 108 μmol), sodium triacetoxyborohydride (68.9 mg, 325 μmol), and glacial acetic acid (6.51 mg, 108 μmol). The reaction mixture was stirred for 12 h at 25°C and concentrated under reduced pressure. The obtained residue was purified through preparative HPLC (column: Phenomenex Luna C18 80×40 mm × 3 μm; mobile phase: [$H_2O$ (FA)-ACN]; gradient: 1%-35% B, 8 min) to obtain Compound **14** as a yellow solid (8.00 mg).

$^1H$ NMR (400 MHz, DMSO-*d*$_6$) δ 10.97 (s, 1H), 10.19 (s, 1H), 9.35 (s, 1H), 8.38-8.47 (m, 2H), 8.21-8.26 (m, 1H), 7.65 (s, 1H), 7.43 (d, *J* = 8.5 Hz, 1H), 7.24-7.29 (m, 1H), 7.16 (d, *J* = 2.0 Hz, 1H), 7.07 (s, 1H), 5.05-5.13 (m, 1H), 4.30-4.38 (m, 1H), 4.17-4.24 (m, 1H), 3.98 (s, 3H), 3.19 (br s, 4H), 2.85-3.02 (m, 4H), 2.58-2.61 (m, 4H), 2.53 (br s, 4H), 2.32-2.43 (m, 2H), 2.16 (br t, *J* = 10.8 Hz, 2H), 1.93-2.01 (m, 3H), 1.62-1.73 (m, 2H).

MS (ESI+) m/z 774.3 $[M+H]^+$.

**Example 15:**

*N*-(2-(1-((1-(2-(2,6-dioxopiperidin-3-yl)-6-fluoro-1,3-dioxoisoindolin-5-yl)piperidin-4-yl)methyl)piperidin-4-yl)-7-(2-hydroxypropan-2-yl)imidazo[1,2-a]pyridin-6-yl)-6-(trifluorometh yl)pyridine-2-carboxamide

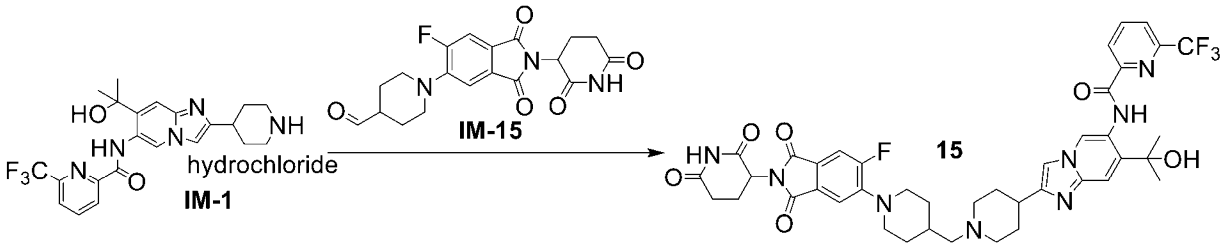


At room temperature, Intermediate **IM-1** (173 mg, 387 μmol) was dissolved in *N,N*-dimethylformamide (1.00 mL) and tetrahydrofuran (1.00 mL) and added with sodium acetate (47.6 mg, 580 μmol), Intermediate **IM-15** (150 mg, 387 μmol), sodium triacetoxyborohydride (246 mg, 1.16 mmol), and sodium acetate (69.7 mg, 1.16 mmol). The reaction mixture was stirred for 12 h at 25°C and concentrated under reduced pressure. The obtained residue was purified through preparative HPLC (column: Waters Xbridge BEH C18 250×50 mm × 10 m; mobile phase: [$H_2O$ ($NH_4HCO_3$)-ACN]; B%: 25%-55%, 10 min) to obtain Compound 15 as a yellow solid (11.0 mg).

$^1H$ NMR (400 MHz, DMSO-d$_6$) δ 12.23 (s, 1H), 11.12 (br s, 1H), 9.54 (s, 1H), 8.29-8.53 (m, 2H), 8.18 (br d, *J* = 7.5 Hz, 1H), 7.63-7.83 (m, 2H), 7.38-7.50 (m, 2H), 6.17 (s, 1H), 5.00-5.18 (m, 1H), 3.60 (br d, *J* = 11.2 Hz, 2H), 2.81-2.97 (m, 5H), 2.52-2.67 (m, 3H), 2.19 (br d, *J* = 5.4 Hz, 2H), 1.92-2.06 (m, 5H), 1.65-1.87 (m, 5H), 1.60 (br s, 6H), 1.25 (br d, *J* = 10.4 Hz, 2H).

MS (ESI+) m/z 819.3 $[M+H]^+$.

**Example 16:**

*N*-(2-(1-(2-(2-(2-(2,6-dioxopiperidin-3-yl)-1,3-dioxoisoindolin-5-yl)-2,7-diazaspiro[3.5 ]nonan-7-yl)ethyl)piperidin-4-yl)-7-(2-hydroxypropan-2-yl)imidazo[1,2-a]pyridin-6-yl)-6-(triflu oromethyl)pyridine-2-carboxamide

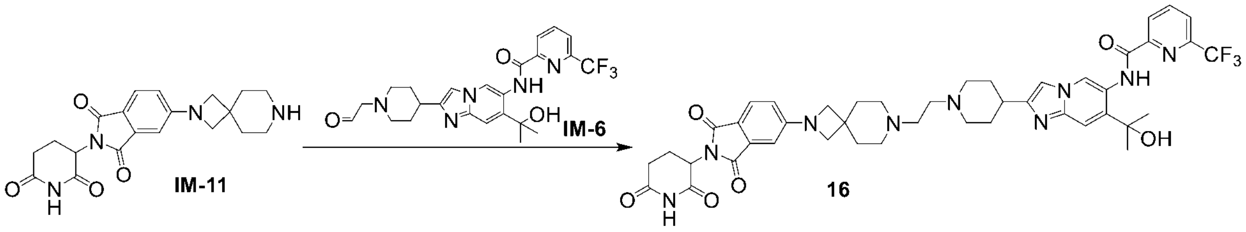

IM-11

16

At room temperature, Intermediate **IM-11** (200 mg, 402 μmol) was dissolved in *N,N*-dimethylformamide (2.00 mL) and 1,2-dichloroethane (2.00 mL) and added with *N,N*-diisopropylethylamine (208 mg, 1.61 mmol). The reaction mixture was stirred for 15 min and then added with Intermediate **IM-6** (197 mg, 402 μmol), sodium triacetoxyborohydride (256 mg, 1.21 mmol), and acetic acid (24.19 mg, 402 μmol). The reaction mixture was stirred for 12 h at room temperature and diluted with water (10 mL). An aqueous phase was extracted with ethyl acetate (10 mL × 3). Combined organic phases were washed with saturated brine, dried over anhydrous sodium sulfate, filtered, and concentrated under reduced pressure. The obtained residue was purified through preparative HPLC (column: Waters Xbridge Prep OBD C18 150×40 mm × 10 μm; mobile phase: [$H_2O$ (10 mM $NH_4HCO_3$)-ACN]; gradient: 30%-65% B, 8.0 min) to obtain Compound **16** as a yellow solid (18.0 mg).

$^1H$ NMR (400 MHz, DMSO-$d_6$) δ 12.22 (s, 1H), 11.06 (br s, 1H), 9.53 (s, 1H), 8.35-8.48 (m, 2H), 8.19 (d, *J* = 7.8 Hz, 1H), 7.78 (s, 1H), 7.63 (d, *J* = 8.3 Hz, 1H), 7.41 (s, 1H), 6.77 (d, *J* = 1.6 Hz, 1H), 6.61-6.69 (m, 1H), 6.15 (s, 1H), 4.99-5.11 (m, 1H), 3.74 (s, 3H), 2.82-2.59 (m, 3H), 2.54-2.68 (m, 3H), 2.33-2.46 (m, 8H), 1.90-2.14 (m, 6H), 1.63-1.77 (m, 6H), 1.60 (s, 6H).

**Example 17:**

*N*-(2-(1-(2-(7-(2-(2,6-dioxopiperidin-3-yl)-1,3-dioxoisoindolin-5-yl)-2,7-diazaspiro[3.5 ]nonan-2-yl)ethyl)piperidin-4-yl)-7-(2-hydroxypropan-2-yl)imidazo[1,2-a]pyridin-6-yl)-6-(triflu oromethyl)pyridine-2-carboxamide

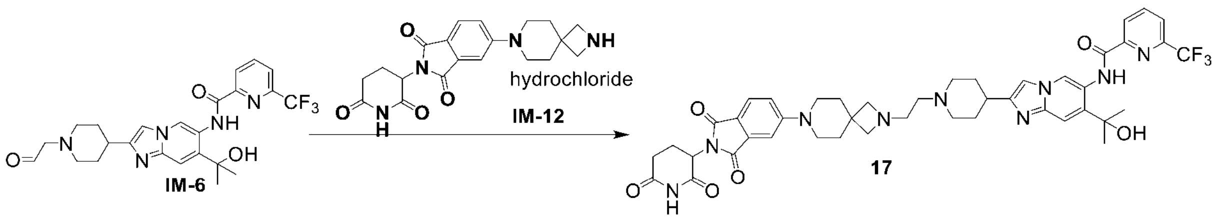

IM-6

17

At 25°C, Compound **IM-12** (150 mg, 302 μmol) and DIEA (156 mg, 1.21 mmol) were dissolved in 1,2-dichloroethane (2.00 mL) and N,N-dimethylformamide (2.00 mL). The resulting mixture was stirred for 0.5 h. Sodium triacetoxyborohydride (192 mg, 906 μmol), Compound **IM-6** (158 mg, 302 μmol), and acetic acid (18.1 mg, 302 μmol) were added in sequence. The reaction mixture was stirred for 12 h at 25°C and concentrated under reduced pressure. The obtained residue was purified through preparative HPLC (column: Phenomenex Gemini NX-C18 75×30 mm × 3 μm; mobile phase: [$H_2O$ (0.05% $NH_3H_2O$ + 10 mM $NH_4HCO_3$)-ACN]; gradient: 20%-40% B, 8.0 min) to obtain Compound **17** as an off-white solid (6.00 mg).

$^1H$ NMR (400 MHz, DMSO-$d_6$) δ 12.22 (s, 1H), 9.54 (s, 1H), 8.33-8.52 (m, 2H), 8.25 (s, 1H), 8.19 (d, *J* = 7.6 Hz, 1H), 7.78 (s, 1H), 7.65 (d, *J* = 8.6 Hz, 1H), 7.40 (s, 1H), 7.32 (s, 1H), 7.24 (br d, *J* = 8.8 Hz, 1H), 5.93-6.34 (m, 1H), 5.06 (dd, *J* = 5.4, 12.9 Hz, 1H), 3.44 (br s, 5H), 3.13 (s, 4H), 2.84-2.98 (m, 3H), 2.53-2.72 (m, 5H), 2.34 (br t, *J* = 6.2 Hz, 2H), 2.10 (br t, *J* = 10.9 Hz, 2H), 1.91-2.03 (m, 3H), 1.76 (br s, 4H), 1.67 (br d, *J*= 10.4 Hz, 1H), 1.60 (s, 6H).

**Example 18:**

*N*-(2-(1-(2-(1-(2-(2,6-dioxopiperidin-3-yl)-1,3-dioxoisoindolin-5-yl)piperidin-4-yl)ethy l)piperidin-4-yl)-7-(2-hydroxypropan-2-yl)imidazo[1,2-a]pyridin-6-yl)-6-(trifluoromethyl)pyridi ne-2-carboxamide

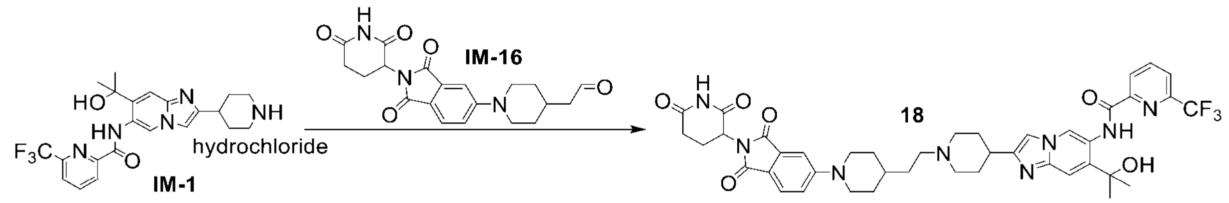


Compound **IM-1** (200 mg, 413 μmol) was dissolved in dichloroethane (2.00 mL) and *N,N*-dimethylformamide (2.00 mL) and added with *N,N*-diisopropylethylamine (213 mg, 1.65 mmol). The reaction mixture was stirred for 5 min. Intermediate **IM-16** (290 mg, 454 μmol), sodium triacetoxyborohydride (262 mg, 1.24 mmol), and acetic acid (24.8 mg, 413 μmol) were added. The reaction mixture was stirred for 12 h at 25°C and concentrated to dryness under reduced pressure. The obtained residue was purified through HPLC (in a formic acid system) to obtain Compound **18** as a yellow solid (21.0 mg).

$^1$H NMR (400 MHz, DMSO-$d_6$) δ 12.22 (s, 1H), 11.07 (s, 1H), 9.54 (s, 1H), 8.32-8.50 (m, 2H), 8.17-8.19 (m, 1H), 7.78 (s, 1H), 7.65 (d, *J* = 8.5 Hz, 1H), 7.41 (s, 1H), 7.30 (s, 1H), 7.21-7.25 (m, 1H), 6.14 (s, 1H), 4.98-5.13 (m, 1H), 4.04 (br d, *J* = 12.8 Hz, 2H), 2.82-3.00 (m, 5H), 2.55-2.68 (m, 3H), 2.32-2.44 (m, 2H), 1.93-2.12 (m, 5H), 1.62-1.82 (m, 5H), 1.60 (s, 6H), 1.38-1.48 (m, 2H), 1.13-1.26 (m, 2H).

MS (ESI+) m/z 815.3 [M+H]$^+$.

### Example 19:

*N*-(2-(1-(3-(4-(2-(2,6-dioxopiperidin-3-yl)-1,3-dioxoisoindolin-5-yl)piperazin-1-yl)pro pyl)piperidin-4-yl)-7-(2-hydroxypropan-2-yl)imidazo[1,2-a]pyridin-6-yl)-6-(trifluoromethyl)pyri dine-2-carboxamide

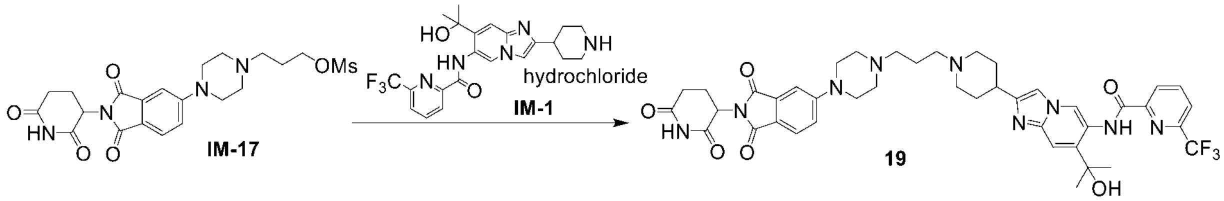


At room temperature, Intermediate **IM-1** (85.0 mg, 189 μmol) was dissolved in N,N-dimethylformamide (3.00 mL) and added with *N,N*-diisopropylethylamine (73.6 mg, 569 μmol), sodium iodide (85.43 mg, 569 μmol), and Intermediate **IM-17** (200 mg, 208 μmol). The reaction mixture was heated to 80°C, stirred for 12 h, and concentrated under reduced pressure. The obtained residue was purified through HPLC (in an ammonium bicarbonate system) to obtain Compound **19** (15.0 mg).

$^1$H NMR (400 MHz, DMSO-$d_6$) δ 12.22 (s, 1H), 11.07 (br s, 1H), 9.54 (s, 1H), 8.35-8.48 (m, 2H), 8.19 (d, *J* = 7.7 Hz, 1H), 7.62-7.85 (m, 2H), 7.24-7.43 (m, 3H), 6.13 (s, 1H), 4.99-5.15 (m, 1H), 3.44 (br s, 4H), 2.83-2.95 (m, 3H), 2.52-2.69 (m, 6H), 2.31-2.38 (m, 4H), 1.94-2.05 (m, 5H), 1.62-1.74 (m, 5H), 1.60 (s, 6H).

MS (ESI+) m/z 830.4 [M+H]$^+$.

### Example 20:

*N*-(2-(1-(1-(1-(2-(2,6-dioxopiperidin-3-yl)-1,3-dioxoisoindolin-5-yl)piperidin-4-yl)azeti din-3-yl)piperidin-4-yl)-7-(2-hydroxypropan-2-yl)imidazo[1,2-a]pyridin-6-yl)-6-(trifluoromethyl )pyridine-2-carboxamide

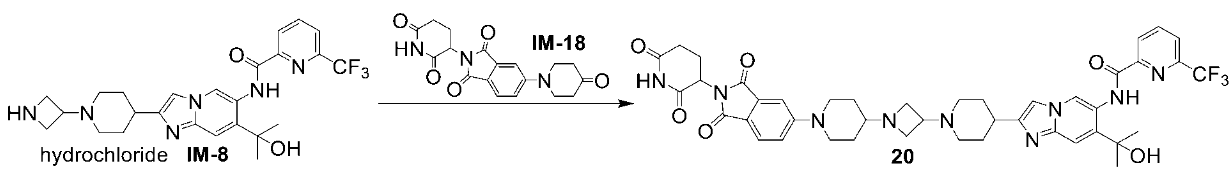


Intermediate **IM-8** (100 mg, 185 μmol) was dissolved in N,N-dimethylformamide (2.00 mL) and tetrahydrofuran

(2.00 mL) and added with sodium acetate (30.4 mg, 371 μmol). The reaction mixture was stirred for 15 min and then added with sodium triacetoxyborohydride (117 mg, 556 μmol), Intermediate **IM-18** (82.4 mg, 185 μmol), and acetic acid (33.4 mg, 556 μmol). The reaction mixture was stirred for 12 h at 25°C and suction-filtered. The filtrate was concentrated under reduced pressure. The obtained residue was purified through HPLC (in an ammonium bicarbonate system) to obtain Compound **20** as a yellow solid (25 mg).

$^1$H NMR (400 MHz, DMSO-$d_6$) δ 12.22 (s, 1H), 11.07 (s, 1H), 9.54 (s, 1H), 8.34-8.52 (m, 2H), 8.19 (d, *J* = 7.7 Hz, 1H), 7.78 (s, 1H), 7.65 (d, *J* = 8.6 Hz, 1H), 7.40 (s, 1H), 7.31 (s, 1H), 7.19-7.26 (m, 1H), 6.14 (s, 1H), 5.00-5.12 (m, 1H), 3.79-3.88 (m, 2H), 3.40 (br t, *J* = 4.8 Hz, 2H), 3.07-3.18 (m, 2H), 2.75-2.94 (m, 6H), 2.54-2.68 (m, 3H), 2.25-2.33 (m, 1H), 1.82-2.07 (m, 6H), 1.64-1.73 (m, 3H), 1.60 (s, 6H), 1.18-1.27 (m, 2H).

MS (ESI+) m/z 842.4 [M+H]$^+$.

**Example 21:**

*N*-(2-(1-(2-(6-(2-(2,6-dioxopiperidin-3-yl)-1,3-dioxoisoindolin-5-yl)-2,6-diazaspiro[3.3 ]heptan-2-yl)ethyl)piperidin-4-yl)-7-(2-hydroxypropan-2-yl)imidazo[1,2-a]pyridin-6-yl)-6-(triflu oromethyl)pyridine-2-carboxamide

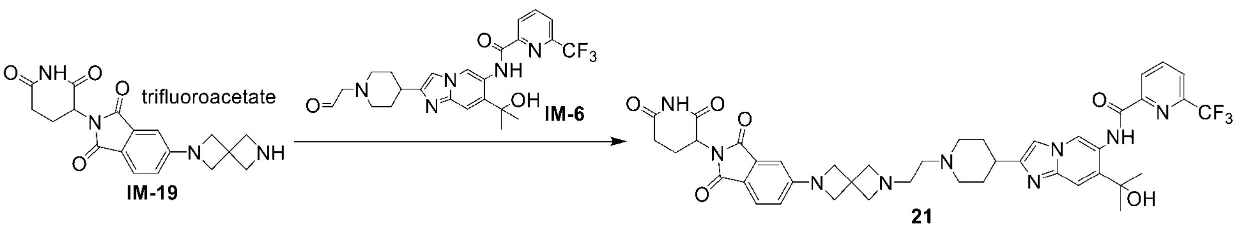


At 25°C, Intermediate **IM-19** (100 mg, 282 μmol) and *N,N*-diisopropylethylamine (100 mg, 282 μmol) were dissolved in 1,2-dichloroethane (2.00 mL) and *N,N*-dimethylformamide (2.00 mL). The reaction mixture was stirred for 0.5 h. Sodium triacetoxyborohydride (179 mg, 846 μmol), Compound **IM-6** (100 mg, 282 μmol), and acetic acid (16.9 mg, 282 μmol) were added in sequence. The reaction mixture was stirred for 12 h at 25°C and concentrated under reduced pressure. The obtained residue was purified through preparative HPLC (column: Waters Xbridge BEH C18 100×30 mm × 10 μm; mobile phase: [$H_2O$ (10 mM $NH_4HCO_3$)-ACN]; gradient: 20%-65% B, 8.0 min). The obtained crude product was purified through preparative HPLC (column: Phenomenex Luna C18 100×40 mm × 5 μm; mobile phase: [$H_2O$ (0.2% FA)-ACN]; gradient: 5%-45% B, 8.0 min) to obtain Compound **21** as a white solid (10.4 mg).

$^1$H NMR (400 MHz, DMSO-$d_6$) *δ* 12.22 (s, 1H), 11.06 (s, 1H), 9.53 (s, 1H), 8.36-8.47 (m, 2H), 8.16-8.24 (m, 1H), 7.77 (s, 1H), 7.64 (d, *J* = 8.2 Hz, 1H), 7.40 (s, 1H), 6.79 (s, 1H), 6.65 (br d, *J* = 8.2 Hz, 1H), 6.13 (s, 1H), 5.05 (dd, *J* = 5.4, 12.7 Hz, 1H), 4.10 (s, 4H), 3.33-3.34 (m, 4H), 2.82-2.97 (m, 3H), 2.52-2.69 (m, 5H), 2.27-2.35 (m, 2H), 1.89-2.11 (m, 5H), 1.62-1.74 (m, 2H), 1.60 (s, 6H).

MS (ESI+) m/z 828.4 [M+H]$^+$.

**Example 22:**

N-(2-(1-(2-(4-(2-(2,6-dioxopiperidin-3-yl)-6-fluoro-1,3-dioxoisoindolin-5-yl)piperazin-1-yl)ethyl)piperidin-4-yl)-7-(2-hydroxypropan-2-yl)imidazo[1,2-a]pyridin-6-yl)-6-(trifluorometh yl)pyridine-2-carboxamide

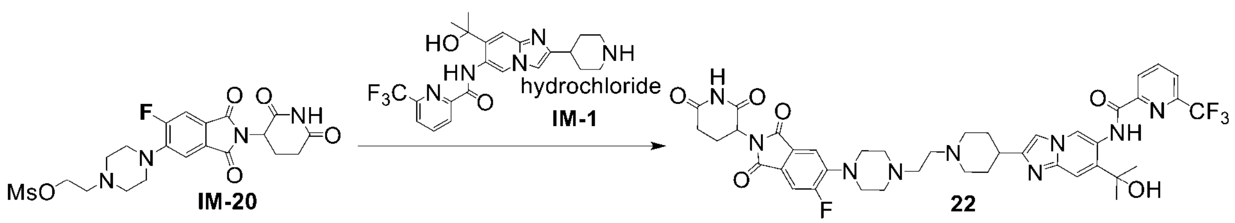


At 25°C, Intermediate **IM-20** (100 mg, 207 μmol) and Intermediate **IM-1** (92.7 mg, 207 μmol) were dissolved in dimethylformamide (2.00 mL) and added with *N,N*-diisopropylethylamine (80.3 mg, 621 μmol) and sodium iodide (93.2 mg, 621 μmol). The reaction mixture was stirred for 12 h at 80°C and concentrated under reduced pressure. The obtained

residue was purified through HPLC (in an ammonium bicarbonate system) to obtain Compound **22** as a yellow solid (5.30 mg).

$^1$H NMR (400 MHz, DMSO-*d*$_6$) $\delta$ 12.22 (s, 1H), 11.11 (br s, 1H), 9.53 (s, 1H), 8.32-8.50 (m, 2H), 8.13-8.28 (m, 1H), 7.69-7.82 (m, 2H), 7.38-7.48 (m, 2H), 6.15 (br s, 1H), 5.03-5.17 (m, 1H), 3.25 (br s, 6H), 2.81-3.04 (m, 4H), 2.55-2.70 (m, 7H), 1.90 (br s, 6H), 1.64-1.74 (m, 2H), 1.60 (s, 6H).

MS (ESI+) m/z 834.3 [M+H]$^+$.

**Example 23:**

*N*-(2-(1-((1-(2-(2,6-dioxopiperidin-3-yl)-6-fluoro-1-oxoisoindolin-5-yl)piperidin-4-yl) methyl)piperidin-4-yl)-7-(2-hydroxypropan-2-yl)imidazo[1,2-a]pyridin-6-yl)-6-(trifluoromethyl) pyridine-2-carboxamide

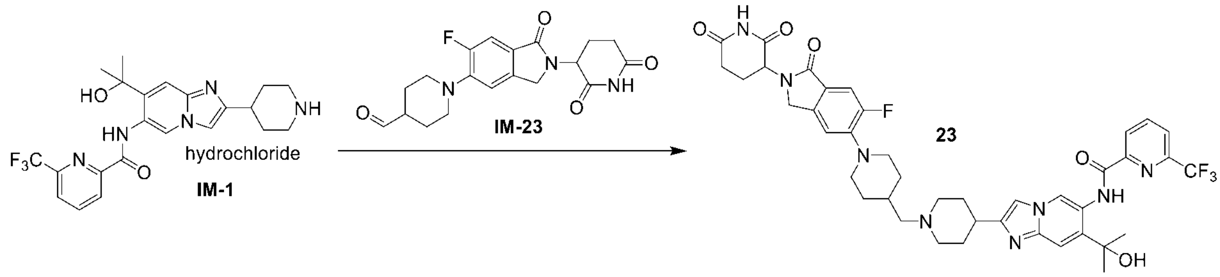


At room temperature, Intermediate **IM-1** (155 mg, 321 μmol) was dissolved in N,N-dimethylformamide (2.00 mL) and tetrahydrofuran (2.00 mL) and added with sodium acetate (79 mg, 964 μmol). The mixture was stirred for 15 min and then added with Intermediate **IM-23** (150 mg, 321 μmol), sodium acetate (102 mg, 482 μmol), and acetic acid (57.9 mg, 964 μmol). The reaction mixture was stirred for 12 h at 25°C and concentrated to dryness under reduced pressure. The obtained residue was purified through HPLC (column: Waters Xbridge BEH C18 250×50 mm × 10 μm; mobile phase: [$H_2O$ ($NH_4HCO_3$)-ACN]; B%: 25%-55%, 10 min) to obtain Compound **23** as a white solid (20.0 mg).

$^1$H NMR (400 MHz, DMSO-*d*$_6$) $\delta$ 12.22 (s, 1H), 10.96 (br s, 1H), 9.54 (s, 1H), 8.43-8.49 (m, 1H), 8.36-8.41 (m, 1H), 8.16-8.23 (m, 1H), 7.78 (s, 1H), 7.37-7.43 (m, 2H), 7.23 (br d, *J* = 7.6 Hz, 1H), 6.14 (s, 1H), 5.02-5.11 (m, 1H), 4.32-4.40 (m, 1H), 4.20-4.28 (m, 1H), 3.48 (br d, *J* = 2.1 Hz, 2H), 2.86-2.96 (m, 3H), 2.73-2.79 (m, 2H), 2.61-2.68 (m, 2H), 2.32-2.40 (m, 1H), 2.22 (br d, *J* = 7.0 Hz, 2H), 1.94-2.07 (m, 5H), 1.83 (br d, *J* =11.6 Hz, 2H), 1.75-1.66 (m, 3H), 1.60 (s, 6H), 1.35-1.24 (m, 2H).

MS (ESI+) m/z 805.4 [M+H]$^+$.

**Example 24:**

*N*-(2-(1-((3-(2-(2,6-dioxopiperidin-3-yl)-1,3-dioxoisoindolin-5-yl)-3-azabicyclo[3.1.0]h exan-6-yl)methyl)piperidin-4-yl)-7-(2-hydroxypropan-2-yl)imidazo[1,2-a]pyridin-6-yl)-6-(trifluo romethyl)pyridine-2-carboxamide

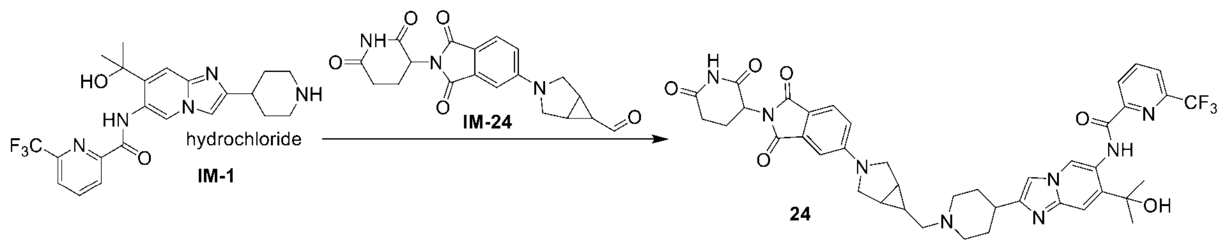


Intermediate **IM-1** (131 mg, 272 μmol) was dissolved in N,N-dimethylformamide (0.50 mL) and tetrahydrofuran (0.50 mL) and added with sodium acetate (33.5 mg, 0.406 mmol). The mixture was stirred for 0.5 h at 25°C. Sodium triacetoxyborohydride (173 mg, 0.816 mmol), Intermediate **IM-24** (100 mg, 272 μmol), and acetic acid (49.0 mg, 0.816 mmol) were added in sequence. The obtained reaction mixture was stirred for 12 h at 25°C. The reaction mixture was concentrated under reduced pressure. The obtained residue was purified through preparative HPLC (column: Waters

Xbridge Prep OBD C18 150×40 mm × 10 μm; mobile phase: [$H_2O$ (10 mM $NH_4HCO_3$)-ACN]; gradient 30%-70% B, 8.0 min) to obtain Compound **24** as a yellow solid (11.0 mg).

$^1H$ NMR (400 MHz, DMSO-*d*$_6$) δ 12.22 (s, 1H), 11.06 (br s, 1H), 9.54 (s, 1H), 8.36-8.48 (m, 2H), 8.19 (d, *J* = 7.7 Hz, 1H), 7.79 (s, 1H), 7.63 (d, *J* = 8.4 Hz, 1H), 7.41 (s, 1H), 6.92 (s, 1H), 6.83 (br d, *J* = 8.7 Hz, 1H), 6.14 (s, 1H), 5.05 (dd, *J* = 5.4, 13.0 Hz, 1H), 3.68 (br d, *J* = 10.3 Hz, 2H), 3.42-3.50 (m, 2H), 3.02 (br d, *J* = 10.9 Hz, 2H), 2.81-2.94 (m, 1H), 2.58-2.68 (m, 2H), 2.54-2.58 (m, 1H), 2.32 (br d, *J* = 6.0 Hz, 2H), 2.10 (br t, *J* = 10.5 Hz, 2H), 1.93-2.04 (m, 3H), 1.62-1.72 (m, 4H), 1.60 (s, 6H), 0.49-1.05 (m, 1H).

MS (ESI+) m/z 799.4 [M+H]$^+$.

**Example 25:**

(*R*)-*N*-(2-(1-((1-(2-(2,6-dioxopiperidin-3-yl)-6-fluoro-1,3-dioxoisoindolin-5-yl)piperidi n-4-yl)methyl)piperidin-4-yl)-7-(2-hydroxypropan-2-yl)imidazo[1,2-a]pyridin-6-yl)-6-(trifluoro methyl)pyridine-2-carboxamide

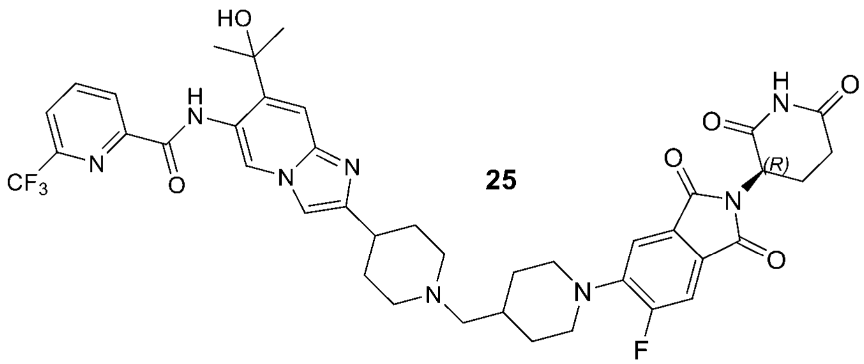

25

**Step 1: Preparation of (*R*)-*t*-butyl 5-amino-4-(5,6-difluoro-1,3-dioxoisoindolin-2-yl)-5-oxopentanoate (IM-25a)**

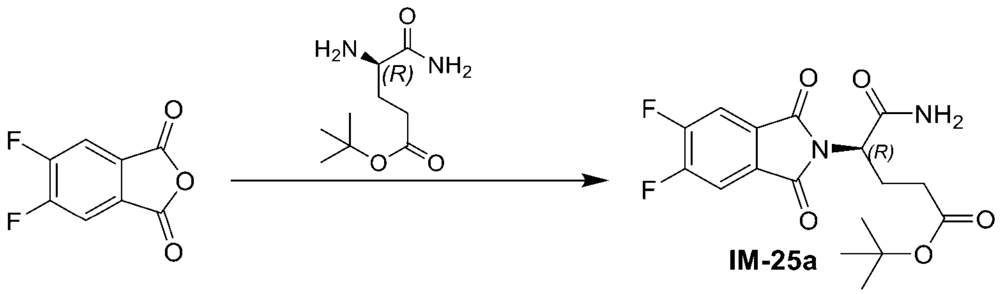

IM-25a

5,6-Difluoroisobenzofuran-1,3-dione (5.00 g, 27.1 mmol) and (*R*)-*t*-butyl 4,5-diamino-5-oxopentanoate (6.59 g, 32.5 mmol) were dissolved in toluene (50.0 mL). Triethylamine (8.24 g, 81.4 mmol) was added to the reaction system at 25°C. After addition, the obtained reaction mixture was heated to 80°C and stirred for 12 h. The reaction mixture was quenched with 50 mL of water at 25°C and extracted with ethyl acetate (50 mL × 3). Combined organic layers were dried over anhydrous sodium sulfate, filtered, and concentrated under reduced pressure. The obtained residue was purified through column chromatography (silica gel:petroleum ether:ethyl acetate = 100:1-0:1 (v:v:v)) to obtain Intermediate **IM-25a** as a white solid (5.00 g).

MS (ESI+) m/z 313.2 [M-55]$^+$.

**Step 2: Preparation of (*R*)-*t*-butyl 5-amino-4-(5-(4-(dimethoxymethyl)piperidin-1-yl)-6-fluoro-1,3-dioxoisoindolin-2-yl)-5-oxo pentanoate (IM-25b)**

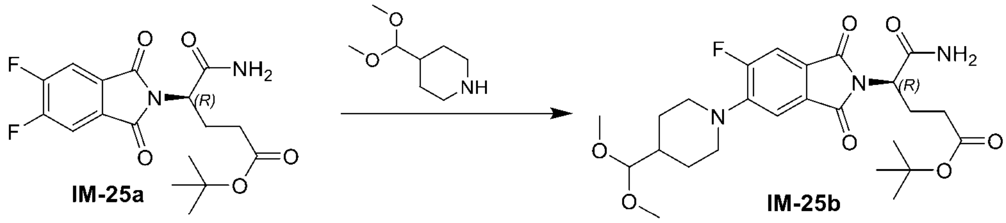

IM-25a IM-25b

Intermediate **IM-25a** (2.00 g, 5.43 mmol) and 4-(dimethoxymethyl)piperidine (1.04 g, 6.52 mmol) were dissolved in dimethylsulfoxide (20.0 mL). *N,N*-diisopropylethylamine (1.40 g, 10.8 mmol) was added at 25°C. The obtained reaction mixture was stirred for 15 h at 90°C. The reaction was quenched with 20 mL of water at 25°C and extracted with ethyl acetate (30 mL × 3). Combined organic layers were dried over anhydrous sodium sulfate, filtered, and concentrated under reduced pressure. The obtained residue was purified through column chromatography (silica gel:petroleum ether:ethyl acetate = 100:1-0:1 (v:v:v)) to obtain Intermediate **IM-25b** as a yellow solid (2.30 g).
MS (ESI+) m/z 508.2 [M+H]+.

**Step 3: Preparation of (*R*)-t-butyl 5-amino-4-(5-fluoro-6-(4-formylpiperidin-1-yl)-1,3-dioxoisoindolin-2-yl)-5-oxopentanoate (IM-25c)**

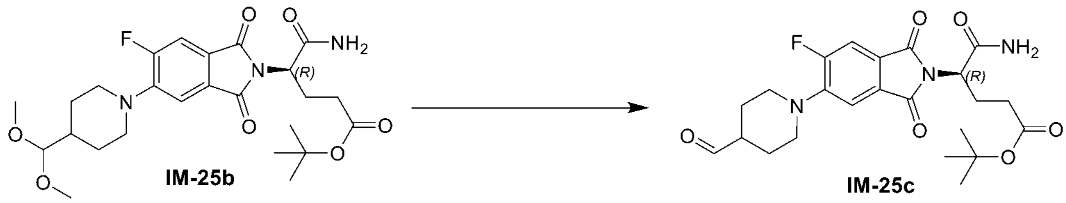

IM-25b IM-25c

Intermediate **IM-25b** (1.00 g, 1.97 mmol) was dissolved in tetrahydrofuran (10.0 mL) and water (2.00 mL). Pyridinium *p*-toluenesulfonate (1.49 g, 5.91 mmol) was added to the reaction system at 25°C under nitrogen protection. The reaction was heated to 70°C and stirred for 12 h at 70°C. The reaction mixture was concentrated under reduced pressure to obtain Intermediate **IM-25c** as a yellow solid (700 mg, crude product), which was used directly without further purification.
MS (ESI+) m/z 406.3 [M-55].

**Step 4: Preparation of (*R*)-*t*-butyl 5-amino-4-(5-fluoro-6-(4-((4-(7-(2-hydroxypropan-2-yl)-6-(6-(trifluoromethyl) pyridine-2-ca rboxamido)imidazo[1,2-a]pyridin-2-yl)piperidin-1-yl)methyl)piperidin-1-yl)-1,3-dioxoisoin dolin-2-yl)-5-oxopentanoate (IM-25d)**

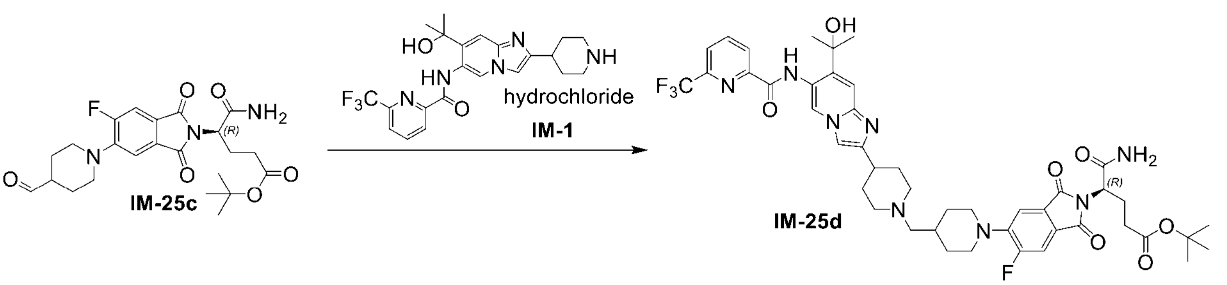


IM-25c IM-25d

Intermediate **IM-1** (678 mg, 1.52 mmol) was dissolved in *N,N*-dimethylformamide (7.00 mL) and tetrahydrofuran (7.00 mL). Anhydrous sodium acetate (248 mg, 3.03 mmol) was added to the reaction system at 25°C. The reaction was stirred for 30 min at 25°C. Intermediate **IM-25c** (700 mg, 1.52 mmol), sodium triacetoxyborohydride (964 mg, 4.55 mmol), and glacial acetic acid (273 mg, 4.55 mmol) were added to the reaction system. The reaction mixture was stirred for 3.5 h at 25°C, filtered, and concentrated under reduced pressure to obtain Intermediate **IM-25d** as a yellow solid (1.20 g, crude product).

MS (ESI+) m/z 893.6 $[M+H]^+$.

**Step 5: Preparation of (*R*)-*N*-(2-(1-((1-(2-(2,6-dioxopiperidin-3-yl)-6-fluoro-1,3-dioxoisoindolin-5-yl)piperidin-4-yl) methyl)piperidin-4-yl)-7-(2-hydroxypropan-2-yl)imidazo[1,2-a]pyridin-6-yl)-6-(trifluorome thyl)pyridine-2-carboxamide (25)**

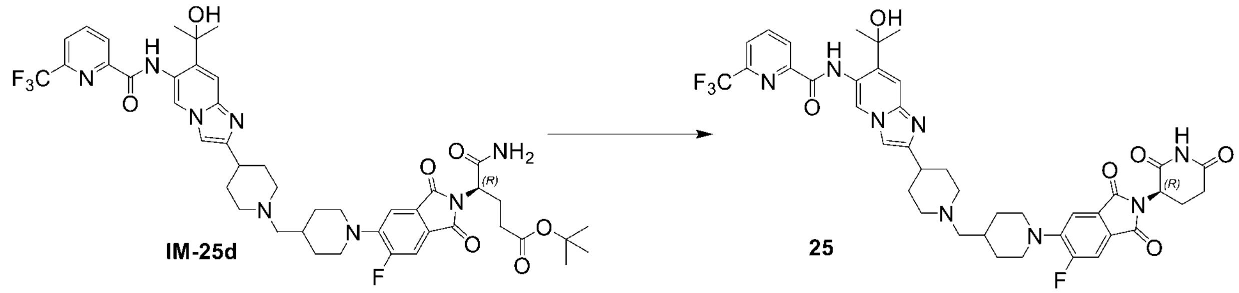

IM-25d 25

Intermediate **IM-25d** (100 mg, 111.99 μmol) was dissolved in acetonitrile (1.00 mL). Anhydrous benzenesulfonic acid (35.4 mg, 223 μmol) was added to the reaction system at 25°C. The reaction was heated to 80°C and stirred for 12 h at 80°C. The mixture was filtered and concentrated under reduced pressure to obtain a residue. The residue was purified through preparative HPLC (column: Phenomenex Luna C18 100×40 mm × 5 μm; mobile phase: [$H_2O$ (0.2% FA)-ACN]; gradient: 1%-40% B, 8.0 min) to obtain Compound **25** as a yellow solid (12.0 mg).

$^1H$ NMR (400 MHz, DMSO-$d_6$) δ 12.22 (s, 1H), 11.10 (br s, 1H), 9.54 (s, 1H), 8.34-8.49 (m, 2H), 8.18 (s, 1H), 7.79 (s, 1H), 7.70 (d, *J* = 11.4 Hz, 1H), 7.37-7.47 (m, 2H), 6.15 (br s, 1H), 5.10 (dd, *J* = 5.4, 12.8 Hz, 1H), 3.62 (br d, *J* = 12.0 Hz, 2H), 2.83-2.96 (m, 5H), 2.54-2.72 (m, 3H), 2.23 (br d, *J* = 6.6 Hz, 2H), 1.93-2.11 (m, 5H), 1.65-1.90 (m, 5H), 1.60 (s, 6H), 1.20-1.35 (m, 2H).

MS (ESI+) m/z 819.4 $[M+H]^+$.

**Example 26:**

(*S*)-*N*-(2-(1-((1-(2-(2,6-dioxopiperidin-3-yl)-6-fluoro-1,3-dioxoisoindolin-5-yl)piperidi n-4-yl)methyl)piperidin-4-yl)-7-(2-hydroxypropan-2-yl)imidazo[1,2-a]pyridin-6-yl)-6-(trifluoro methyl)pyridine-2-carboxamide

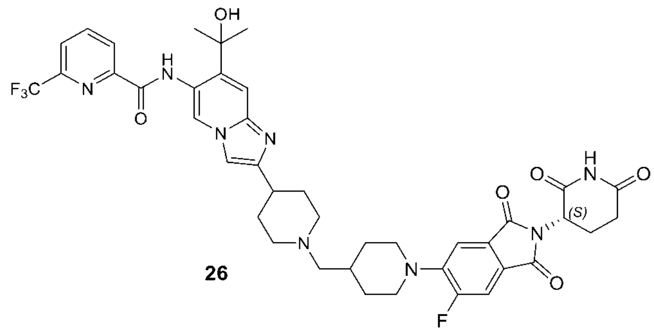

26

**Step 1: Preparation of (*S*)-*t*-butyl 5-amino-4-(5,6-difluoro-1,3-dioxoisoindolin-2-yl)-5-oxopentanoate (IM-26a)**

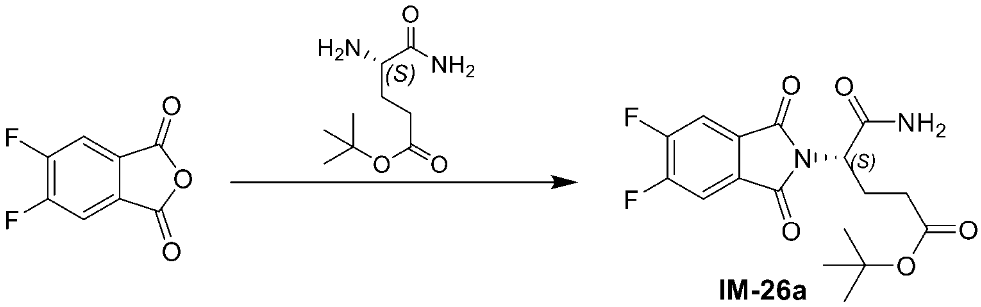

IM-26a

5,6-Difluoroisobenzofuran-1,3-dione (10.0 g, 54.3 mmol) and (*S*)-*t*-butyl 4,5-diamino-5-oxopentanoate (15.5 g, 65.1 mmol) were dissolved in toluene (50.0 mL). Triethylamine (16.4 g, 162 mmol) was added to the reaction system at 25°C. After addition, the obtained mixture was heated to 80°C and stirred for 12 h. The reaction mixture was quenched with water (30 mL) at 25°C and extracted with ethyl acetate (40 mL × 3). Combined organic layers were dried over anhydrous sodium sulfate, filtered, and concentrated under reduced pressure. The obtained residue was purified through column chromatography (silica gel:petroleum ether:ethyl acetate = 100:1-0:1 (v:v:v)) to obtain Intermediate **IM-26a** as a white solid (1.50 g).

MS (ESI+) m/z 313.2 [M-55].

**Step 2: Preparation of (*S*)-*t*-butyl 5-amino-4-(5-(4-(dimethoxymethyl)piperidin-1-yl)-6-fluoro-1,3-dioxoisoindolin-2-yl)-5-oxo pentanoate (IM-26b)**

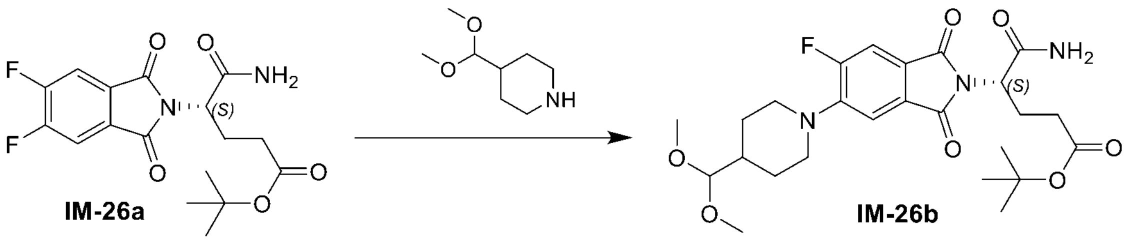

IM-26a IM-26b

Intermediate **IM-26a** (1.50 g, 4.07 mmol) and 4-(dimethoxymethyl)piperidine (778 mg, 4.89 mmol) were dissolved in dimethylsulfoxide (15.0 mL). N,N-diisopropylethylamine (1.05 g, 8.14 mmol) was added at 25°C. The reaction mixture was stirred for 15 h at 90°C. The reaction was quenched with 20 mL of water at 25°C and extracted with ethyl acetate (30 mL × 3). Combined organic layers were dried over anhydrous sodium sulfate, filtered, and concentrated under reduced pressure. The obtained residue was purified through column chromatography (silica gel:petroleum ether:ethyl acetate = 100:1-0:1 (v:v:v)) to obtain Intermediate **IM-26b** as a yellow solid (1.50 g, 2.96 mmol).

MS (ESI+) m/z 508.2 $[M+H]^+$.

**Step 3: Preparation of (*S*)-*t*-butyl 5-amino-4-(5-fluoro-6-(4-formylpiperidin-1-yl)-1,3-dioxoisoindolin-2-yl)-5-oxopentanoate (IM-26c)**

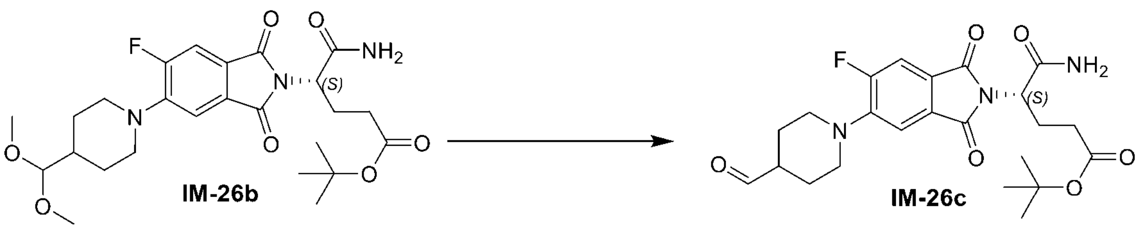

IM-26b IM-26c

Intermediate **IM-26b** (700 mg, 1.38 mmol) was dissolved in tetrahydrofuran (5.00 mL) and water (1.00 mL). Pyridinium *p*-toluenesulfonate (1.04 g, 4.14 mmol) was added at 25°C under nitrogen protection. The reaction was heated to 70°C and stirred for 12 h at 70°C. The mixture was concentrated under reduced pressure to obtain Intermediate **IM-26c**

as a yellow solid (600 mg, crude product), which was used directly without further purification.

MS (ESI+) m/z 462.2 $[M+H]^+$.

**Step 4: Preparation of (*S*)-*t*-butyl 5-amino-4-(5-fluoro-6-(4-((4-(7-(2-hydroxypropan-2-yl)-6-(6-(trifluoromethyl) pyridine-2-ca rboxamido)imidazo[1,2-a]pyridin-2-yl)piperidin-1-yl)methyl)piperidin-1-yl)-1,3-dioxoisoin dolin-2-yl)-5-oxopentanoate (IM-26d)**

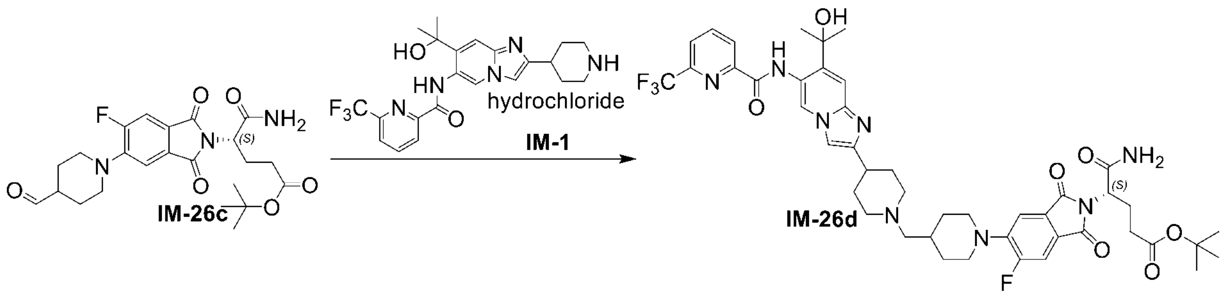


Intermediate **IM-1** (484 mg, 1.08 mmol) was dissolved in N,N-dimethylformamide (5.00 mL) and tetrahydrofuran (5.00 mL). Anhydrous sodium acetate (266 mg, 3.25 mmol) was added at 25°C. The reaction was stirred for 30 min at 25°C. Intermediate **IM-26c** (500 mg, 1.08 mmol), sodium triacetoxyborohydride (688 mg, 3.25 mmol), and glacial acetic acid (65.0 mg, 1.08 mmol) were added to the reaction mixture. The reaction mixture was stirred for 3.5 h at 25°C, filtered, and concentrated under reduced pressure to obtain Intermediate **IM-26d** as a yellow solid (500 mg, crude product).

MS (ESI+) 893.3 $[M+H]^+$.

**Step 5: Preparation of (*S*)-*N*-(2-(1-((1-(2-(2,6-dioxopiperidin-3-yl)-6-fluoro-1,3-dioxoisoindolin-5-yl)piperidin-4-yl) methyl)piperidin-4-yl)-7-(2-hydroxypropan-2-yl)imidazo[1,2-a]pyridin-6-yl)-6-(trifluorome thyl)pyridine-2-carboxamide (26)**

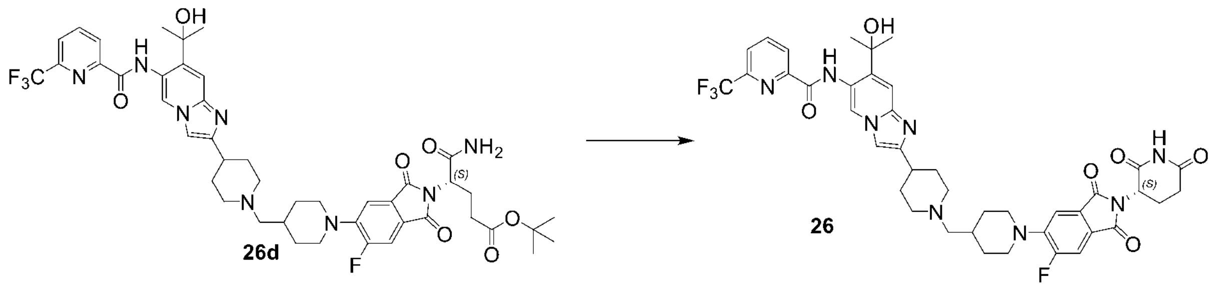


Compound **IM-26d** (50.0 mg, 56.0 μmol) was dissolved in acetonitrile (1.00 mL). Anhydrous benzenesulfonic acid (17.7 mg, 111 μmol) was added to the reaction system at 25°C. The reaction was heated to 80°C and stirred for 12 h at 80°C. The mixture was filtered and concentrated under reduced pressure. The obtained residue was purified through HPLC (column: Phenomenex Luna C18 100×40 mm × 5 μm; mobile phase: [$H_2O$ (0.2% FA)-ACN]; gradient: 10%-50% B, 8.0 min) to obtain Compound **26** as a yellow solid (11.0 mg).

$^1H$ NMR (400 MHz, DMSO-$d_6$) $\delta$ 12.22 (s, 1H), 11.10 (br s, 1H), 9.54 (s, 1H), 8.31-8.51 (m, 2H), 8.09-8.25 (m, 1H), 7.63-7.86 (m, 2H), 7.35-7.51 (m, 2H), 6.15 (s, 1H), 5.10 (br dd, *J* = 4.7, 12.3 Hz, 1H), 2.81-3.06 (m, 6H), 2.67 (br s, 2H), 2.31 (br d, *J* = 17.3 Hz, 3H), 2.00 (br d, *J* = 9.4 Hz, 4H), 1.69-1.89 (m, 5H), 1.60 (br s, 6H), 1.19-1.35 (m, 4H).

MS (ESI+) m/z 819.3 $[M+H]^+$.

**Comparative Example 1:**

***N*-(2-(1-(2-(2-((2-(2,6-dioxopiperidin-3-yl)-1,3-dioxoisoindolin-4-yl)amino)ethoxy)e thyl)piperidin-4-yl)-7-(2-hydroxypropan-2-yl)imidazo[1,2-a]pyridin-6-yl)-6-(trifluoromethy l)pyridine-2-carboxamide**

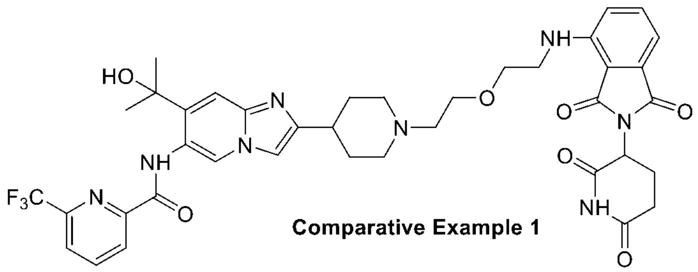

**Comparative Example 1**

**Step 1: Preparation of 2-(2,6-dioxopiperidin-3-yl)-4-((2-(2-hydroxyethoxy)ethyl)amino)isoindoline-1,3-dione (IM-25a)**

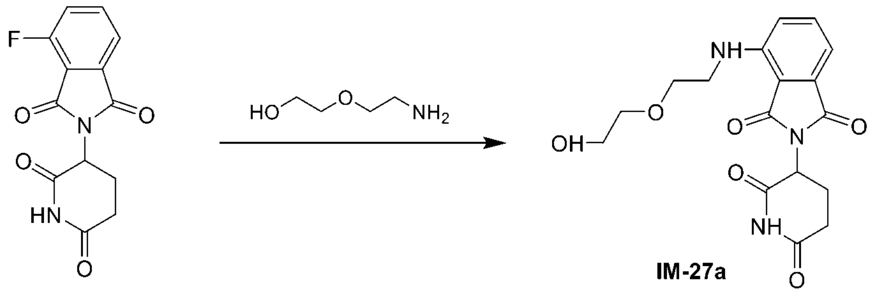

**IM-27a**

2-(2,6-Dioxopiperidin-3-yl)-4-fluoroisoindoline-1,3-dione (5.00 g, 26.8 mmol) and 2-(2-aminoethoxy)ethanol (2.82 g, 26.8 mmol) were dissolved in dimethylsulfoxide (10.0 mL) and added with N,N-diisopropylethylamine (3.47 g, 26.8 mmol). The obtained reaction mixture was heated to 70°C and stirred for 12 h. The reaction mixture was concentrated under reduced pressure. The obtained residue was purified through silica gel column chromatography (silica gel:petroleum ether:ethyl acetate = 100:1-10:1 (v:v:v)) to obtain Intermediate **IM-27a** as a yellow oil (7.70 g).

$^1$H NMR (400 MHz, DMSO-$d_6$) $\delta$ 11.05 (s, 1H), 7.55 (dd, $J$ = 7.3, 8.3 Hz, 1H), 7.11 (d, $J$ = 8.6 Hz, 1H), 7.00 (d, $J$ = 7.0 Hz, 1H), 6.57 (t, $J$ = 5.7 Hz, 1H), 5.02 (dd, $J$ = 5.4, 12.9 Hz, 1H), 4.56 (t, $J$ = 5.2 Hz, 1H), 3.53-3.65 (m, 2H), 3.39-3.51 (m, 6H), 2.77-3.02 (m, 1H), 2.67 (s, 1H), 2.60-2.55 (m, 2H).

**Step 2: Preparation of 2-(2-((2-(2,6-dioxopiperidin-3-yl)-1,3-dioxoisoindolin-4-yl)amino)ethoxy)ethyl methanesulfonate (IM-27b)**

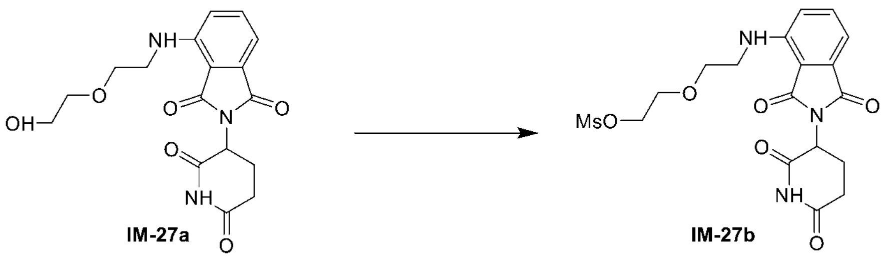

**IM-27a** **IM-27b**

Intermediate **IM-27a** (1.00 g, 2.77 mmol) was dissolved in a dichloromethane solution (10 mL). Triethylamine (1.12 g, 11.0 mmol) and methanesulfonic anhydride (2.23 g, 19.4 mmol) were added at 0°C. The obtained reaction mixture was stirred for 3 h at 25°C. The reaction mixture was quenched with water (20 mL) at 0°C and extracted with dichloromethane (30.0 mL × 3). Combined organic layers were washed with an aqueous solution of sodium chloride

(10.0 mL × 3), dried over anhydrous sodium sulfate, filtered, and concentrated under reduced pressure. The obtained residue was purified through silica gel column chromatography (silica gel:petroleum ether:ethyl acetate = 100:1-10:1 (v:v:v)) to obtain Intermediate **IM-27b** as a yellow solid (500 mg).

$^1H$ NMR (400 MHz, DMSO-$d_6$) δ 11.10 (s, 1H), 7.59 (dd, *J* = 7.2, 8.4 Hz, 1H), 7.16 (d, *J* = 8.6 Hz, 1H), 7.05 (d, *J* = 7.0 Hz, 1H), 6.63 (br t, *J* = 5.9 Hz, 1H), 5.05 (dd, *J* = 5.4, 12.8 Hz, 1H), 4.31 (dd, *J* = 3.5, 5.3 Hz, 2H), 3.61-3.77 (m, 4H), 3.45-3.55 (m, 3H), 3.16 (s, 3H), 2.82-2.94 (m, 1H), 2.52-2.63 (m, 2H).

**Step** 3: **Preparation of *N*-(2-(1-(2-(2-((2-(2,6-dioxopiperidin-3-yl)-1,3-dioxoisoindolin-4-yl)amino)ethoxy)ethyl) pipe ridin-4-yl)-7-(2-hydroxypropan-2-yl)imidazo[1,2-a]pyridin-6-yl)-6-(trifluoromethyl)pyridin e-2-carboxamide (Comparative Example 1)**

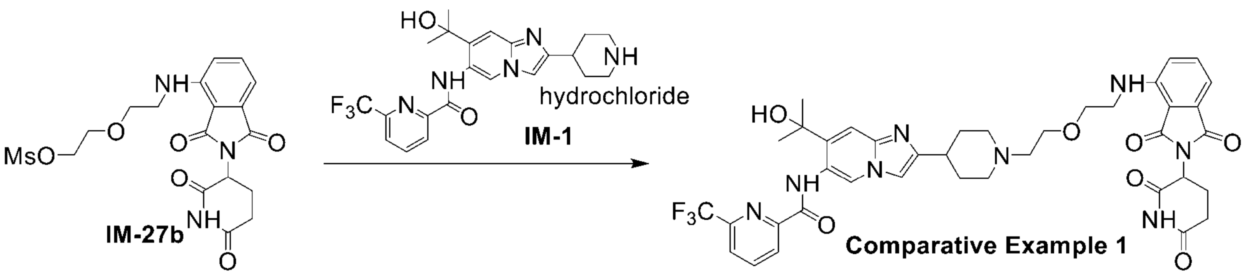


Intermediate **IM-27b** (162 mg, 368 μmol) and **IM-1** (150 mg, 335 μmol) were dissolved in *N,N*-dimethylformamide (2.00 mL) and added with *N,N*-diisopropylethylamine (129 mg, 1.01 mmol) and sodium iodide (100 mg, 670 μmol) in sequence. The obtained mixture was heated to 80°C and stirred for 4 h. The reaction mixture was concentrated under reduced pressure. The obtained residue was purified through preparative HPLC (column: Phenomenex Luna C18 80×40 mm × 3 μm; mobile phase: [$H_2O$ (FA)-ACN]; B%: 5%-45%, 8 min) to obtain Comparative Example 1 as a yellow solid (27.0 mg).

$^1H$ NMR (400 MHz, DMSO-$d_6$) δ 12.23 (s, 1H), 11.21 (br s, 1H), 9.54 (s, 1H), 8.36-8.47 (m, 2H), 8.18-8.20 (m, 1H), 7.74 (s, 1H), 7.56-7.62 (m, 1H), 7.42 (s, 1H), 7.17 (d, *J* = 8.7 Hz, 1H), 7.05 (d, *J* = 7.1 Hz, 1H), 6.62 (br t, *J* = 5.6 Hz, 1H), 6.15(s, 1H), 5.05 (dd, *J* = 5.4, 12.4 Hz, 1H), 3.56-3.64 (m, 4H), 3.44-3.52 (m, 3H), 2.74-3.00 (m, 4H), 2.52-2.68 (m, 5H), 2.12 (br t, *J* = 11.3 Hz, 2H), 1.85-2.03 (m, 3H), 1.60 (s, 6H).

MS (ESI+) m/z 791.4 $[M+H]^+$.

**Comparative Example 2:**

**N-(2-(1-(6-(2-(2,6-dioxopiperidin-3-yl)-1-oxoisoindolin-4-yl)hex-5-yn-1-yl)piperidi n-4-yl)-7-(2-hydroxypropan-2-yl)imidazo[1,2-a]pyridin-6-yl)-6-(trifluoromethyl)pyridine-2-carboxamide**

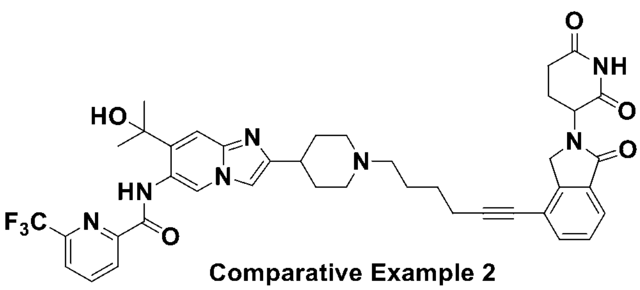

**Comparative Example 2**

**Step 1: Preparation of 3-(4-(6-hydroxyhex-1-yn-1-yl)-1-oxoisoindolin-2-yl)piperidine-2,6-dione (IM-28a)**

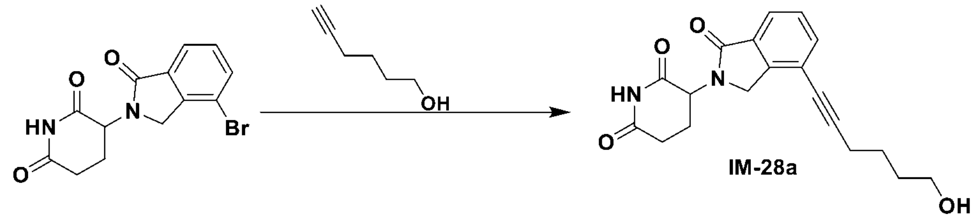

IM-28a

3-(4-Bromo-1-oxoisoindolin-2-yl)piperidine-2,6-dione (1.00 g, 3.09 mmol) and 5-hexyn-1-ol (607 mg, 6.19 mmol) were dissolved in dimethylformamide (20.0 mL). Cuprous iodide (353 mg, 1.86 mmol), bis(triphenylphosphine) palladium(II) chloride (217 mg, 309 μmol), and triethylamine (7.27 g, 71.8 mmol) were added in sequence at 25°C. The reaction mixture was heated to 60°C and stirred for 12 h. The reaction mixture was diluted with water (20 mL) and extracted with ethyl acetate (20 mL × 3). Combined organic phases were washed with saturated brine, dried over anhydrous sodium sulfate, filtered, and concentrated under reduced pressure. The obtained residue was subjected to column chromatography (silica gel:petroleum ether:ethyl acetate = 100:1-10:1 (v:v:v)) to obtain Intermediate **IM-28a** as a yellow solid (200 mg).

$^1H$ NMR (400 MHz, DMSO-$d_6$) δ 11.00 (s, 1H), 7.70 (d, *J* = 7.3 Hz, 1H), 7.63 (br d, *J* = 7.5 Hz, 1H), 7.53 (br d, *J* = 7.6 Hz, 1H), 5.09-5.19 (m, 1H), 4.41-4.48 (m, 2H), 4.27-4.34 (m, 1H), 3.41-3.48 (m, 4H), 2.88-2.98 (m, 1H), 2.59 (br d, *J* = 17.7 Hz, 2H), 1.97-2.07 (m, 1H), 1.53-1.63 (m, 4H).

**Step 2: Preparation of 6-(2-(2,6-dioxopiperidin-3-yl)-1-oxoisoindolin-4-yl)hex-5-yn-1-yl methanesulfonate (IM-28b)**

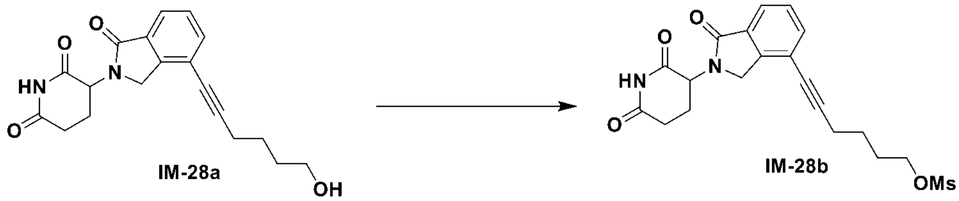

IM-28a IM-28b

At room temperature, Intermediate **IM-28a** (200 mg, 587 μmol) was dissolved in tetrahydrofuran (1.00 mL) and added with triethylamine (148 mg, 1.47 mmol) and methanesulfonic anhydride (178 mg, 881 μmol). The obtained reaction mixture was stirred for 12 h at room temperature. The reaction mixture was diluted with water (10 mL) and extracted with ethyl acetate (10 mL × 3). Combined organic phases were washed with saturated brine, dried over anhydrous sodium sulfate, filtered, and concentrated under reduced pressure. The obtained residue was purified through column chromatography (silica gel:petroleum ether:ethyl acetate = 100:1-10:1 (v:v:v)) to obtain Intermediate **IM-28b** as a white solid (200 mg).

**Step 3: Preparation of *N*-(2-(1-(6-(2-(2,6-dioxopiperidin-3-yl)-1-oxoisoindolin-4-yl)hex-5-yn-1-yl)piperidin-4-yl)-7-(2-hydroxypropan-2-yl)imidazo[1,2-a]pyridin-6-yl)-6-(trifluoromethyl)pyridine-2-carboxa mide (Comparative Example 2)**

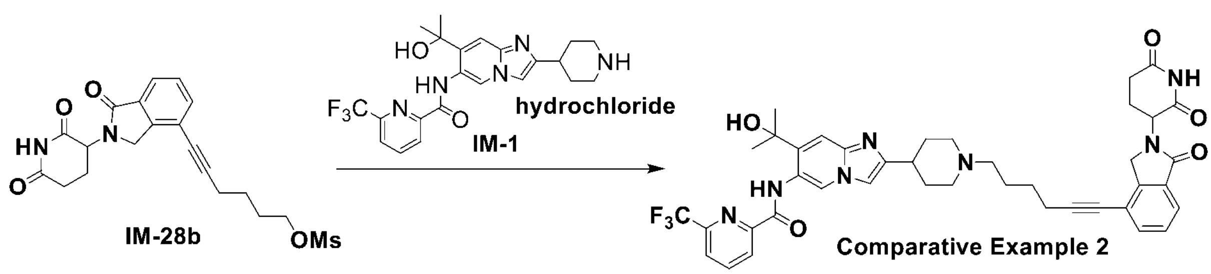


IM-28b Comparative Example 2

Intermediate **IM-28b** (110 mg, 210 μmol) was dissolved in acetonitrile (2.00 mL). Sodium bicarbonate (35.3 mg, 420 μmol) and Intermediate **IM-1** (94.1 mg, 210 μmol) were added at room temperature. The reaction mixture was heated

to 80°C and stirred for 12 h. The reaction mixture was concentrated under reduced pressure. The obtained residue was purified through preparative HPLC (column: Phenomenex Luna C18 80×40 mm × 3 μm; mobile phase: [$H_2O$ (FA)-ACN]; B%: 10%-50%, 8 min) to obtain Comparative Example **2** as a white solid (16.1 mg).

$^1H$ NMR (400 MHz, DMSO-$d_6$) δ 12.22 (s, 1H), 11.04 (br s, 1H), 9.54 (s, 1H), 8.43-8.47 (m, 1H), 8.35-8.41 (m, 1H), 8.17-8.23 (m, 2H), 7.78 (s, 1H), 7.71 (d, *J* = 7.5 Hz, 1H), 7.64 (d, *J* = 7.5 Hz, 1H), 7.50-7.55 (m, 1H), 7.41 (s, 1H), 5.11-5.18 (m, 1H), 4.44-4.50 (m, 1H), 4.29-4.35 (m, 1H), 2.84-3.12 (m, 4H), 2.60-2.79 (m, 2H), 2.53-2.60 (m, 2H), 2.38-2.48 (m, 3H), 2.11-2.33 (m, 3H), 1.96-2.05 (m, 3H), 1.65-1.78 (m, 4H), 1.60 (s, 6H).

MS (ESI+) m/z 770.3 $[M+H]^+$.

IRAK4 protein degradation experiments were conducted on the compounds prepared in the examples and comparative examples. Experimental processes are described below.

HiBiT-based intracellular degradation assay of THP1-HIBiT-IRAK4 cells

THP1-HIBiT-IRAK4 cells were cultured in RPMI 1640 medium supplemented with 10% fetal bovine serum (FBS) and 1% penicillin-streptomycin (PS) (37°C and 5% $CO_2$). The THP1-HIBiT-IRAK4 cells were inoculated in a 384-well plate at 5000 cells/40 μL/well. Compound solutions with different concentrations were added at 40 nL/well and incubated for 24 h at 37°C in 5% $CO_2$. HiBiT lysis detection reagents were added to the wells, the plate was incubated for 30 min at room temperature, and luminescence values were read on Envision.

Degradation rate (%) = (luminescence value of a compound well/mean luminescence value of DMSO wells) × 100

The $DC_{50}$ (half-maximal degradation concentration) of each compound was obtained using XLfit software and the following nonlinear fitting equation:

$$Y = \text{bottom} + (\text{top} - \text{bottom})/(1 + 10\text{^}((\text{Log}DC_{50} - X) \times \text{Hill slope})).$$

X denotes a log value of the compound concentration.

Y denotes the degradation rate.

Table 1 lists the results of $DC_{50}$ values of IRAK4 protein degradation. The experimental results show that the compounds in the examples exhibit significant IRAK4 protein degradation activity, while the compounds in the comparative examples exhibit no IRAK4 protein degradation activity ($DC_{50}$ > 10,000 nM).

Table 1

| Compound | Structure | $DC_{50}$ (nM) |
|---|---|---|
| Example 1 | 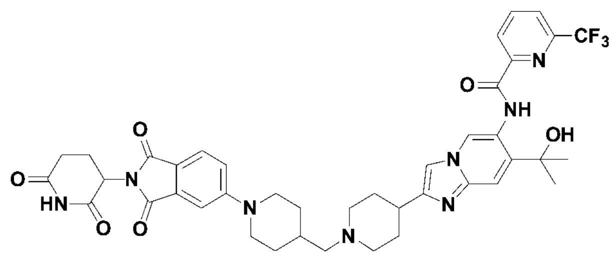 | 4.53 |
| Example 2 | 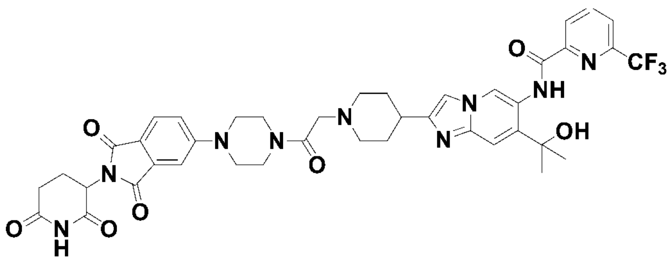 | 4.46 |
| Example 3 | 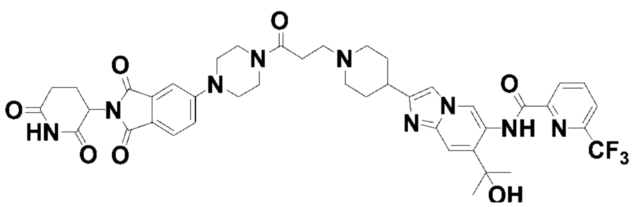 | 13.91 |

(continued)

| Compound | Structure | $DC_{50}$ (nM) |
|---|---|---|
| Example 4 | 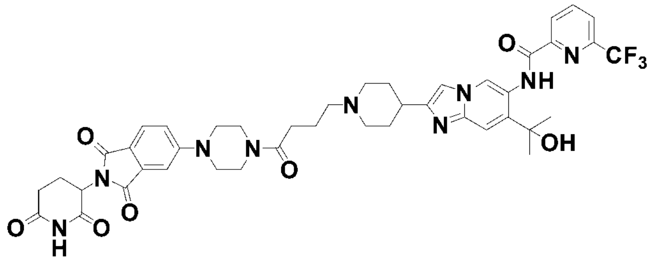 | 5.05 |
| Example 5 | 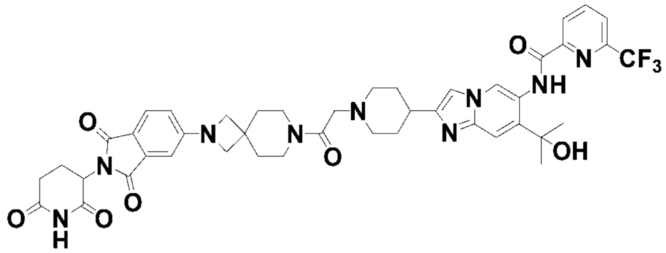 | 3.69 |
| Example 6 | 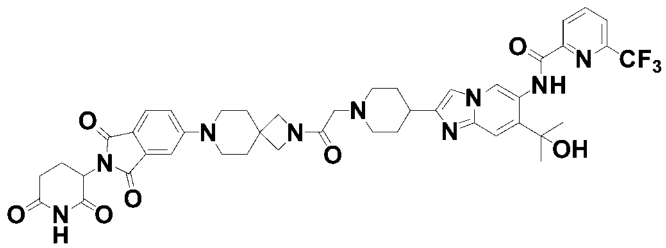 | 7.83 |
| Example 7 | 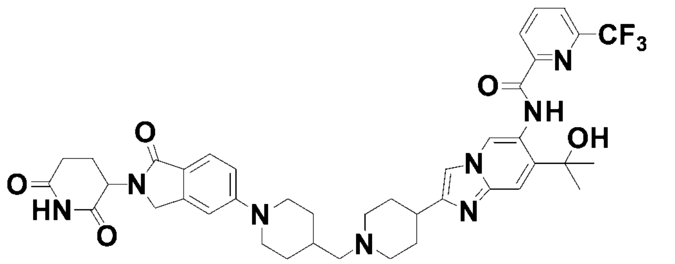 | 1.36 |
| Example 8 | 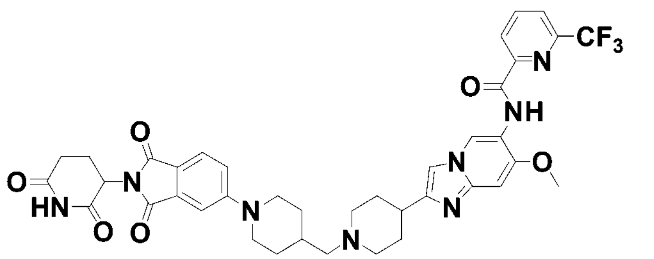 | 16.33 |
| Example 9 | 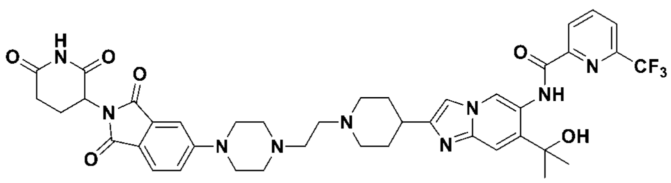 | 0.87 |

(continued)

| Compound | Structure | $DC_{50}$ (nM) |
|---|---|---|
| Example 10 | 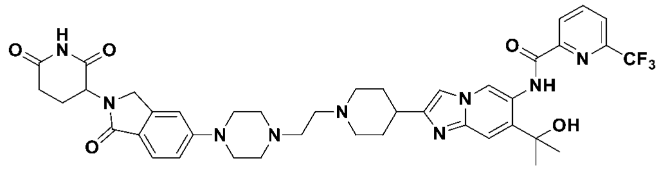 | 0.51 |
| Example 11 | 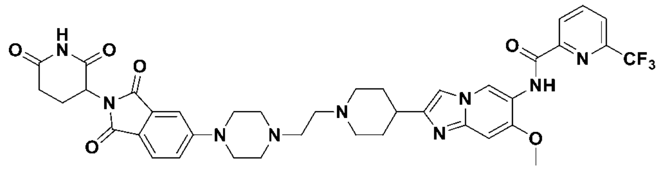 | 2.23 |
| Example 12 | 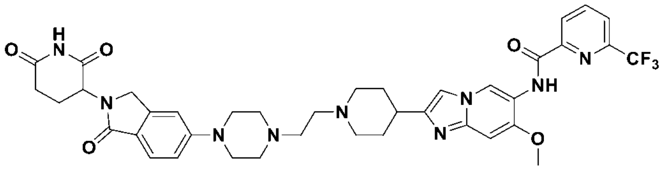 | 1.05 |
| Example 13 | 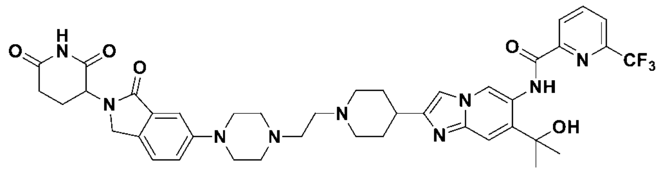 | 0.64 |
| Example 14 | 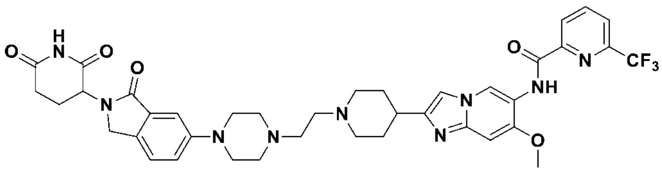 | 1.33 |
| Example 15 | 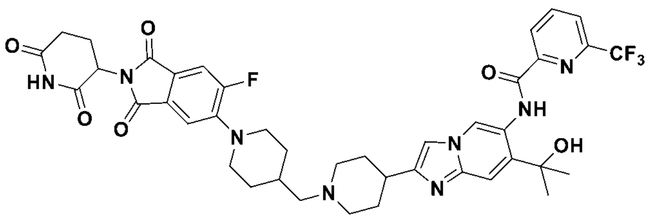 | 1.62 |
| Example 16 | 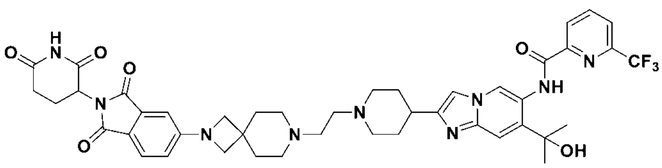 | 3.33 |
| Example 17 | 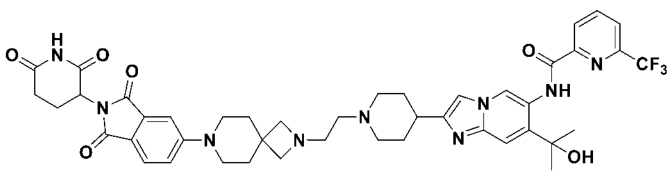 | 4.86 |

(continued)

| Compound | Structure | DC50 (nM) |
|---|---|---|
| Example 18 | 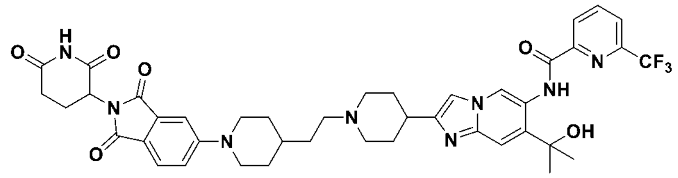 | 4.29 |
| Example 19 | 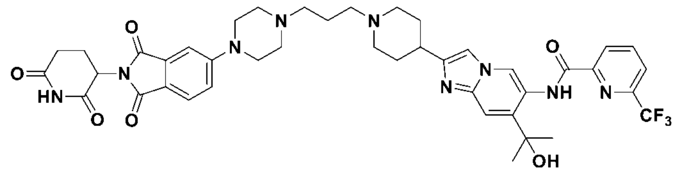 | 1.95 |
| Example 20 | 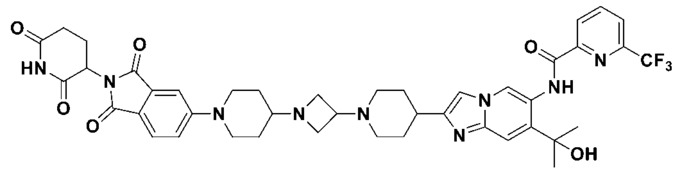 | 1.35 |
| Example 21 | 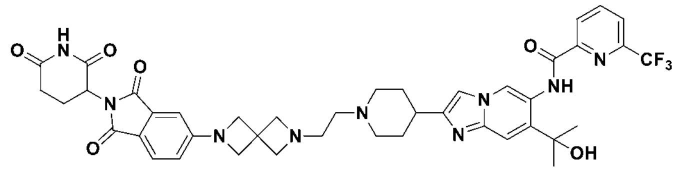 | 2.30 |
| Example 22 | 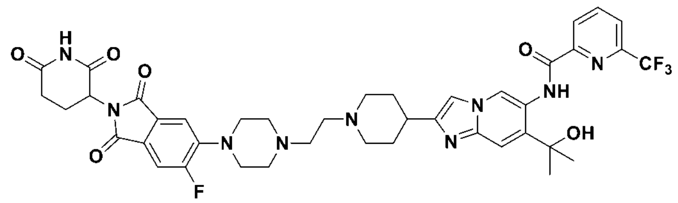 | 4.76 |
| Example 23 | 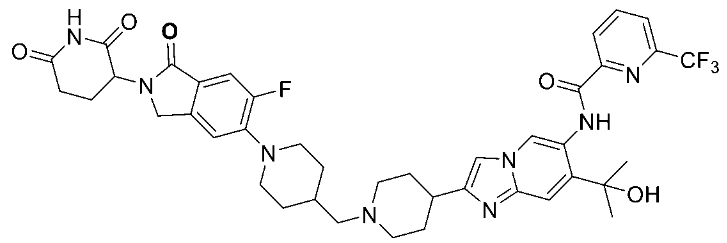 | 0.95 |
| Example 24 | 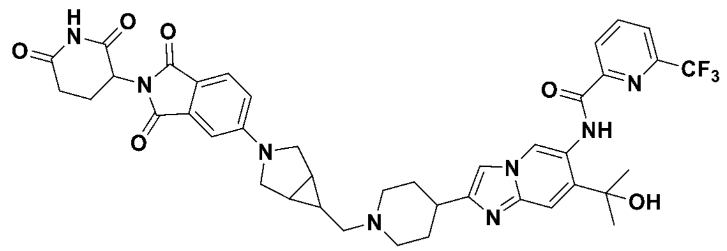 | 1.87 |

(continued)

| Compound | Structure | $DC_{50}$ (nM) |
|---|---|---|
| Example 25 | 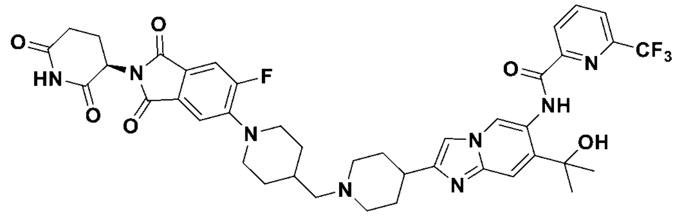 | 12.84 |
| Example 26 | 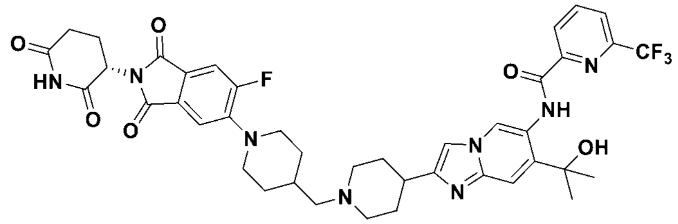 | 2.15 |
| Comparative Example 1 | 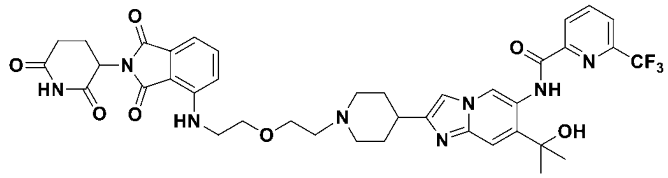 | >10,000 |
| Comparative Example 2 | 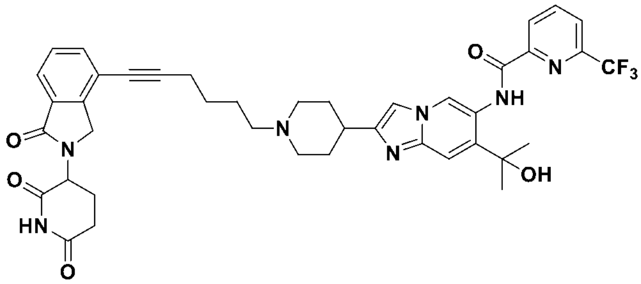 | >10,000 |

**Determination of IRAK4 degradation through Western blotting**

**(1) Compound treatment and lysate preparation**

(a) On the first day, OCI-LY10 cells were inoculated into a 12-well plate at 1.5×10$^6$ cells/mL/well. The test compound was diluted with DMSO, and 1 μL of the diluted compound solution was added to the cell supernatant and incubated overnight in a 37°C and 5% $CO_2$ incubator.
(b) On the second day, the cells were harvested, transferred into a 1.5 mL centrifuge tube, and centrifuged for 5 min at 3000 rpm.
(c) The cells were washed once with ice-cold PBS and centrifuged for 5 min at 3000 rpm and 4°C.
(d) One tablet of protease inhibitor and one tablet of phosphatase inhibitor were added to 20 mL of cell lysis buffer and gently mixed to complete dissolution. The prepared cell lysis buffer was added to a centrifuge tube at 50 μL/tube, and the cells were lysed on ice for 30 min.
(e) The lysate was centrifuged for 15 min at 13000 rpm and 4°C.
(f) The supernatant was transferred to a new tube, and the protein concentration was determined with a BCA kit.

**(2) Western blotting experiment**

(a) The sample was loaded onto 4-12% BTMidi protein gel (1 mm, 26 wells) at 20 μg/well and subjected to electrophoresis for 5 min at a constant voltage of 120 V and electrophoresis for 55 min at a constant voltage of 150

V.

(b) The sample was transferred to a PVDF membrane using a Bio-Rad wet transfer device at 300 mA for 90 min.

(c) The membrane was blocked for 1 h with 1× TBS-T containing 5% BSA at room temperature.

(d) The membrane was incubated with the corresponding primary antibody solutions (rabbit anti-IRAK4 and mouse anti-GAPDH) overnight at 4°C.

(e) The membrane was washed for 3 × 5 min with 1× TBST. The membrane was incubated with secondary antibody solutions (HRP-labeled anti-mouse antibody and HRP-labeled anti-rabbit antibody) for 90 min at room temperature.

(f) The membrane was washed for 3 × 5 min with 1× TBST.

(g) An enhanced chemiluminescence (ECL) substrate was added and scanned using the iBright CL1500 imaging system.

The experimental results are shown in FIGS. 1 to 26. Table 2 correspondingly lists the correspondences between FIGS. 1 to 26 and the compounds. In the figures, "rabbit anti-IRAK4" indicates detection of IRAK4 proteins with a rabbit anti-IRAK4 antibody as a primary antibody, and "mouse anti-GAPDH" indicates detection of GAPDH with a mouse anti-GAPDH antibody as a primary antibody. The results show that the compounds in all the examples exhibit significant IRAK4 protein degradation activity at concentrations of 100 nM and 1000 nM.

Table 2

| Compound | Structure | Figure Corresponding to Western Blotting Result |
|---|---|---|
| Example 1 | 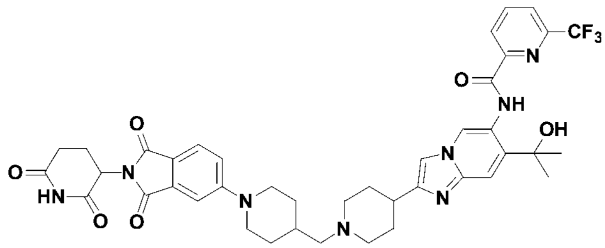 | FIG. 1 |
| Example 2 | 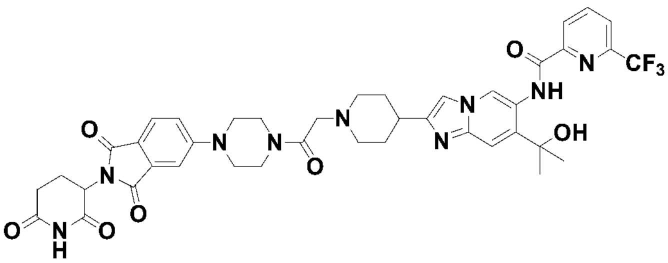 | FIG. 2 |
| Example 3 | 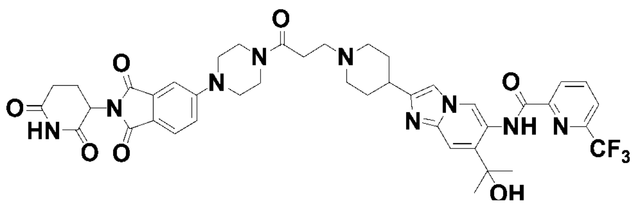 | FIG. 3 |
| Example 4 | 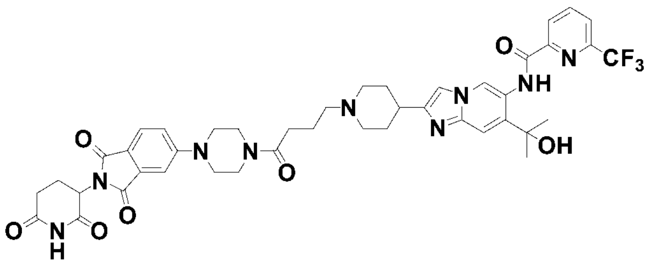 | FIG. 4 |

(continued)

| Compound | Structure | Figure Corresponding to Western Blotting Result |
|---|---|---|
| Example 5 | 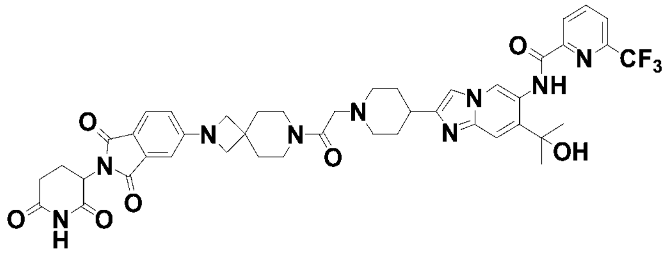 | FIG. 5 |
| Example 6 | 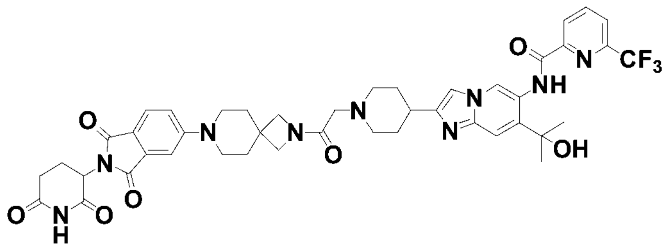 | FIG. 6 |
| Example 7 | 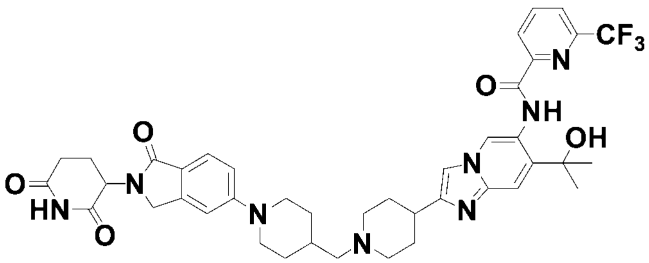 | FIG. 7 |
| Example 8 | 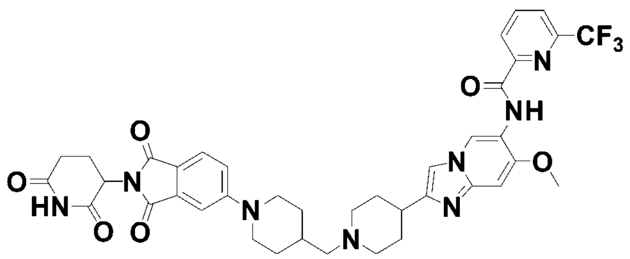 | FIG. 8 |
| Example 9 | 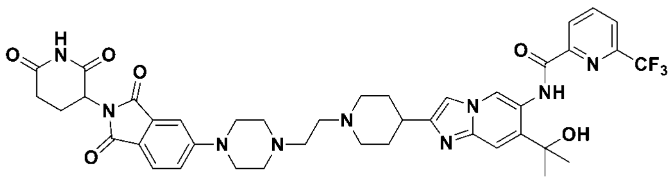 | FIG. 9 |
| Example 10 | 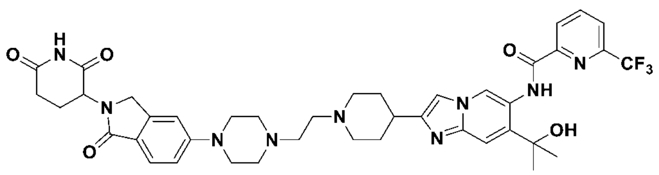 | FIG. 10 |
| Example 11 | 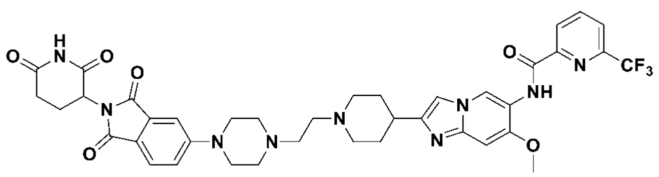 | FIG. 11 |

(continued)

| Compound | Structure | Figure Corresponding to Western Blotting Result |
|---|---|---|
| Example 12 | 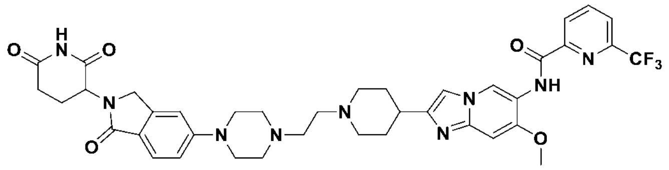 | FIG. 12 |
| Example 13 | 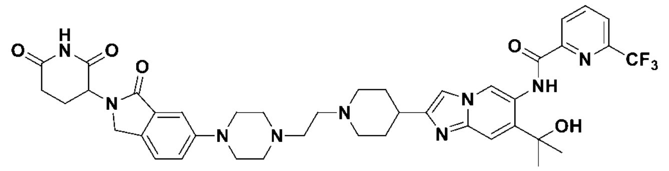 | FIG. 13 |
| Example 14 | 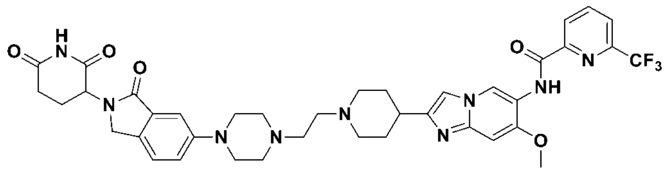 | FIG. 14 |
| Example 15 | 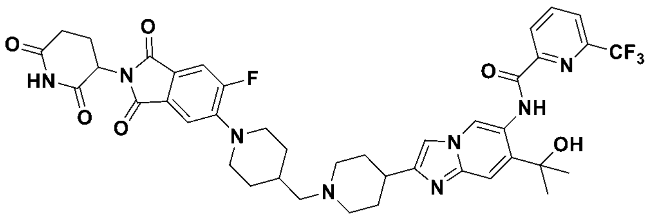 | FIG. 15 |
| Example 16 | 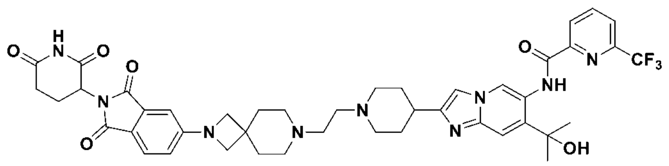 | FIG. 16 |
| Example 17 | 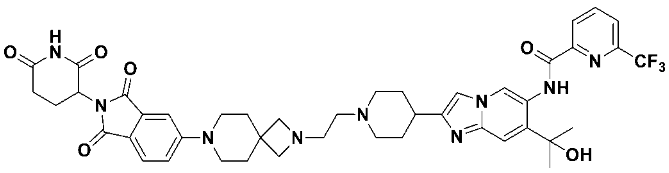 | FIG. 17 |
| Example 18 | 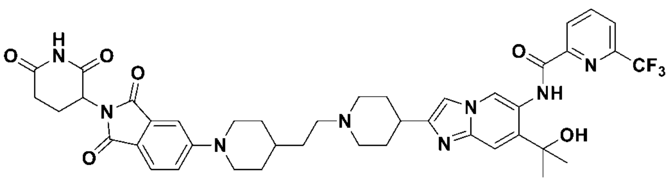 | FIG. 18 |
| Example 19 | 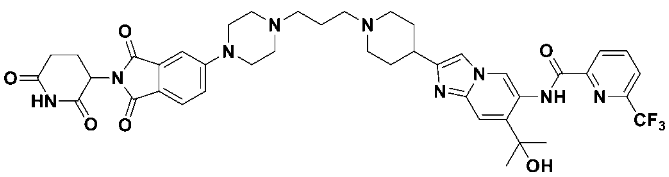 | FIG. 19 |

(continued)

| Compound | Structure | Figure Corresponding to Western Blotting Result |
| --- | --- | --- |
| Example 20 | 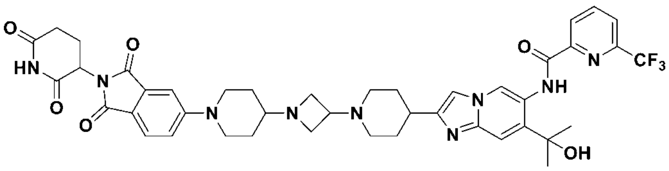 | FIG. 20 |
| Example 21 | 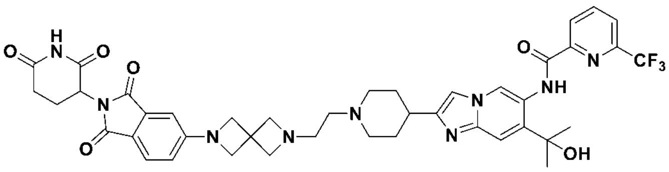 | FIG. 21 |
| Example 22 | 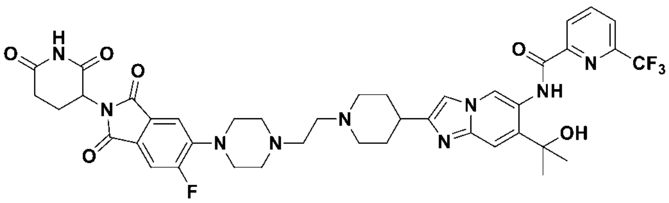 | FIG. 22 |
| Example 23 | 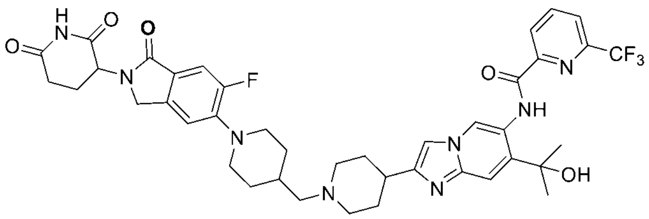 | FIG. 23 |
| Example 24 | 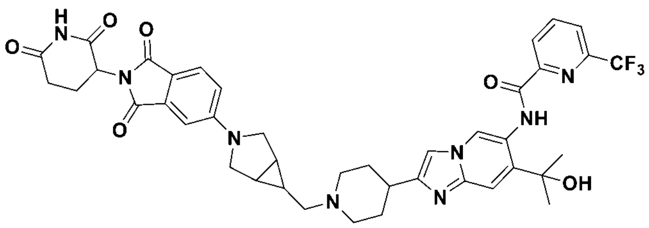 | FIG. 24 |
| Example 25 | 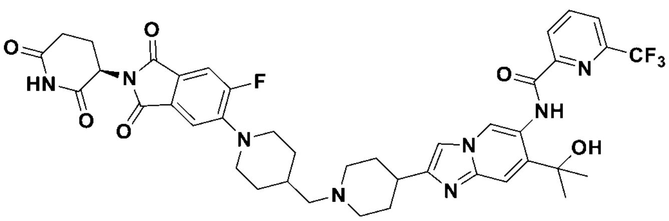 | FIG. 25 |
| Example 26 | 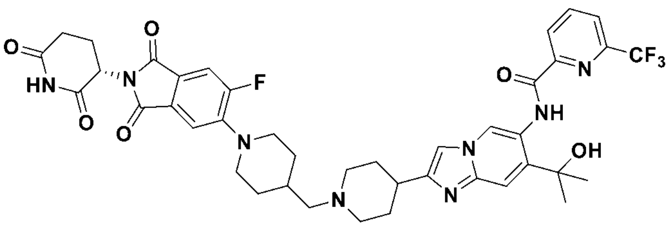 | FIG. 26 |

**Liver microsomal stability assay**

(1) Materials

Microsomes were stored in a -80°C refrigerator, thawed in a 37°C water bath before use, and placed on ice after thawing. All other reagents were purchased from local suppliers.

(2) Experimental design

In step 1, a reaction system was configured as follows:

| Reagent | Stock Concentration | Volume | Final Concentration |
|---|---|---|---|
| Microsomes | 20 mg/mL | 10 μL | 0.5 mg/mL |
| Phosphate buffer | 200 mM | 200 μL | 100 mM |
| Magnesium chloride | 50 mM | 40 μL | 5 mM |
| Alamethicin | 5 mg/mL | 2 μL | 25 μg/mL |
| Water | - | 64 μL | - |

In step 2, 40 μL of 10 mM NADPH solution and 40 μL of 20 mM UDPGA solution were added to the above reaction system, where the final concentrations of NADPH and UDPGA were 1 mM and 2 mM, respectively. 80 μL of ultrapure water was used as negative control to replace the NADPH and UDPGA solutions. The reaction system was pre-incubated for 10 min in a 37°C water bath.

In step 3, 4 μL of 100 μM test compound and reference drugs (verapamil and 7-OHcoumarin) were added to initiate reactions, where the final concentration of each drug was 1 μM.

In step 4, after 0, 15, 30, 45, and 60 min, 50 μL of the reaction sample was taken out separately and quenched with 4 volumes of cold acetonitrile containing internal standards (100 nM alprazolam, 200 nM labetalol, 2 μM ketoprofen, and 200 nM caffeine). The sample was centrifuged for 40 min at 3220 g. After centrifugation, 100 μL of the supernatant and 100 μL of ultrapure water were mixed uniformly for LC-MS/MS analysis and detection.

(3) Data analysis

All data calculations were performed using Microsoft Excel software. Peak areas were determined from extracted ion chromatograms and used for calculating the remaining percentage at each time point.

The remaining percentage was calculated by the following formula:

$$\text{remaining percentage (\%)} = (\text{peak area ratio}_{\text{time point}}/\text{peak area ratio}_{0\ \text{min}}) \times 100.$$

An elimination rate constant (k) was determined through linear fitting of a natural logarithm of the remaining percentage of a parent drug versus time.

The half-life t1/2 was calculated from the elimination rate constant: t1/2 = 0.693/k.

The *in vitro* intrinsic clearance (μL/min/mg) was calculated by the following formula:

$$\textit{invitro}\text{CLint} = \text{kV/N}.$$

In the formula, V denotes an incubation volume per well (0.4 mL), and N denotes an amount of microsomes per well (0.2 mg).

Table 3 lists the experimental results. Example 15 exhibits very good metabolic stability in the liver microsomes of different species.

Table 3

| Species | $T_{1/2}$ (min) | Clint (μL/min/mg) |
|---|---|---|
| Human | 400.73 | 3.46 |
| Monkey | 191.84 | 7.22 |
| Dog | 259.72 | 5.34 |

(continued)

| Species | $T_{1/2}$ (min) | Clint (μL/min/mg) |
|---|---|---|
| Rat | 133.68 | 10.37 |
| Mouse | 143.40 | 9.67 |

***In vivo* pharmacokinetic study**

The pharmacokinetic behaviors of Example 1 and Example 15 in mice were studied using the following experimental protocol to evaluate their pharmacokinetic characteristics.

Experimental protocol

Three healthy CD1 mice, which were male and each had a weight of 20-25 g, were each intravenously injected with the compound at 2 mg/kg, where the administration was performed at a dosing volume of 10 mL/kg formulated in 5% DMSO/15% Solutol/80% PBS (w/v). The mice had free access to food and water before the test.

Three healthy CD1 mice, which were male and each had a weight of 20-25g, were each orally administered with the compound at 20 mg/kg, where the administration was performed at a dosing volume of 10 mL/kg formulated in 5% DMSO/15% Solutol/80% PBS (w/v). The mice had free access to food and water before the test.

Approximately 0.03 mL of venous blood was collected from the dorsal metatarsal vein of each mouse separately after 0.033 h, 0.083 h, 0.25 h, 0.5 h, 1 h, 2 h, 4 h, 8 h, and 24 h since intravenous injection. The blood was placed in an EDTA-$K_2$ tube and centrifuged for 5 min at 4000 g and 4°C. The plasma was separated. The concentration of the compound in the plasma was determined through liquid chromatography-tandem mass spectrometry.

Approximately 0.03 mL of venous blood was collected from the dorsal metatarsal vein of each mouse separately after 0.083 h, 0.25 h, 0.5 h, 1 h, 2 h, 4 h, 8 h, and 24 h since oral administration. The blood was placed in an EDTA-$K_2$ tube and centrifuged for 5 min at 4000 g and 4°C. The plasma was separated. The concentration of the compound in the plasma was determined through liquid chromatography-tandem mass spectrometry.

As shown in Table 4, the experimental results show that the compounds of the present application have low clearance, high plasma exposure, and good oral absorption, and in particular, Example 15 has an oral bioavailability of as high as 61.7% (in Table 4, Cl represents clearance, F represents bioavailability, and AUC represents the area under the curve).

Table 4

| | Intravenous Injection (2 mg/kg) | | | Oral Administration (20 mg/kg) | | |
|---|---|---|---|---|---|---|
| | Cl (mL/min/kg) | $T_{1/2}$ (h) | $AUC_{last}$ (h×ng/mL) | $T_{1/2}$ (h) | $AUC_{last}$ (h×ng/mL) | F (%) |
| Example 1 | 59.5 | 7.88 | 550 | 6.81 | 626 | 12.1 |
| Example 15 | 37.2 | 8.05 | 870 | 8.01 | 4761 | 61.7 |

The applicant has stated that although the compound and the preparation and use thereof in the present application are described through the preceding embodiments, the present application is not limited to the preceding embodiments, which means that the implementation of the present application does not necessarily depend on the preceding embodiments. It is to be understood by those skilled in the art that any improvements made to the present application, equivalent replacements of raw materials selected in the present application, additions of adjuvant ingredients, and selections of specific methods, etc., all fall within the protection scope and the disclosed scope of the present application.

**Claims**

**1.** An imidazo[1,2-a]pyridine IRAK4 degrader or a stereoisomer, geometric isomer, tautomer, or pharmaceutically acceptable salt thereof, wherein the imidazo[1,2-a]pyridine IRAK4 degrader has a structure represented by Formula I:

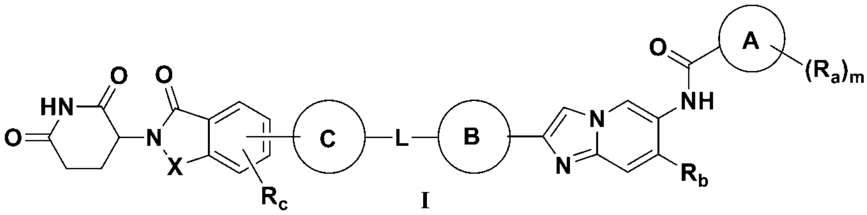

I

;

wherein

the ring A is 6- to 10-membered aryl or 5- to 10-membered heteroaryl;
the ring B is substituted or unsubstituted 4- to 10-membered cycloalkyl or substituted or unsubstituted 4- to 10-membered heterocycloalkyl;
the ring C is substituted or unsubstituted 4- to 10-membered cycloalkyl or substituted or unsubstituted 4- to 10-membered heterocycloalkyl;
X is $CH_2$ or C=O;
$R_a$ is each independently selected from halogen, cyano, substituted or unsubstituted C1 to C4 alkyl, substituted or unsubstituted C1 to C4 alkoxy, di(C1 to C4 alkyl)amino, 5- to 10-membered cycloalkyl, or 5- to 10-membered heterocycloalkyl;
m is an integer from 0 to 5;
$R_b$ is selected from C1 to C4 alkoxy, C3 to C6 cycloalkyloxy, or hydroxyldi(C1 to C4 alkyl)methyl;
$R_c$ is hydrogen, C1 to C4 alkyl, C1 to C4 alkoxy, or halogen; and
L is $(CH_2)_n$, $(CH_2)_nCO$, 4- to 8-membered cycloalkyl, or 4- to 8-membered heterocycloalkyl, wherein n is an integer from 0 to 5.

**2.** The imidazo[1,2-a]pyridine IRAK4 degrader or the stereoisomer, geometric isomer, tautomer, or pharmaceutically acceptable salt thereof according to claim 1, wherein a substituent in the substituted group is selected from halogen, oxo, cyano, amino, hydroxyl, C1 to C6 alkyl, or -O-(C1 to C6 alkyl).

**3.** The imidazo[1,2-a]pyridine IRAK4 degrader or the stereoisomer, geometric isomer, tautomer, or pharmaceutically acceptable salt thereof according to claim 1 or 2, wherein the ring

A is 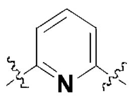, 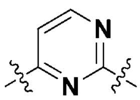, 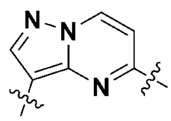, or 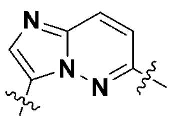,

wherein the squiggle represents a linkage site of the group.

**4.** The imidazo[1,2-a]pyridine IRAK4 degrader or the stereoisomer, geometric isomer, tautomer, or pharmaceutically acceptable salt thereof according to any one of claims 1 to 3, wherein the ring B is

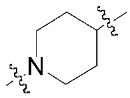, 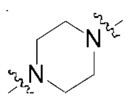, or 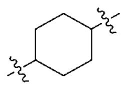,

wherein the squiggle represents a linkage site of the group.

**5.** The imidazo[1,2-a]pyridine IRAK4 degrader or the stereoisomer, geometric isomer, tautomer, or pharmaceutically acceptable salt thereof according to any one of claims 1 to 4, wherein the ring C is

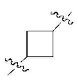, 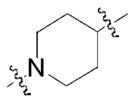, 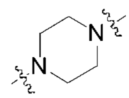, 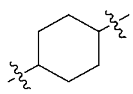, 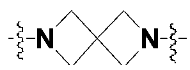,

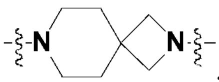, 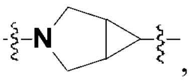, or 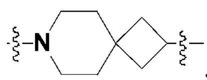,

wherein the squiggle represents a linkage site of the group.

**6.** The imidazo[1,2-a]pyridine IRAK4 degrader or the stereoisomer, geometric isomer, tautomer, or pharmaceutically acceptable salt thereof according to any one of claims 1 to 5, wherein $R_a$ is each independently selected from fluorine, chlorine, cyano, trifluoromethyl, trifluoromethoxy,

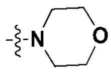, or 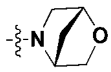,

wherein the squiggle represents a linkage site of the group;

preferably, $R_b$ is selected from any one of methoxy, ethoxy, isopropoxy, cyclopropyloxy, or

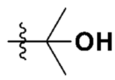,

wherein the squiggle represents a linkage site of the group;
preferably, $R_c$ is selected from hydrogen, fluorine, chlorine, or bromine;
preferably, L is $-CH_2-$, $-CH_2CH_2-$, $-CH_2CH_2CH_2-$, $-COCH_2-$, $-COCH_2CH_2-$, $-COCH_2CH_2CH_2-$, or

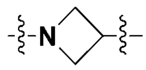,

wherein the squiggle represents a linkage site of the group.

**7.** The imidazo[1,2-a]pyridine IRAK4 degrader or the stereoisomer, geometric isomer, tautomer, or pharmaceutically acceptable salt thereof according to any one of claims 1 to 6, wherein the imidazo[1,2-a]pyridine IRAK degrader is selected from any one of the following compounds:

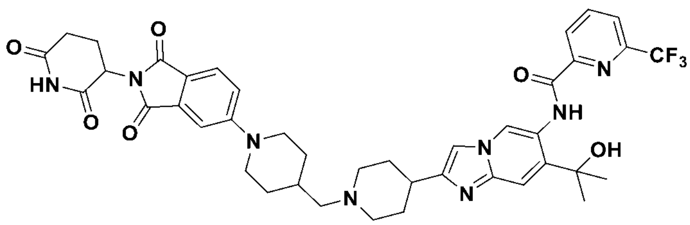,

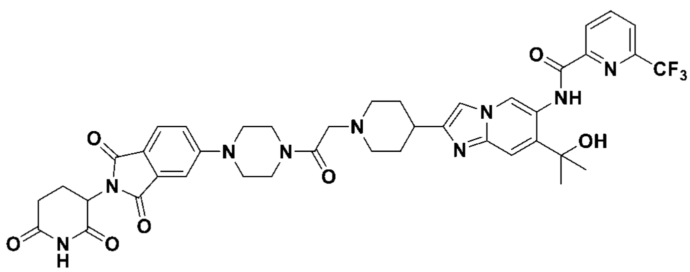,

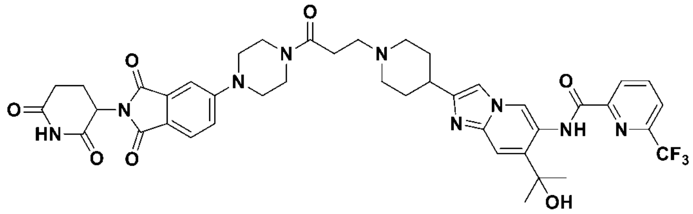

,

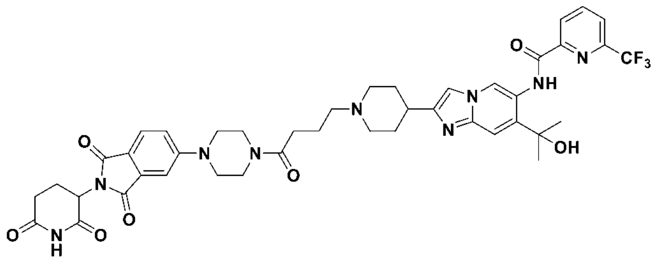

,

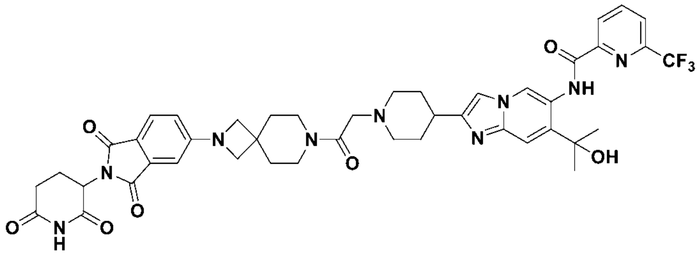

,

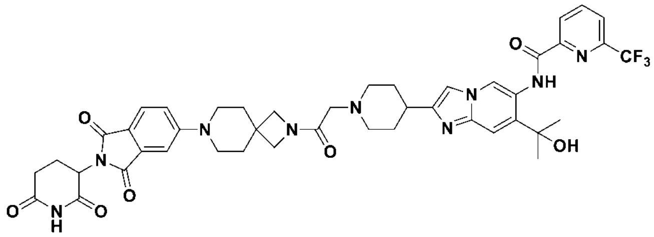

,

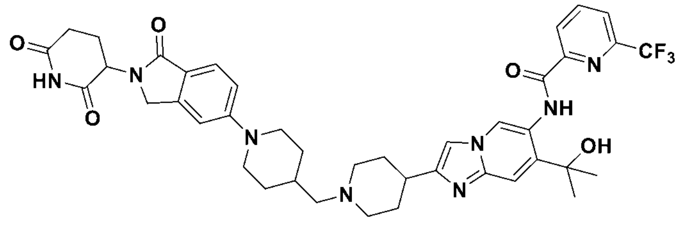

,

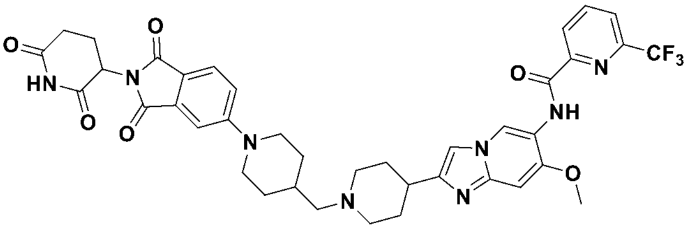

,

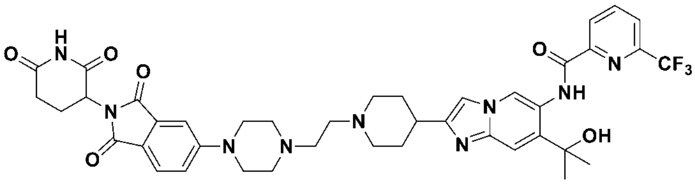
,

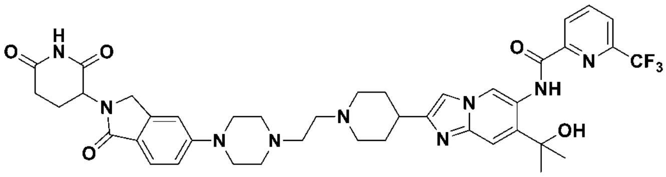
,

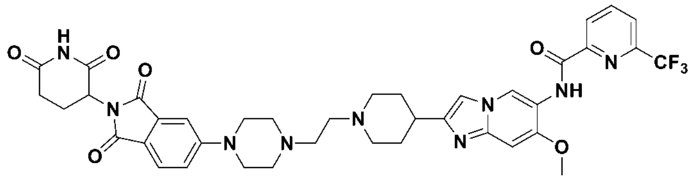
,

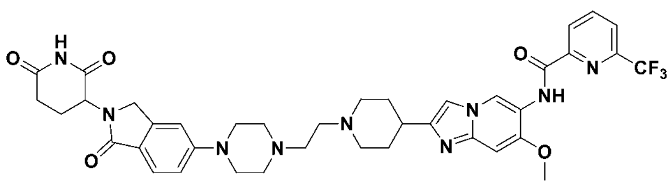
,

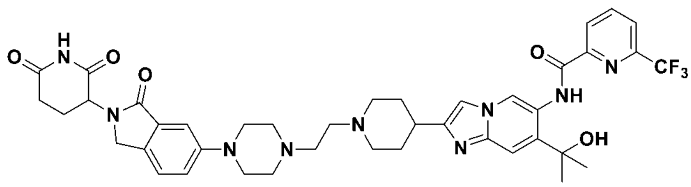
,

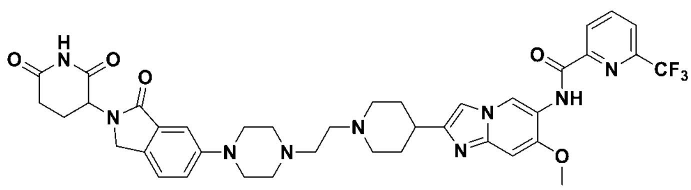
,

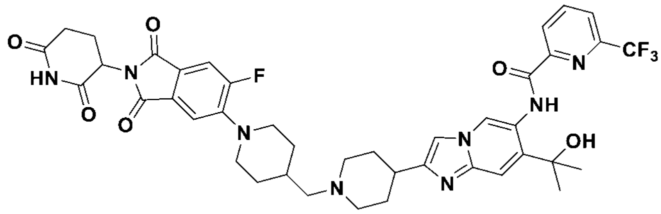
,

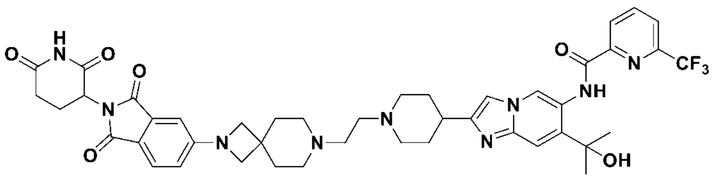

,

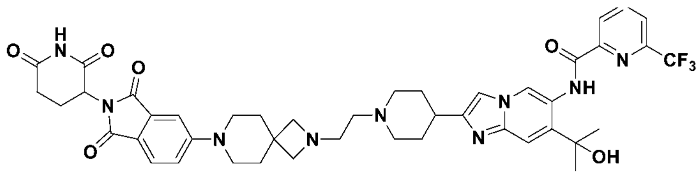

,

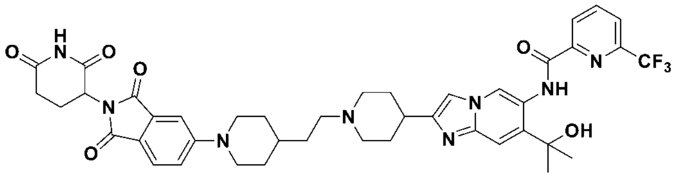

,

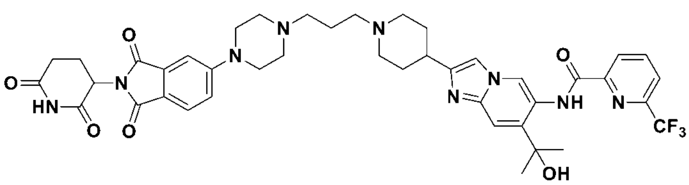

,

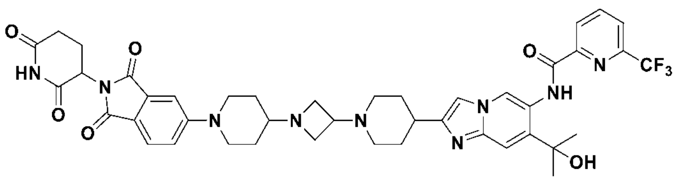

,

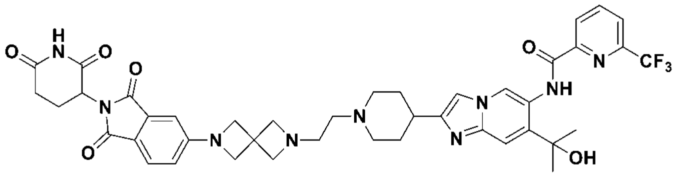

,

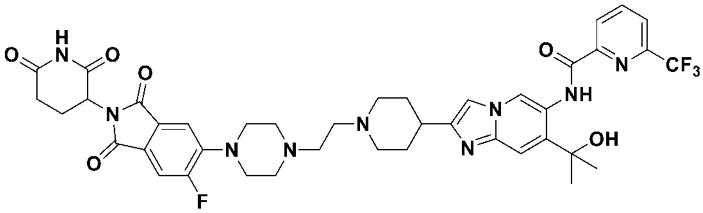

,

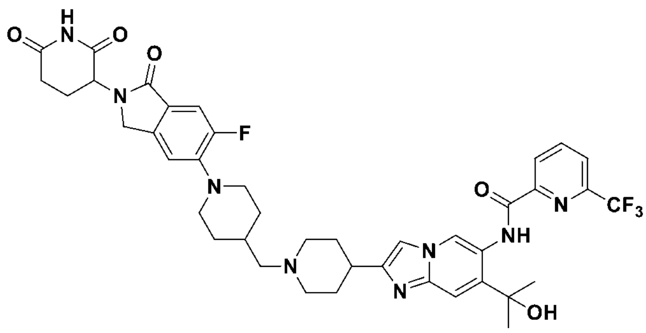

,

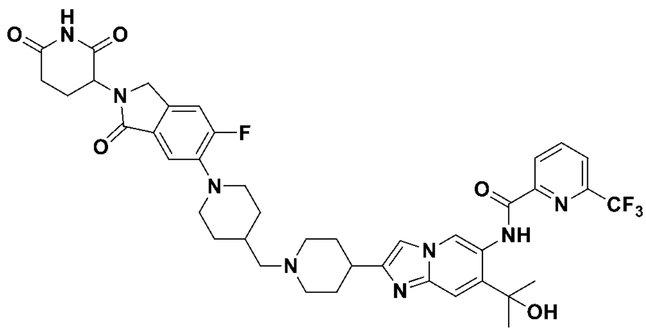

,

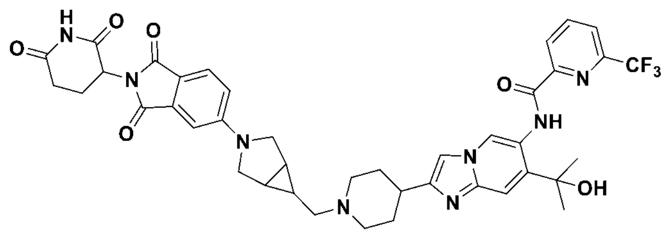

,

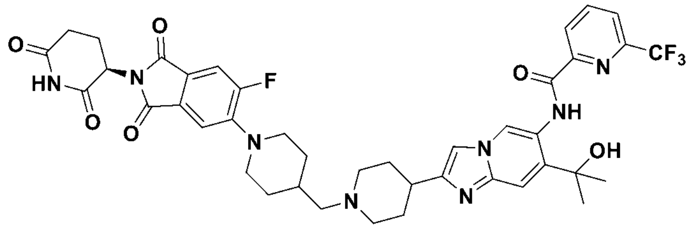

,

or

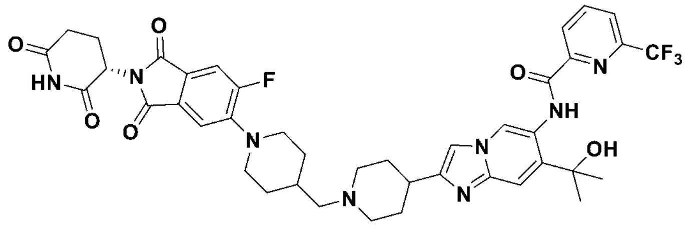

.

**8.** The imidazo[1,2-a]pyridine IRAK4 degrader or the stereoisomer, geometric isomer, tautomer, or pharmaceutically acceptable salt thereof according to any one of claims 1 to 7, wherein the pharmaceutically acceptable salt comprises any one of the following salts: sulfate, pyrosulfate, bisulfate, sulfite, bisulfite, phosphate, monohydrogen phosphate, dihydrogen phosphate, metaphosphate, pyrophosphate, hydrochloride, hydrobromide, hydroiodide, acetate, propionate, decanoate, octanoate, acrylate, formate, isobutyrate, hexanoate, heptanoate, propiolate, oxalate, malonate,

succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, γ-hydroxybutyrate, glycolate, tartrate, methanesulfonate, propanesulfonate, naphthalene-1-sulfonate, naphthalene-2-sulfonate, or mandelate.

**9.** A pharmaceutical composition, comprising the imidazo[1,2-a]pyridine IRAK4 degrader or the stereoisomer, geometric isomer, tautomer, or pharmaceutically acceptable salt thereof according to any one of claims 1 to 8.

**10.** Use of the imidazo[1,2-a]pyridine IRAK4 degrader or the stereoisomer, geometric isomer, tautomer, or pharmaceutically acceptable salt thereof according to any one of claims 1 to 9 or the pharmaceutical composition according to claim 8 for preparing a medicament for treating a disease or disorder mediated by IRAK4 .

**11.** The use according to claim 9, wherein the disease comprises an inflammatory disease, an autoimmune disease, or a tumor;

preferably, the inflammatory disease is selected from Crohn's disease, ulcerative colitis, asthma, graft-versus-host disease, chronic obstructive pulmonary disease, atopic dermatitis, gout, gouty arthritis, conjunctivitis, hepatitis, chronic pulmonary inflammatory disease, thyroiditis, or interstitial cystitis;
preferably, the autoimmune disease is selected from Graves' disease, rheumatoid arthritis, systemic lupus erythematosus, lupus nephritis, cutaneous lupus, psoriasis, psoriatic arthritis, cryopyrin-associated periodic syndrome, TNF receptor associated periodic syndrome, multiple sclerosis, allergic rhinitis, scleroderma, dermatomyositis, vasculitis, or nephritis;
preferably, the tumor is selected from lung cancer, breast cancer, prostate cancer, pancreatic cancer, kidney cancer, liver cancer, gastrointestinal cancer, cervical cancer, endometrial carcinoma, testicular cancer, genitourinary tract cancer, colorectal cancer, laryngeal cancer, skin cancer, bone cancer, head and neck tumor, sarcoma, cerebroma, glioblastoma, melanoma, multiple myeloma, lymphoma, or leukemia.

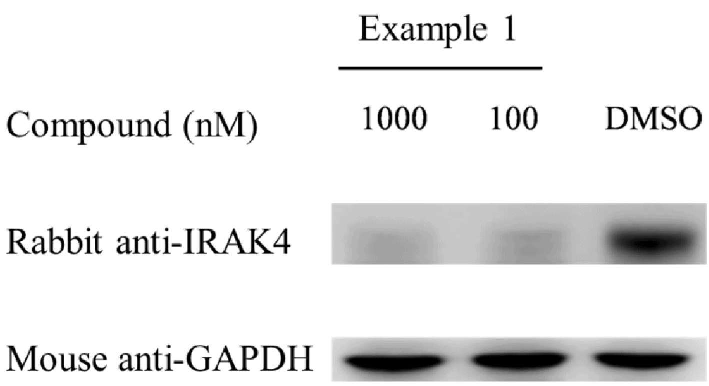


Figure 1

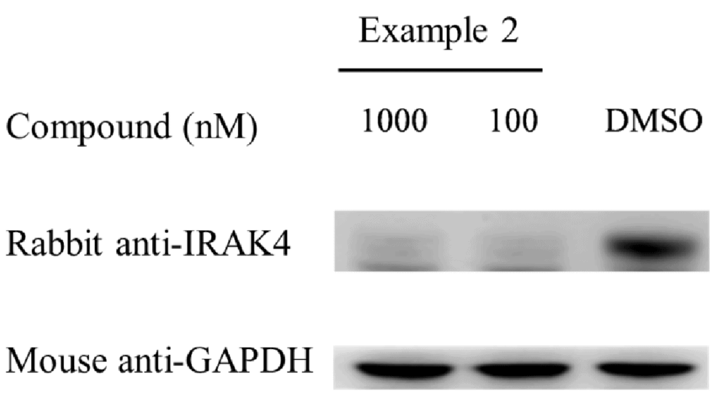


Figure 2

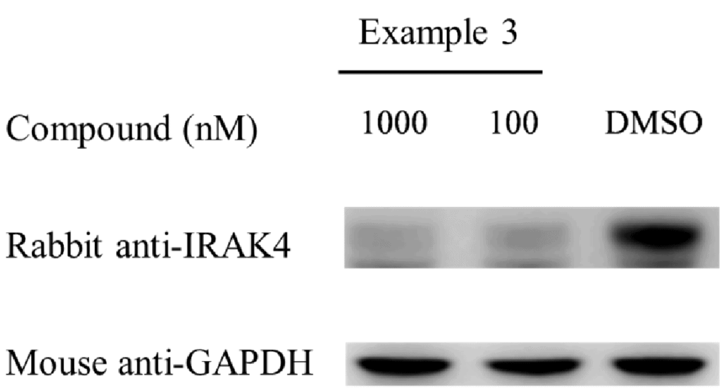


Figure 3

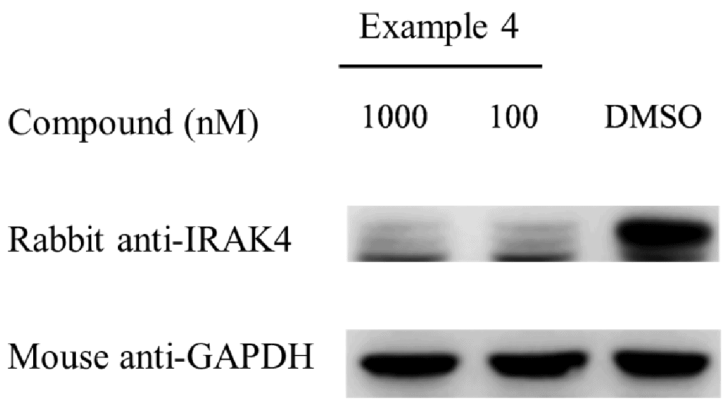


Figure 4

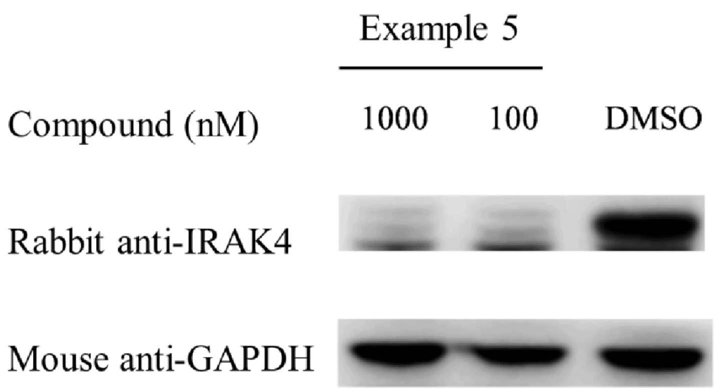


Figure 5

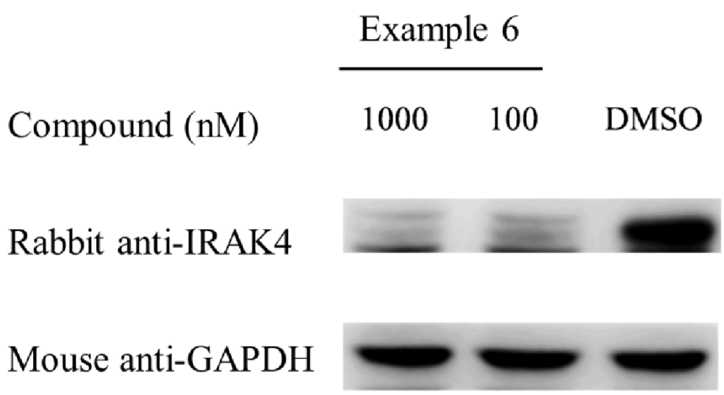


Figure 6

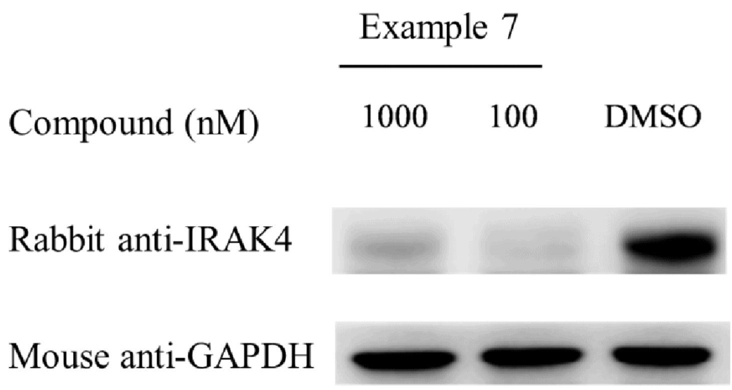


Figure 7

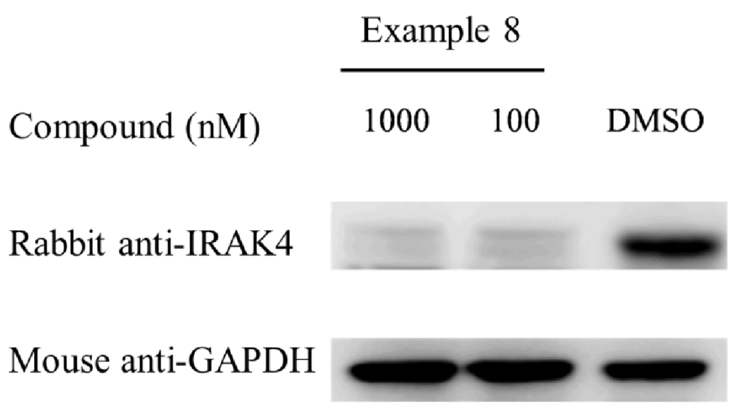


Figure 8

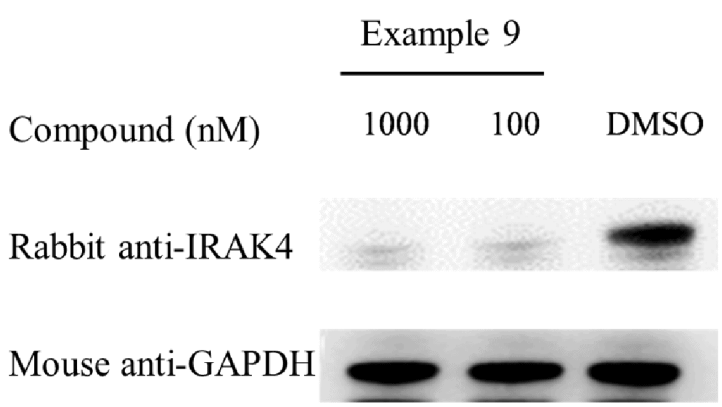


Figure 9

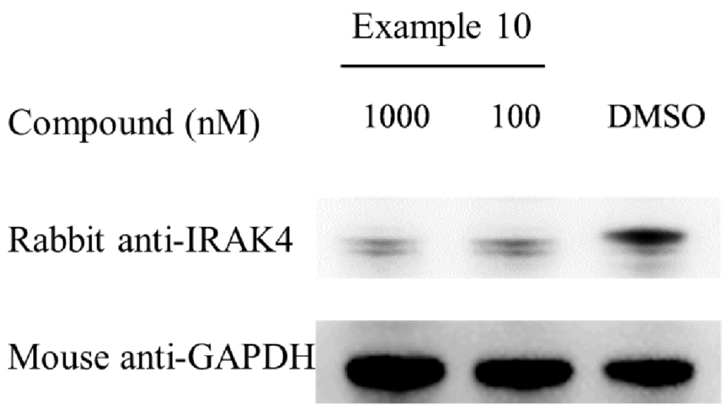


Figure 10

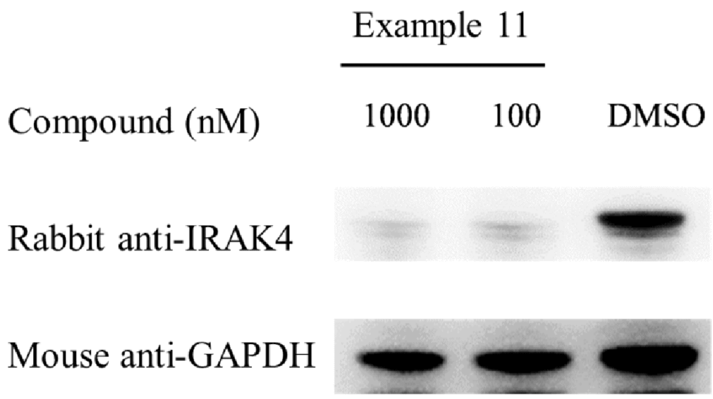


Figure 11

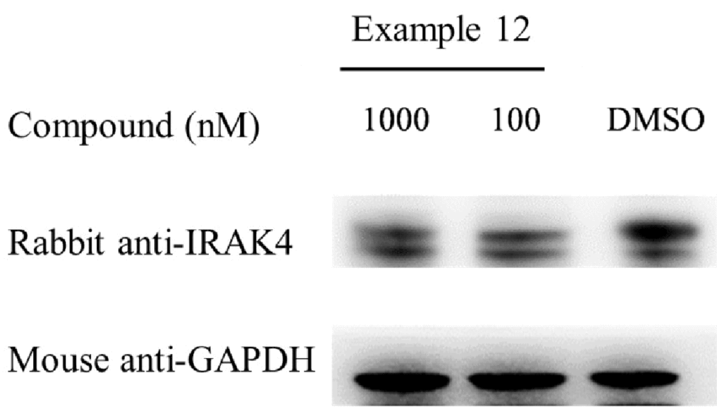


Figure 12

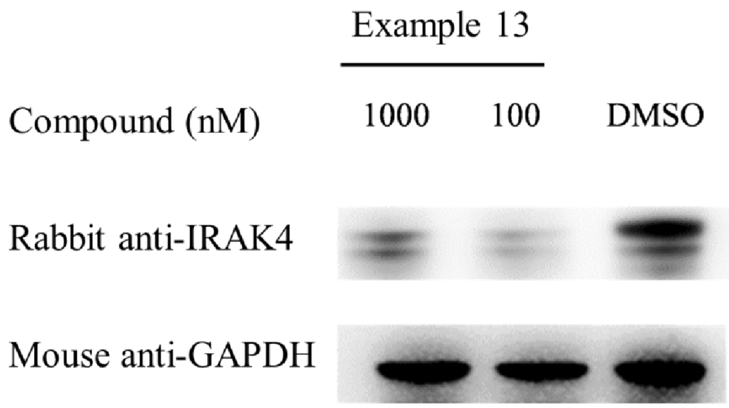


Figure 13

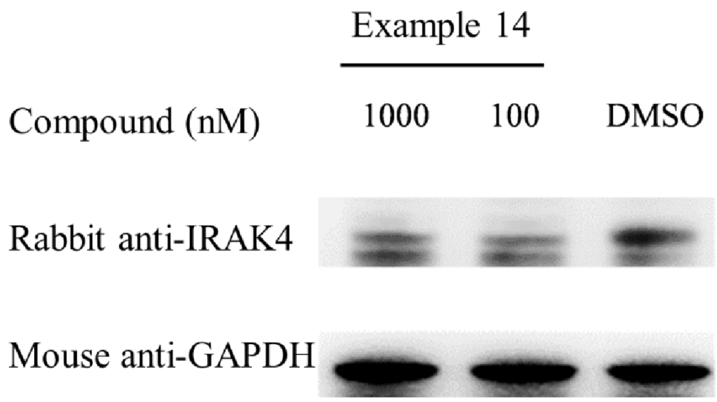


Figure 14

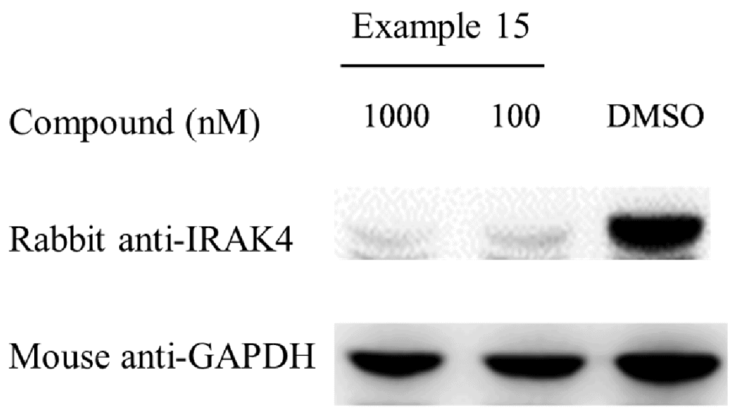


Figure 15

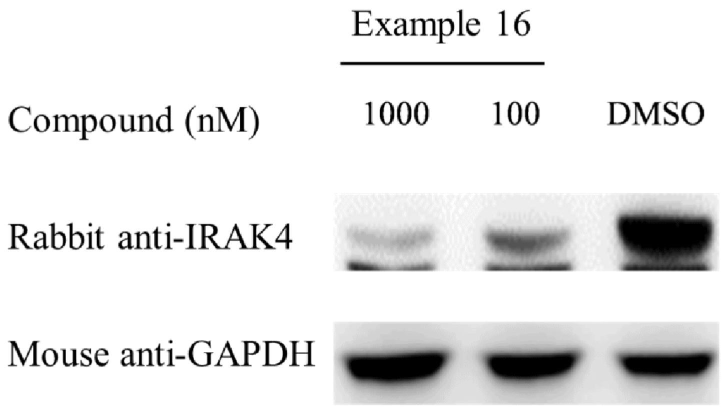


Figure 16

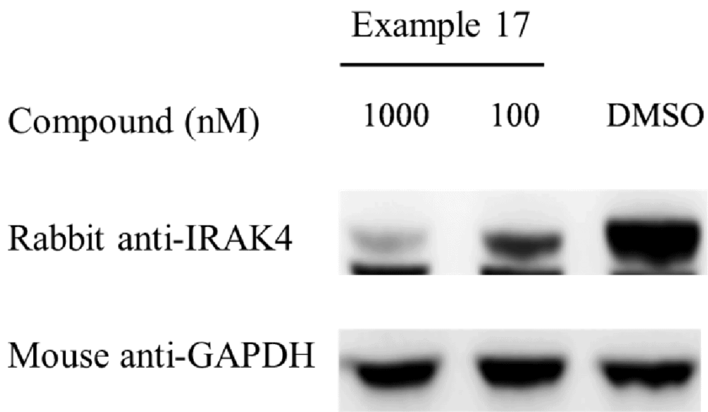


Figure 17

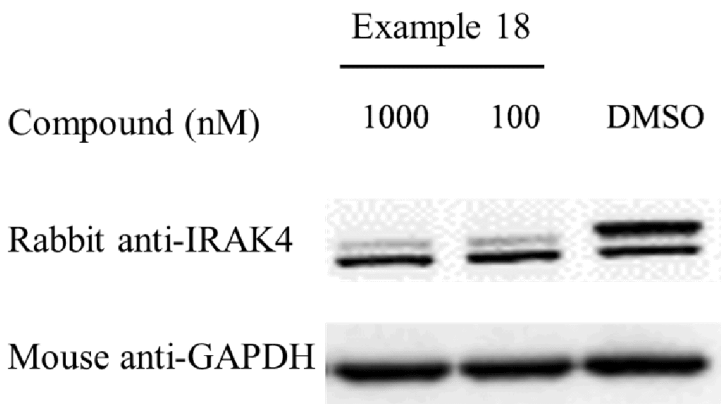


Figure 18

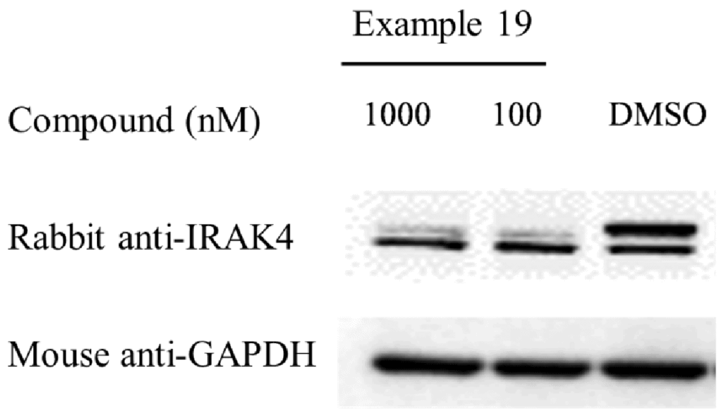


Figure 19

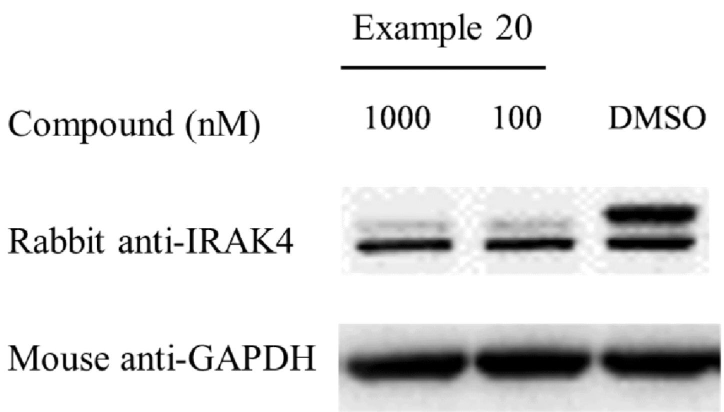


Figure 20

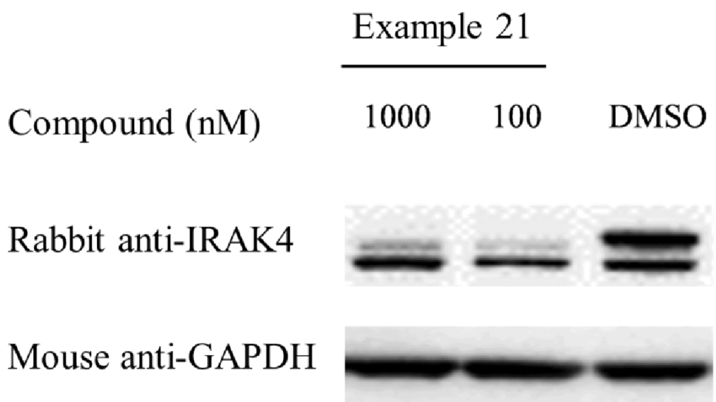


Figure 21

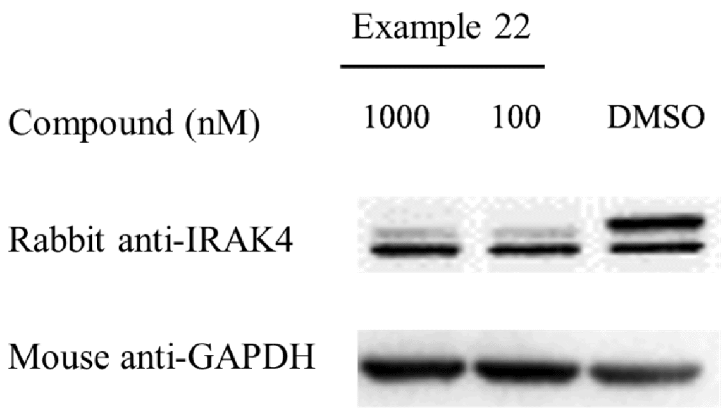


Figure 22

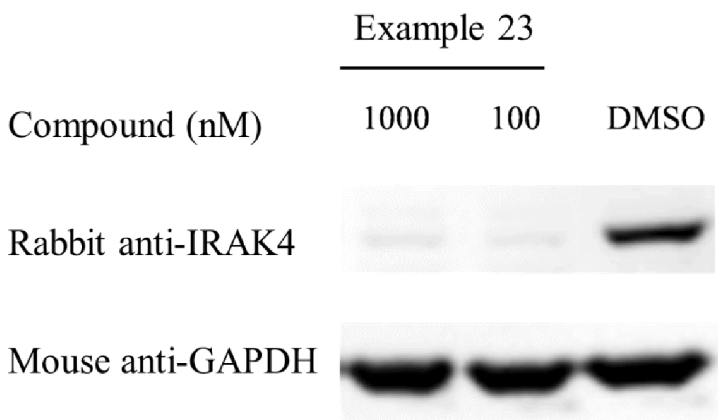


Figure 23

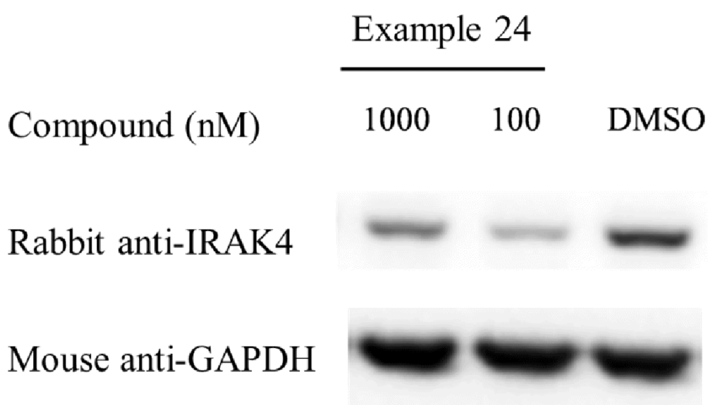


Figure 24

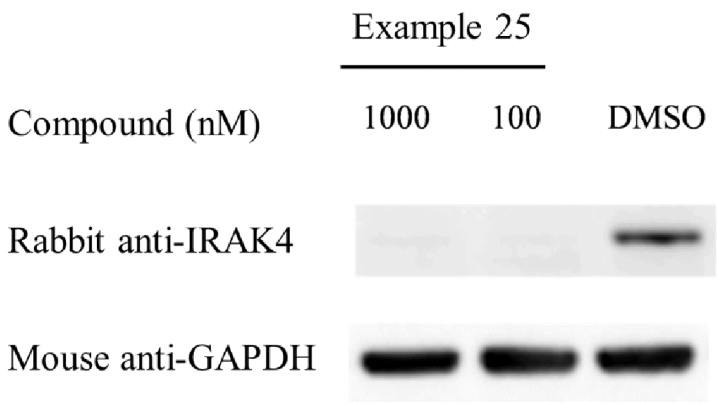


Figure 25

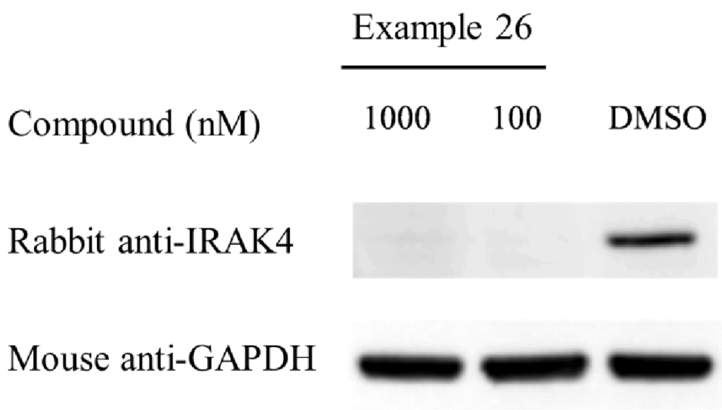


Figure 26

**INTERNATIONAL SEARCH REPORT**

International application No.
**PCT/CN2024/104246**

**A. CLASSIFICATION OF SUBJECT MATTER**

C07D471/04(2006.01)i; A61K31/454(2006.01)i; A61K31/4188(2006.01)i; A61P29/00(2006.01)i; A61P35/00(2006.01)i; A61P37/02(2006.01)i

According to International Patent Classification (IPC) or to both national classification and IPC

**B. FIELDS SEARCHED**

Minimum documentation searched (classification system followed by classification symbols)

IPC: C07D471/-. A61K31/-, A61P29/-, A61P35/-, A61P37/-

Documentation searched other than minimum documentation to the extent that such documents are included in the fields searched

Electronic data base consulted during the international search (name of data base and, where practicable, search terms used)

CNTXT: ENTXTC; VEN; CNKI; Caplus; REG: IRAK4, 根据权利要求1的结构式进行了结构式检索, structural formula search performed on the basis of the formula of claim 1

**C. DOCUMENTS CONSIDERED TO BE RELEVANT**

| Category* | Citation of document, with indication, where appropriate, of the relevant passages | Relevant to claim No. |
|---|---|---|
| PX | WO 2024020522 A1 (ARVINAS OPERATIONS, INC.) 25 January 2024 (2024-01-25) claims 1-80, and description, embodiments 1-4 | 1-11 |
| PX | CN 117777126 A (WUHAN HUMANWELL INNOVATIVE DRUG RESEARCH AND DEVELOPMENT CENTER LIMITED COMPANY et al.) 29 March 2024 (2024-03-29) claims 1-10 | 1-11 |
| Y | WO 2023283372 A1 (BIOGEN MA INC. et al.) 12 January 2023 (2023-01-12) claims 1-58, description, embodiments 1 and 37, et al. | 1-11 |
| Y | CN 114502158 A (KYMERA THERAPEUTICS, INC.) 13 May 2022 (2022-05-13) description, formulas I-oo-5 and I-zz-1 to I-zz-4, paragraphs [0209]-[0249], and a compound of formula I-345 | 1-11 |
| Y | CN 115244042 A (SHANGHAI LEADINGTAC PHARMACEUTICAL CO., LTD.) 25 October 2022 (2022-10-25) claims 1-19 | 1-11 |
| Y | CN 116867758 A (KYMERA THERAPEUTICS, INC.) 10 October 2023 (2023-10-10) claims 1-27 | 1-11 |

☐ Further documents are listed in the continuation of Box C.   ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 September 2024** | **09 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/104246**

| Patent document cited in search report | Publication date (day/month/year) | Patent family member(s) | Publication date (day/month/year) |
|---|---|---|---|
| WO 2024020522 A1 | 25 January 2024 | None | |
| CN 117777126 A | 29 March 2024 | WO 2024067845 A1 | 04 April 2024 |
| WO 2023283372 A1 | 12 January 2023 | JP 2024525580 A | 12 July 2024 |
| | | EP 4366834 A1 | 15 May 2024 |
| CN 114502158 A | 13 May 2022 | EP 3989966 A1 | 04 May 2022 |
| | | EP 3989966 A4 | 27 September 2023 |
| | | JP 2022538192 A | 31 August 2022 |
| | | WO 2020264499 A1 | 30 December 2020 |
| | | US 2023069104 A1 | 02 March 2023 |
| | | BR 112021026517 A2 | 10 May 2022 |
| | | CO 2021017893 A2 | 17 January 2022 |
| | | CA 3144805 A1 | 30 December 2020 |
| | | KR 20220032063 A | 15 March 2022 |
| | | IL 289267 A | 01 February 2022 |
| | | MX 2021015995 A | 11 March 2022 |
| | | AU 2020302118 A1 | 24 February 2022 |
| CN 115244042 A | 25 October 2022 | US 2023192655 A1 | 22 June 2023 |
| | | EP 4238968 A1 | 06 September 2023 |
| | | JP 2023529108 A | 07 July 2023 |
| | | JP 7474527 B2 | 25 April 2024 |
| | | WO 2022088551 A1 | 05 May 2022 |
| CN 116867758 A | 10 October 2023 | IL 303987 A | 01 August 2023 |
| | | TW 202241891 A | 01 November 2022 |
| | | CA 3202360 A1 | 07 July 2022 |
| | | AU 2021413371 A1 | 13 July 2023 |
| | | JP 2024503300 A | 25 January 2024 |
| | | CO 2023008362 A2 | 21 July 2023 |
| | | WO 2022147465 A1 | 07 July 2022 |
| | | AR 124547 A1 | 05 April 2023 |
| | | MX 2023007852 A | 07 July 2023 |
| | | EP 4271664 A1 | 08 November 2023 |
| | | US 2023101353 A1 | 30 March 2023 |
| | | KR 20230143632 A | 12 October 2023 |

Form PCT/ISA/210 (patent family annex) (July 2022)